# EUROPEAN PATENT APPLICATION

(11) **EP 3 401 337 A1**
(43) Date of publication of application: **14.11.2018**
(21) Application number: 18177798.8
(22) Date of filing: 25.09.2013
(51) Int. Cl.: C07K 16/46, B01D 15/38

(54) **PURIFICATION OF HETERO-DIMERIC IMMUNOGLOBULINS**

(30) Priority: 25.09.2012 US 201261705278 P
(62) Divisional of application: 13766540.2
(71) Applicant: Glenmark Pharmaceuticals S.A., 2300 La Chaux-de-Fonds (CH)
(72) Inventor: BLEIN, Stanislas, 2300 La Chaux-de-Fonds (CH); COMPER, Fabrizio, 2300 La Chaux-de-Fonds (CH); OLLIER, Romain, 2300 La Chaux-de-Fonds (CH); WASSMANN, Paul, 2300 La Chaux-de-Fonds (CH)
(74) Representative: Thomas, Dean

(57) **Abstract**

The present invention describes novel hetero-dimeric immunoglobulin variants or fragments thereof, which have reduced or eliminated binding to Protein A, Protein G or both Protein A and Protein G. Also encompassed in the present invention are methods for the selective purification of hetero-dimeric immunoglobulins or fragments thereof using Protein A and Protein G.

## Description

### Field of the Invention

The present invention relates generally to methods for the selective purification of hetero-dimeric immunoglobulins. Specific substitutions that eliminate the affinity for Protein A or G can be introduced in one heavy chain of the hetero-dimeric immunoglobulin. In a further aspect of the present invention, substitutions that eliminate the affinity for Protein A can be introduced in one heavy chain of the hetero-dimeric immunoglobulin, and substitutions that eliminate the affinity for Protein G are introduced in the other heavy chain of the hetero-dimeric immunoglobulin, thereby providing methods to readily purify the hetero-dimeric immunoglobulin using a combination of Protein A and Protein G affinity chromatography.

### Background

Methods to produce hetero-dimeric immunoglobulins are known in the art and one of the simplest methods relies on expressing the two distinct immunoglobulin chains in a single cell (WO95/33844, Lindhofer H & Thierfelder S). Without engineering, this straightforward method is limited by the formation of homo-dimeric species over the hetero-dimer of interest (Kufer P et al., (2004) Trends Biotechnol., 22(5): 238-244). When using complementary technologies that will enhance heavy chain hetero-dimerization (Merchant AM et al., (1998) Nat. Biotechnol., 16(7): 677-681), greater hetero-dimer production can be achieved but still results in the production of a significant amount of undesirable homo-dimers (Jackman J et al., (2010) J Biol Chem., 285(27):20850-9, Klein C et al., (2012) MAbs, 4(6):653-63).

Techniques that ease the recovery of hetero-dimers from homo-dimers based on a differential affinity of the hetero-dimers for an affinity reagent have been described. The first example of differential affinity technique involved the use of two different heavy chains from two different animal species, wherein one of which does not bind the affinity reagent Protein A (Lindhofer H et al., (1995) J Immunol., 155(1): 219-225). The same authors also described the use of two different heavy chains originating from two different human immunoglobulin isotypes (IGHG1 and IGHG3), one of which does not bind the affinity reagent Protein A (IGHG3; see US6,551,592 Lindhofer H et al.)*.* A variation of the latter technique has been described in WO10/151792 (Davis S et al.) and involved the use of the two amino acid substitutions H435R/Y436F described by Jendeberg *et al* (Jendeberg et al., (1997) J. Immunol. Methods, 201(1): 25-34) to abrogate the affinity for the reagent Protein A in one of the hetero-dimer heavy chains.
The drawbacks of current differential purification techniques based on Protein A are that they do not address the contribution of VH3 domains that may be present in the heavy chains thereby creating additional Protein A binding sites that will interfere with the purification methods.

The known differential purification techniques described above preferably use gradient mode chromatography to allow for the separation of homo-dimers from hetero-dimers. To readily separate the two homo-dimers of heavy chains from the hetero-dimer of interest using capture-elution mode, two different purification methods need to be run sequentially.

A combination of differential purification techniques has been proposed that is based on a modification of one CH1 domain of a hetero-dimeric antibody for reduced binding to the CaptureSelect® IgG-CH1 affinity reagent (PCT Publication No: WO13/136186 Fischer N et al). However a drawback to this technique is that at least one heavy chain needs to encompass a CH1 region to remove both homo-dimers, thereby limiting the scope of this technology. Hence there is need for a technique complementary to the differential Protein A purification technique that would create a difference in binding to a second affinity reagent, that would ideally bind a region confined to the Fc region of immunoglobulins thereby avoiding the modification of antigen binding sites, and which is amendable to any antigen binding scaffold.

In naturally occurring human immunoglobulins of gamma isotype that are known to bind the bacterial surface Protein A and Protein G (IGHG1, IGHG2, and IGHG4; Jendeberg *et al.,* (1997) *supra* and Nezlin R & Ghetie V, (2004) Advances in Immunology, Academic Press, Vol. 82: 155-215), each heavy chain carries a binding site at the CH2-CH3 domain interface for each of the two bacterial surface proteins. Since the binding sites for Protein A and Protein G overlap in heavy chains, specific substitutions that would reduce or eliminate Protein G binding would be useful to purify hetero-dimers of heavy chains in a similar manner to the Protein A based methods described above. In addition, a differential affinity method based on Protein G will offer new strategies for the purification of hetero-dimeric immunoglobulins. Combining both differential affinity methods would be advantageous to readily prepare hetero-dimers of heavy chains with a high degree of purity and without running any forms of gradient elution. In this approach, the hetero-dimer of heavy chains has one heavy chain which binds Protein A but has reduced or no binding to Protein G, while its other heavy chain binds Protein G but has reduced or no binding to Protein A.

The amino acid residues which are involved in Protein A or G binding can be deduced from the experimentally solved crystal structures of immunoglobulins in complex with the bacterial surface proteins (Protein Data Bank (PDB) database; www.pdb.org), however since the binding sites for Protein A, Protein G and FcRn receptor overlap at the same CH2-CH3 domain interface, it is impossible to predict the outcome of any substitution in terms of its effect towards the affinity for either Protein A or Protein G and furthermore its impact on FcRn affinity.

In contrast to naturally occurring immunoglobulins wherein heavy chains are homo-dimers, hetero-dimeric immunoglobulins of the present invention have two different heavy chains (hetero-dimers of heavy chains) and include but are not limited to full length bispecific antibodies, monovalent FAB-Fc fusions and bispecific scFv/FAB Fc fusions.

### Summary of the Invention

The present invention relates generally to novel immunoglobulin and hetero-dimeric immunoglobulin variants, which have reduced or eliminated binding to Protein G, Protein A or both Protein G and Protein A. Also encompassed in the present invention are methods for the selective purification of hetero-dimeric immunoglobulins.

In a first aspect the present invention provides an immunoglobulin or fragment thereof, comprising:
a polypeptide comprising an epitope-binding region and an immunoglobulin constant region wherein the immunoglobulin constant region is selected from the group consisting of: a CH1 region, a CH2 region and a CH3 region,
wherein the immunoglobulin constant region comprises a modification that reduces or eliminates binding of the immunoglobulin or fragment thereof to Protein G, and
wherein if the immunoglobulin constant region is a CH2 and/or a CH3 region said reduction is at least 30% compared to the binding of an unmodified immunoglobulin or fragment thereof.

The immunoglobulin or fragment thereof comprises an immunoglobulin constant region, which is preferably from human IGHG. The immunoglobulin constant region can comprise a CH3 region or a CH2 region, preferably, the immunoglobulin constant region comprises a CH3 and a CH2 region.

The immunoglobulin or fragment thereof may be modified in the immunoglobulin constant region to reduce binding to Protein G. Preferably, the immunoglobulin constant region comprises an amino acid substitution at a position selected from the group consisting of: 251, 252, 253, 254, 255, 311, 380, 382, 385, 387, 426, 428, 433, 434, 435, 436, 437, and 438. All positions are numbered according to the EU numbering system (Edelman GM et al., (1969) Proc Natl Acad Sci USA, 63(1): 78-85). Preferably, the immunoglobulin constant region comprises an amino acid substitution at a position selected from the group consisting of: 251, 252, 253, 254, 311, 380, 382, 426, 428, 434, 435, 436, and 438. More preferably, immunoglobulin constant region comprises an amino acid substitution selected from the group consisting of: 252A, 254M, 380A, 380M, 382A, 382L, 426M, 428G, 428S, 428T, 428V, 433D, 434A, 434G, 434S, and 438A. In one embodiment, the immunoglobulin constant region further comprises an amino acid substitution at position 250. Preferably this amino acid substitution is not 250Q. The immunoglobulin constant region may comprise an amino acid substitution at position 428 wherein this substitution is not 428L.

In one embodiment, the immunoglobulin constant region may comprise more than one amino acid substitution, for example, substitutions selected from the group consisting of: 252A/380A/382A/436A/438A, 254M/380M/382L/426M/428G and 426M/428G/433D/434A. Specifically, the immunoglobulin constant region may comprise a variant Fc fragment of human IGHG1 selected from the group consisting of: SEQ ID NO: 20, SEQ ID NO: 21 and SEQ ID NO: 22. Preferably, the immunoglobulin constant region comprises an amino acid substitution selected from 428G, 428S, 428T or 428V and a further substitution at any position within its CH2 region and/or CH3 region or alternatively, the immunoglobulin constant region comprises an amino acid substitution selected from 434A or 434S and a further substitution at any position within its CH2 region and/or CH3 region. More preferably, the amino acid substitution may be 428G with a further substitution at position 434 or alternatively, the amino acid substitution may be 434A or 434S with a further substitution at position 428. Even more preferably the amino acid substitution may be 428G with either 434A or 434S. Specifically, the immunoglobulin constant region comprises a variant Fc fragment of human IGHG1 selected from SEQ ID NO: 24 or SEQ ID NO: 25.

Besides the above described modifications in the CH2 and/or CH3 region of the immunoglobulin constant region, the immunoglobulin or fragment thereof of the present invention may also comprise a CH1 region of the immunoglobulin constant region, wherein the CH1 region is modified to result in a reduction or elimination of binding to Protein G. Preferably the CH1 region is from a human IGHG.

In one embodiment wherein the CH1 region is from a human IGHG, the CH1 region may be replaced by a CH1 region from IGHA1 or IGHM. Alternatively, CH1 strand G and part of the FG loop of the CH1 region may be replaced by a CH1 strand G and part of the FG loop of a CH1 region from IGHA1 or IGHM.

In an alternative embodiment, the CH1 region of the modified immunoglobulin constant region may comprise an amino acid substitution at a position selected from the group of: 209, 210, 213 and 214. Preferably, the amino acid substitution is at position 209 and 213. Alternatively, the modified immunoglobulin constant region may comprise amino acid substitutions selected from the group of substitutions consisting of: 209P/210S; 213V/214T; and 209G/210N. More preferably, the modified immunoglobulin constant region may comprise the amino acid modification 209G or 213V. Specifically, the immunoglobulin constant region may comprise a variant human IGHG1 CH1 region comprising amino acids 118 to 222 of SEQ ID NOS: 57, 59 or 56.

The above described substitutions have the effect of reducing binding of the immunoglobulin or fragment thereof to Protein G. The reduction of binding may be by at least a minimum of 10%. Preferably the binding to Protein G is reduced by 20%, 30%, 40%, 50%, 60%, 70%, 80% or 90%. The binding to Protein G may be reduced by up to 100%, which corresponds to elimination and this means that there is no binding at all to Protein G.

The substitutions described above may have the effect of altering the binding affinity of the immunoglobulin constant region for the human FcRn. However binding to FcRn is required for effector function and therefore loss of binding to FcRn is undesirable where effector function such as ADCC or CDC is desired.

In a further embodiment, the present invention provides an immunoglobulin or fragment thereof, comprising a polypeptide comprising an epitope-binding region and an immunoglobulin constant region wherein the immunoglobulin constant region is selected from the group consisting of: a CH2 region and a CH3 region, wherein the immunoglobulin constant region comprises a modification that reduces or eliminates binding of the immunoglobulin or fragment thereof to an affinity reagent;
wherein the modification of the immunoglobulin constant region alters the binding affinity of the immunoglobulin constant region for human FcRn; and
wherein the immunoglobulin or fragment thereof retains at least 80% binding to FcRn compared to an unmodified immunoglobulin or fragment thereof.

In an alternative embodiment, the present invention provides a hetero-dimeric immunoglobulin or fragment thereof, comprising:
(a) a first polypeptide comprising an epitope-binding region that binds a first epitope and an immunoglobulin constant region; and
(b) a second polypeptide comprising an epitope-binding region that binds a second epitope and an immunoglobulin constant region wherein the immunoglobulin constant region is selected from the group consisting of a CH2 region and a CH3;
wherein the second polypeptide comprises a modification in the immunoglobulin constant region that reduces or eliminates binding of the hetero-dimeric immunoglobulin or fragment thereof to an affinity reagent;
wherein the modification of the immunoglobulin constant region alters the binding affinity of the immunoglobulin constant region for human FcRn; and
wherein the modified second polypeptide retains at least 80% binding to FcRn compared to the binding of an unmodified hetero-dimeric immunoglobulin or fragment thereof without the modification in the immunoglobulin constant region.

The affinity reagent may bind to a binding site in the immunoglobulin constant region of the immunoglobulin or heterodimeric immunoglobulin or fragment thereof that overlaps with a binding site in the immunoglobulin constant region for human FcRn. This overlap may be partial or complete. Preferably the affinity reagent is a bacterial surface protein. More preferably, the affinity reagent is Protein G.

In one embodiment, the immunoglobulin of the second polypeptide of the hetero-dimeric immunoglobulin or fragment thereof comprises a modification in its immunoglobulin constant region that reduces or eliminates binding to Protein G. Preferably, the immunoglobulin constant region is from human IGHG. Such a modification may be an amino acid substitution in the CH3 and/or CH2 region as described herein.

In a second aspect, the present invention provides an immunoglobulin or fragment thereof, comprising a polypeptide comprising an epitope binding region having at least a VH3 region, wherein the VH3 region comprises a modification that reduces or eliminates binding of the immunoglobulin or fragment thereof to Protein A. The immunoglobulin or fragment thereof may comprise one or more additional epitope binding regions having at least a VH3 region.

The immunoglobulin or fragment thereof may be modified in the VH3 region to reduce binding to Protein A. Preferably, the VH3 region comprises an amino acid substitution at position 65 and/or an amino acid substitution selected from the group consisting of: 57A, 57E, 65S, 66Q, 68V, 81E, 82aS and combination 19G/57A/59A. All numbering of amino acid positions in the VH3 region is according to Kabat numbering (Kabat EA et al., (1991) Sequences of proteins of immunological interest. 5th Edition - US Department of Health and Human Services, NIH publication no 91, 3242 as described by Dariavach P et al., (1987) Proc Natl Acad Sci USA, 84(24): 9074-8 and Frangione B et al., (1985) Proc Natl Acad Sci USA, 82(10): 3415-9). More preferably, the modification of the VH3 region comprises an amino acid substitution selected from the group consisting of: 65S, 81E and 82aS. Even more preferably, the modification of the VH3 region comprises the amino acid substitution 65S. Most preferably, the modification of the VH region comprises the amino acid substitution 82aS. For example, SEQ ID NO: 34 is the amino acid sequence of an anti-HER2 Fab heavy chain having the substitution G65S. SEQ ID NO: 44 is the amino acid sequence of an anti-HER2 Fab- Fc heavy chain of isotype IGHG3 having the substitution G65S and the hinge region substituted for the entire hinge sequence from the naturally occurring human IGHG1 isotype. SEQ ID NO: 95 is the amino acid sequence of an anti-HER3 VH having the substitution 82aS. SEQ ID NO: 83 is the amino acid sequence of an anti-HER3 scFv having the substitution 82aS in the VH sequence.

In one embodiment, the immunoglobulin or fragment thereof may further comprise, in addition to the VH3 region, an immunoglobulin constant region. The immunoglobulin constant region may comprise at least a CH2 and/or a CH3 region. Preferably, the immunoglobulin constant region is from a human IGHG. The human IGHG may be selected from IGHG1, IGHG2 and IGHG4. In a further embodiment, where the immunoglobulin constant region comprises a CH3 region from IGHG1, IGHG2 or IGHG4, the CH3 region is replaced by a CH3 region from a human IGHG3. Specifically, the immunoglobulin region comprises a Fc region having SEQ ID NO: 2. In an alternative embodiment, where the immunoglobulin constant region comprises a CH3 region from IGHG1, IGHG2 or IGHG4, the CH3 region comprises an amino acid substitution at position 435 (EU numbering). Preferably, the amino acid substitution is 435R. Furthermore, the CH3 region may comprise an amino acid substitution at positions 435 and 436. Preferably the amino acid substitutions are 435R and 436F.

In an alternative embodiment, the present invention provides a hetero-dimeric immunoglobulin or fragment thereof, comprising:
(a) a first polypeptide comprising an epitope binding region that binds a first epitope; and
(b) a second polypeptide comprising an epitope binding region having at least a VH3 region that binds a second epitope;
wherein the VH3 region of the second polypeptide comprises a modification that reduces or eliminates binding of the hetero-dimeric immunoglobulin to Protein A.

The second polypeptide of the hetero-dimeric immunoglobulin or fragment thereof may further comprise an immunoglobulin constant region comprising a CH3 region. The CH3 region may be replaced or modified as described herein.

Alternatively, the present invention provides a hetero-dimeric immunoglobulin or fragment thereof, comprising:
(a) a first polypeptide that binds to Protein A comprising an epitope binding region that binds a first epitope and an immunoglobulin constant region; and
(b) a second polypeptide that does not bind to Protein A or has a reduced binding to protein A comprising an epitope binding region having at least a VH3 region that binds a second epitope and an immunoglobulin constant region;
wherein the VH3 region of the second polypeptide comprises a modification that reduces or eliminates binding of the second polypeptide to Protein A.

The VH3 region of the second polypeptide may comprise one or more additional epitope binding regions having at least a VH3 region. The second polypeptide of the hetero-dimeric immunoglobulin or fragment may comprise a modification in its VH3 region that reduces or eliminates binding to Protein A. Such a modification may be an amino acid substitution in the VH3 region as described above.

In a third aspect, the present invention provides a hetero-dimeric immunoglobulin or fragment thereof, comprising:
(a) a first polypeptide comprising an epitope-binding region that binds a first epitope and an immunoglobulin constant region comprising at least a CH1 and/or a CH2 and/or a CH3 region; and
(b) a second polypeptide comprising an epitope-binding region that binds a second epitope comprising at least a VH3 and/or an immunoglobulin constant region comprising at least a CH2 and/or a CH3 region;
wherein the first polypeptide comprises a modification that reduces or eliminates binding of the hetero-dimeric immunoglobulin or fragment thereof to a first affinity reagent; and wherein the second polypeptide comprises a modification that reduces or eliminates binding of the hetero-dimeric immunoglobulin or fragment thereof to a second affinity reagent.

The first affinity reagent can be Protein G and the second affinity reagent can be Protein A. Preferably, the immunoglobulin constant region is from a human IGHG. More preferably, the immunoglobulin constant region of the first polypeptide is from human IGHG and the second polypeptide is selected from IGHG1, IGHG2 or IGHG4.

Where the first affinity reagent is Protein G, the first polypeptide may comprise an immunoglobulin constant region comprising a CH3 region or a CH2 region. Preferably, the immunoglobulin constant region comprises a CH3 and a CH2 region. The immunoglobulin constant region may be modified to reduce binding to Protein G. Preferably, the modified immunoglobulin constant region comprises an amino acid substitution at a position selected from the group consisting of: 251, 252, 253, 254, 255, 311, 380, 382, 385, 387, 426, 428, 433, 434, 435, 436, 437, and 438 (EU numbering system). Preferably, the immunoglobulin constant region comprises an amino acid substitution at a position selected from the group consisting of: 251, 252, 253, 254, 311, 380, 382, 426, 428, 434, 435, 436, and 438. More preferably, immunoglobulin constant region comprises an amino acid substitution selected from the group consisting of: 252A, 254M, 380A, 380M, 382A, 382L, 426M, 428G, 428S, 428T, 428V, 433D, 434A, 434G, 434S, and 438A. In one embodiment, the immunoglobulin constant region further comprises an amino acid substitution at position 250. Preferably this amino acid substitution is not 250Q. The immunoglobulin constant region may comprise an amino acid substitution at position 428 wherein this substitution is not 428L.

In one embodiment, the immunoglobulin constant region may comprise more than one amino acid substitution, for example, substitutions selected from the group consisting of: 252A/380A/382A/436A/438A; 254M/380M/382L/426M/428G; and 426M/428G/433D/434A. Specifically, the immunoglobulin constant region may comprise a variant Fc fragment of human IGHG1 selected from the group consisting of: SEQ ID NO: 20, SEQ ID NO: 21 and SEQ ID NO: 22. Preferably, the immunoglobulin constant region comprises an amino acid substitution selected from 428G, 428S, 428T or 428V and a further substitution at any position within its CH2 region and/or CH3 region or alternatively, the immunoglobulin constant region comprises an amino acid substitution selected from 434A or 434S and a further substitution at any position within its CH2 region and/or CH3 region. More preferably, the amino acid substitution may be 428G with a further substitution at position 434 or alternatively, the amino acid substitution may be 434A or 434S with a further substitution at position 428. Even more preferably the amino acid substitution may be 428G with either 434A or 434S. Specifically, the immunoglobulin constant region comprises a variant Fc fragment of human IGHG1 selected from SEQ ID NO: 24 or SEQ ID NO: 25.

Besides the above described modifications in the CH2 and/or CH3 region of the immunoglobulin constant region of the first polypeptide, the immunoglobulin constant region may also comprise a CH1 region, wherein the CH1 region is modified to reduce or eliminate binding to Protein G. In one embodiment, the CH1 region of the immunoglobulin constant region may be replaced by a CH1 region from IGHA1 or IGHM. Alternatively, the CH1 strand G and part of the FG loop of the CH1 region are replaced by a CH1 strand G and part of the FG loop of a CH1 region from IGHA1 or IGHM.

In an alternative embodiment, the CH1 region of the modified immunoglobulin constant region may comprise an amino acid substitution at a position selected from the group of: 209, 210, 213 and 214. Preferably, the amino acid substitution is at position 209 and 213. Alternatively, the modified immunoglobulin constant region may comprise amino acid substitutions selected from the group of substitutions consisting of: 209P/210S; 213V/214T; and 209G/210N. More preferably, the modified immunoglobulin constant region may comprise the amino acid modification 209G or 213V. Specifically, the immunoglobulin constant region may comprise a variant human IGHG1 CH1 region comprising amino acids 118 to 222 of SEQ ID NOS: 57, 59 or 56.

The modifications to the immunoglobulin constant region of the first polypeptide may result in a reduction of binding of the first polypeptide of the hetero-dimeric immunoglobulin or fragment thereof to Protein G of up to 100%; alternatively, the modifications to the immunoglobulin constant region of the first polypeptide may result in elimination of binding of the first polypeptide of the hetero-dimeric immunoglobulin or fragment thereof to Protein G, when compared to the binding of an unmodified hetero-dimeric immunoglobulin or fragment thereof.

Where the second affinity reagent is Protein A, the second polypeptide may comprise a VH3 region modified to reduce binding to Protein A. Preferably, the modified VH3 region comprises an amino acid substitution at position 65 and/or an amino acid substitution selected from the group consisting of: 57A, 57E, 65S, 66Q, 68V, 81E, 82aS and combination 19G/57A/59A (Kabat numbering). More preferably, the modification of the VH3 region comprises an amino acid substitution selected from the group consisting of: 65S, 81E and 82aS. Even more preferably, the modification of the VH3 region comprises the amino acid substitution 65S. Most preferably, the modification of the VH3 regions comprises the amino acid substitution 82aS. For example, SEQ ID NO: 34 is the amino acid sequence of an anti-HER2 Fab heavy chain having the substitution G65S. SEQ ID NO: 44 is the amino acid sequence of an anti-HER2 Fab- Fc heavy chain of isotype IGHG3 having the substitution G65S and the hinge region substituted for the entire hinge sequence from the naturally occurring human IGHG1 isotype. SEQ ID NO: 95 is the amino acid sequence of an anti-HER3 VH having the substitution 82aS. SEQ ID NO: 83 is the amino acid sequence of an anti-HER3 scFv having the substitution 82aS in the VH sequence.

In addition to a modified VH3 region, the second polypeptide may comprise an immunoglobulin constant region modified to reduce binding to Protein A. The immunoglobulin constant region may comprise at least a CH2 and/or a CH3 region. Preferably, the immunoglobulin constant region is from a human IGHG, more preferably from IGHG1, IGHG2 or IGHG4. In one embodiment, where the immunoglobulin constant region comprises a CH3 region from IGHG1, IGHG2 or IGHG4, the CH3 region may be replaced by a CH3 region from a human IGHG3. In an alternative embodiment, where the immunoglobulin constant region comprises a CH3 region from IGHG1, IGHG2 or IGHG4, the CH3 region comprises an amino acid substitution at position 435 (EU numbering). Preferably, the amino acid substitution is 435R. Furthermore, the CH3 region may comprise an amino acid substitution at positions 435 and 436. Preferably the amino acid substitutions are 435R and 436F.

The modifications to the VH3 region and the immunoglobulin constant region of the second polypeptide may result in a reduction of binding of the second polypeptide of the hetero-dimeric immunoglobulin or fragment thereof to Protein A of up to 100%; alternatively, the modifications to the VH3 region and the immunoglobulin constant region of the second polypeptide may result in elimination of binding of the second polypeptide of the hetero-dimeric immunoglobulin or fragment thereof to Protein A, when compared to the binding of an unmodified hetero-dimeric immunoglobulin or fragment thereof.

In an embodiment of the present invention, the modification in the immunoglobulin constant region may result in alteration of the *in vivo* half-life of the immunoglobulin or hetero-dimeric immunoglobulin or fragments thereof. Preferably, the modification results in an increase in the *in vivo* half-life of the immunoglobulin or hetero-dimeric immunoglobulin as compared to an unmodified immunoglobulin or unmodified hetero-dimeric immunoglobulin or unmodified fragments thereof.

In a further embodiment, the modification in the immunoglobulin constant region may result in alteration of the affinity of the immunoglobulin or hetero-dimeric immunoglobulin or fragments thereof for human FcRn. Preferably, the modification results in an increase in the affinity of the immunoglobulin or hetero-dimeric immunoglobulin for FcRn when compared to an unmodified immunoglobulin or unmodified hetero-dimeric immunoglobulin or unmodified fragments thereof.

In a further embodiment, the modification in the immunoglobulin constant region may result in alteration of the binding of the immunoglobulin or hetero-dimeric immunoglobulin or fragments thereof to FcRn. Preferably, the modification results in a retention of binding of at 10% of the immunoglobulin or hetero-dimeric immunoglobulin to FcRn. More preferably, the modification results in a retention of binding of at least 20%, 30%, 40%, 50%, 60% or 70% of the immunoglobulin or hetero-dimeric immunoglobulin to FcRn. Even more preferably, the modification results in a retention of binding of at least 75%, 80%, 85%, 90%, 95% or 99% of the immunoglobulin or hetero-dimeric immunoglobulin to FcRn, as compared to an unmodified immunoglobulin or unmodified hetero-dimeric immunoglobulin or unmodified fragments thereof. Measurement of the binding retention to FcRn can be made using Surface Plasmon Resonance as described in Example 4.

In a further embodiment, the modification in the immunoglobulin constant region may impact on the specificity or affinity of the immunoglobulin or hetero-dimeric immunoglobulin or fragments thereof for FcyR3a. Preferably, the modification has little or no impact on specificity or affinity of the immunoglobulin or hetero-dimeric immunoglobulin for FcyR3a. More preferably, the modification has little or no impact on specificity or affinity of the immunoglobulin or hetero-dimeric immunoglobulin for FcyR3a, as compared to an unmodified immunoglobulin or unmodified hetero-dimeric immunoglobulin or unmodified fragments thereof. Measurement of the binding specificity or affinity for FcyR3a can be made using Surface Plasmon Resonance as described in Example 4.

In a further embodiment, the modification in the immunoglobulin constant region and/or the VH3 region may result in immunogenicity of the immunoglobulin or hetero-dimeric immunoglobulin and can induce an anti-drug antibody response in humans. Preferably, the modification results in only low or no immunogenicity of the immunoglobulin or hetero-dimeric immunoglobulin and therefore presents a low immunogenic potential or risk. Predictions of the immunogenic potential of the modifications used in the present invention can be made using the methods described in Example 5.

In a further embodiment, the modification in the immunoglobulin constant region and/or the VH3 region may alter the thermo-stability of the immunoglobulin or hetero-dimeric immunoglobulin. Preferably the modification to abrogate Protein G binding has a low impact on the thermo-stability of the immunoglobulin or hetero-dimeric immunoglobulin. Preferably the modification to abrogate Protein A binding has a low impact or no impact on the thermo-stability of the immunoglobulin or hetero-dimeric immunoglobulin. Thermo-stability of the immunoglobulins or hetero-dimeric immunoglobulins modified according to the present invention can be analysed as described in Example 6.

In a further embodiment, the modification in the immunoglobulin constant region may impact on the serum half-life of the immunoglobulin or hetero-dimeric immunoglobulin. Preferably, the modification has little or no impact on serum half-life of the immunoglobulin or hetero-dimeric immunoglobulin. More preferably, the modification results in a reduction in serum half-life of less than 30%, 25%, 20%, 15%, 10% or 5%. Most preferably the modification results in a reduction in serum half-life of less than 20%. Pharmacokinetics of the immunoglobulin or hetero-dimeric immunoglobulin can be measured as described in Example 7.

The immunoglobulins or hetero-dimeric immunoglobulins of the present invention as described herein, may also comprise a light chain. Preferably, the immunoglobulin comprises a heavy and light chain having antigen binding capability determined previously, i.e. the immunoglobulin binds to a known antigen. More preferably, the immunoglobulin comprises a common light chain i.e. a light chain that can pair with different heavy chains. Therefore in a hetero-dimeric immunoglobulin, for example, two different heavy chains may be paired with a common light chain (a light chain having identical variable and constant regions). Common light chains may be identified using a variety of methods. These methods may include selecting the most frequently used light chain variable region from an antibody display library displaying, for example, light chain variable sequences or scFv antibody fragments such as a phage display library. Alternatively, both heavy chain variable region sequences of the hetero-dimeric immunoglobulin can be used as probes in the library to identify a light chain that associates with both heavy chain variable regions.

In a further aspect, the present invention provides methods for the selective purification of hetero-dimeric immunoglobulins.

A first embodiment provides a method for the purification of a hetero-dimeric immunoglobulin or fragment thereof comprising the steps:
(i) isolating from a mixture of immunoglobulins a hetero-dimeric immunoglobulin or fragment thereof comprising one modified heavy chain, wherein the modified heavy chain comprises a modification in a CH1 and/or a CH2 and/or a CH3 region of an immunoglobulin constant region and wherein the modification reduces or eliminates binding of the hetero-dimeric immunoglobulin to Protein G;
(ii) applying the mixture of immunoglobulins to Protein G; and
(iii) eluting the hetero-dimeric immunoglobulin or fragment thereof from Protein G.

Also provided is an affinity chromatography method for the purification of hetero-dimers of immunoglobulin heavy chains, comprising the steps:
(i) modifying one of the heavy chains in a CH1 and/or a CH2 and/or a CH3 region to reduce or eliminate binding to Protein G;
(ii) expressing separately or co-expressing both heavy chains;
(iii) applying the co-expressed heavy chains or previously assembled separately expressed heavy chains to Protein G; and
(iv) eluting the hetero-dimers of heavy chains from Protein G.

Also provided is an affinity chromatography method for the purification of hetero-dimers of immunoglobulin heavy chains or fragments thereof comprising at least one CH1 region and one CH2 and/or CH3 region, comprising the steps:
(i) modifying one of the heavy chains in the CH2 and/or CH3 region to reduce or eliminate binding to Protein G;
(iia) if only one CH1 region is present within the hetero-dimer, said CH1 region is part of the unmodified heavy chain that retains binding to protein G, or said CH1 region is modified to reduce or eliminate binding to Protein G; or
(iib) if two or more CH1 regions are present within the hetero-dimer, all except one CH1 region is modified to reduce or eliminate binding to protein G, and the unmodified CH1 region is part of the unmodified heavy chain that retains binding to protein G; or all CH1 regions are modified to reduce or eliminate binding to Protein G;
(iii) expressing separately or co-expressing the heavy chains;
(iv) applying the co-expressed heavy chains or previously assembled separately expressed heavy chains to Protein G; and
(v) eluting the hetero-dimers of heavy chains or fragments thereof from Protein G.

The modified heavy chains as described in these methods can comprise the modifications in an immunoglobulin constant region that reduce or eliminate binding to protein G, as described herein.

A second embodiment provides a method for the purification of a hetero-dimeric immunoglobulin or fragment thereof comprising a VH3 region, comprising the steps:
(i) isolating from a mixture of immunoglobulins a hetero-dimeric immunoglobulin or fragment thereof comprising one modified heavy chain, wherein the modified heavy chain comprises a modification in a VH3 region or in a VH3 region and an immunoglobulin constant region and wherein the modification reduces or eliminates binding of the hetero-dimeric immunoglobulin or fragment thereof to Protein A;
(ii) applying the mixture of immunoglobulins to Protein A; and
(iii) eluting the hetero-dimeric immunoglobulin or fragment thereof from Protein A.

Also provided is an affinity chromatography method for the purification of hetero-dimers of immunoglobulin heavy chains or fragment thereof wherein at least one VH3 region is present, comprising the steps:
(i) modifying one of the heavy chains to reduce or eliminate binding to Protein A;
(iia) if only one VH3 region is present within the hetero-dimer, said VH3 region is part of the unmodified heavy chain that retains binding to Protein A, or said VH3 region is modified to reduce or eliminate binding to Protein A; or
(iib) if two or more VH3 regions are present within the hetero-dimer, all except one VH3 region is modified to reduce or eliminate binding to Protein A, and the unmodified VH3 region is part of the unmodified heavy chain that retains binding to Protein A; or all VH3 regions are modified to reduce or eliminate binding to Protein A;
(iii) expressing separately or co-expressing the two heavy chains;
(iv) applying the co-expressed heavy chains or previously assembled separately expressed heavy chains to Protein A; and
(v) eluting the hetero-dimers of heavy chains or fragments thereof from Protein A.

The modified VH3 region(s) or modified VH3 and immunoglobulin constant regions as described in these methods can comprise the modifications that reduce or eliminate binding to protein A, as described herein.

A third embodiment provides a method for the differential purification of hetero-dimers of heavy chains comprising:
(i) isolating from a mixture of heavy chains a hetero-dimer of heavy chains or fragments thereof having a first heavy chain comprising a modification that reduces or eliminates binding to a first affinity reagent and having a second heavy chain comprising a modification that reduces or eliminates binding to a second affinity reagent;
(ii) applying the mixture of heavy chains to a first column comprising the first affinity reagent;
(iii) eluting the hetero-dimers of heavy chain from the first column;
(iv) applying the eluate from the first column to a second column comprising the second affinity reagent; and
(v) eluting the hetero-dimers of heavy chains or fragments thereof from the second column.

In this method the first and second affinity reagent is derived from a bacterial surface protein. Where the first affinity reagent is Protein A, the second affinity reagent is Protein G or where the first affinity reagent is Protein G, the second affinity reagent is Protein A. The modified heavy chains as described in this method can comprise modifications that reduce or eliminate binding to Protein A and Protein G, as described herein.

The hetero-dimer may be purified to greater than 70% purity. Preferably, the hetero-dimer is purified to greater than 80% or 90% purity. More preferably the hetero-dimer is purified to greater than 95% purity. Even more preferably the hetero-dimer is purified to greater than 98% purity.

A further aspect of the present invention provides a method for isolating an immunoglobulin of interest or fragment thereof from a mixture of immunoglobulins comprising:
(i) isolating the immunoglobulin of interest or fragment thereof from a mixture of immunoglobulins, wherein the immunoglobulin of interest or fragment thereof is eliminated in all its binding sites for Protein A and/or Protein G;
(ii) applying the mixture of immunoglobulins in a first step to Protein A or Protein G;
(iii) collecting the unbound immunoglobulin of interest or fragment thereof from step (ii); and optionally
(iv) applying the unbound immunoglobulin of interest or fragment thereof from step (iii) to Protein A or Protein G; and
(v) collecting the unbound immunoglobulin of interest or fragment thereof from step (iv); wherein in step (ii) the mixture of immunoglobulins is applied to Protein A and in step (iv) the mixture of immunoglobulins is applied to Protein G; or
wherein in step (ii) the mixture of immunoglobulins is applied to Protein G and in step (iv) the mixture of immunoglobulins is applied to Protein A.

In the immunoglobulin of interest or fragment thereof, the binding sites for Protein A are located in VH3 and immunoglobulin constant region. The binding sites for Protein G are located in the immunoglobulin constant region.

In one embodiment, the immunoglobulin of interest or fragment thereof may be a homo-dimeric immunoglobulin. In an alternative embodiment, the immunoglobulin of interest or fragment thereof may be a hetero-dimeric immunoglobulin.

Preferably, the immunoglobulin of interest can be a hetero-dimeric immunoglobulin, more preferably a bispecific hetero-dimeric immunoglobulin or fragment thereof or a bispecific full-length antibody which binds to antigens selected from within the groups of: tumor antigens, cytokines, vascular growth factors and lympho-angiogenic growth factors. Preferably the antigens are selected from the group consisting of: HER1, HER2, HER3, EGFR, CD3, CD19, CD20, EpCAM, IgE and VLA-2. Preferably the antigens are HER2 and HER3, CD3 and EpCAM, CD3 and HER2, CD19 and IgE and CD20 and IgE.

In a preferred embodiment the hetero-dimeric immunoglobulin is a bispecific hetero-dimeric immunoglobulin comprising a HER3 epitope binding region. Preferably, the HER3 epitope binding region comprises a heavy chain CDR1 comprising the amino acid sequence of SEQ ID NO: 88, a heavy chain CDR2 comprising the amino acid sequence of SEQ ID NO: 89 and a heavy chain CDR3 comprising the amino acid sequence of SEQ ID NO: 90. Preferably, the HER3 epitope binding region comprises a light chain CDR1 comprising the amino acid sequence of SEQ ID NO: 91, a light chain CDR2 comprising the amino acid sequence of SEQ ID NO: 92 and a light chain CDR3 comprising the amino acid sequence of SEQ ID NO: 93. More preferably, the HER3 epitope binding region comprises a heavy chain CDR1 comprising the amino acid sequence of SEQ ID NO: 88, a heavy chain CDR2 comprising the amino acid sequence of SEQ ID NO: 89, a heavy chain CDR3 comprising the amino acid sequence of SEQ ID NO: 90, a light chain CDR1 comprising the amino acid sequence of SEQ ID NO: 91, a light chain CDR2 comprising the amino acid sequence of SEQ ID NO: 92 and a light chain CDR3 comprising the amino acid sequence of SEQ ID NO: 93. Even more preferably, the hetero-dimeric immunoglobulin is a bispecific hetero-dimeric immunoglobulin and binds HER3, wherein the HER3 binding region comprises the heavy chain sequence of SEQ ID NO: 86 and light chain sequence of SEQ ID NO: 85. Equally more preferably, the hetero-dimeric immunoglobulin is a bispecific hetero-dimeric immunoglobulin and binds HER3, wherein the HER3 binding region comprises the heavy chain variable region sequence of SEQ ID NO: 95 and light chain variable region sequence of SEQ ID NO: 82.

In a preferred embodiment the hetero-dimeric immunoglobulin is a bispecific hetero-dimeric immunoglobulin which binds HER2 and HER3, comprising a heavy chain having an amino acid sequence of SEQ ID NO: 86 and a light chain having an amino acid sequence of SEQ ID NO: 85. More preferably, the hetero-dimeric immunoglobulin is a bispecific hetero-dimeric immunoglobulin and binds HER2 and HER3, having a first heavy chain amino acid sequence of SEQ ID NO: 87, a second heavy chain amino acid sequence of SEQ ID NO: 86 and a light chain amino acid sequence of SEQ ID NO: 85.

A method for isolating an immunoglobulin of interest as described herein may be useful in medical applications, particularly diagnostics. Isolating an immunoglobulin of interest from patient serum in order to determine the amount of immunoglobulin of interest in the serum is not a straightforward process. In one embodiment, the mixture of immunoglobulins comprises or is derived from serum from a patient or animal that has been administered the immunoglobulin of interest or fragment thereof. In an alternative embodiment, the mixture of immunoglobulins is patient or animal serum wherein, the patient or animal has been administered the immunoglobulin of interest or fragment thereof.

Abrogation of the binding sites for Protein A and/or Protein G may be achieved by modifying the immunoglobulin of interest or fragment thereof in its VH3 and/or immunoglobulin constant region according to the modifications described herein.

In a preferred embodiment of the present invention, the purification methods of the hetero-dimeric immunoglobulins as described herein can be combined with known techniques in the art for optimising the interaction of the Fc regions or more specifically the CH3 regions of hetero-dimeric immunoglobulins.

For example, the first report of an engineered CH3 hetero-dimeric domain pair was made by Carter *et al.* describing a "protuberance-into-cavity" approach for generating a hetero-dimeric Fc moiety (U.S. Patent No. 5,807,706; "knobs-into-holes"; Merchant AM et al., 1988 Nat. Biotechnol., 16(7): 677-81). In this method, one or more small amino acid side chains from the interface of the first antibody molecule are replaced with larger side chains (e.g. tyrosine or tryptophan) to give a "protuberance". Compensatory "cavities" of identical or similar size to the large side chain(s) are created on the interface of the second antibody molecule by replacing large amino acid side chains with smaller ones (e.g. alanine or threonine). Alternative designs have been recently developed and involved either the design of a new CH3 module pair by modifying the core composition of the modules as described in WO07/110205 (Davis JH & Huston JS) or the design of complementary salt bridges between modules as described in WO07/147901 (Kjærgaard K et al.) or WO09/089004 (Kannan G et al.)*.* Preferably, the hetero-dimeric immunoglobulins for use in the present invention comprise engineered immunoglobulin constant regions as described in PCT publication No: WO13/131555 (Blein S et al.)*.*

### Brief Description of the Figures

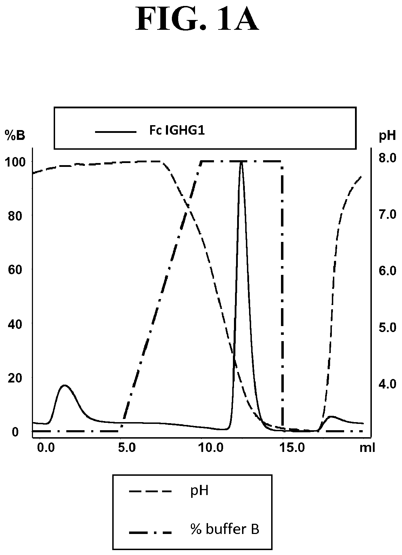

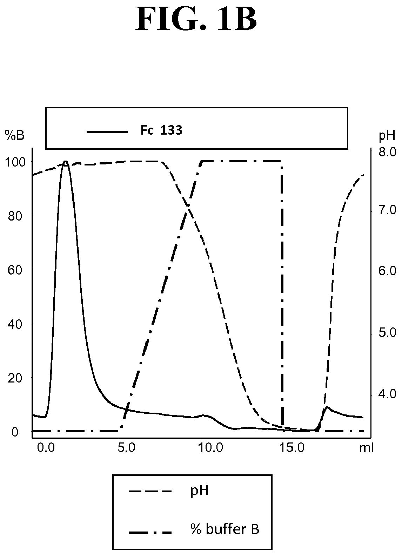

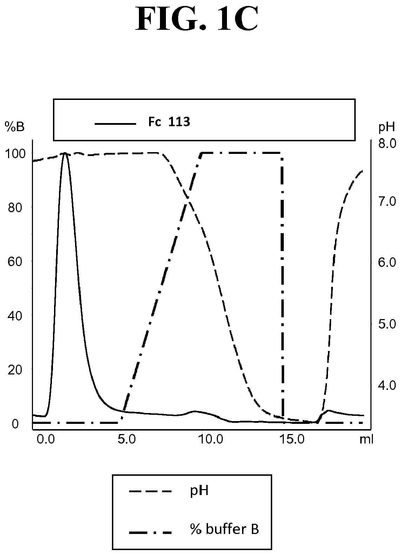

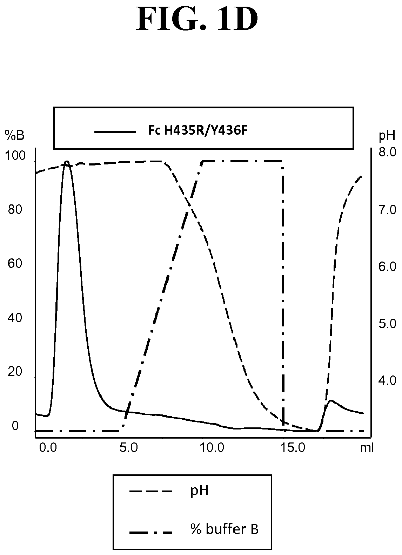

**FIG. 1A-D**: Protein A gradient mode chromatography traces (HiTrap™ MabSelect SuRe™ Protein A column). Plots of absorbance at 280 nm *vs*. total volume of mobile phase are shown as solid line. Plots of mobile phase pH and percentage of eluent buffer (B) present in mobile phase are shown as dashed and dotted-dashed lines, respectively. FIG. 1A: Fc IGHG1. FIG. 1B: Fc 133. FIG. 1C: Fc 113. FIG. 1D: Fc H435R/Y436F.

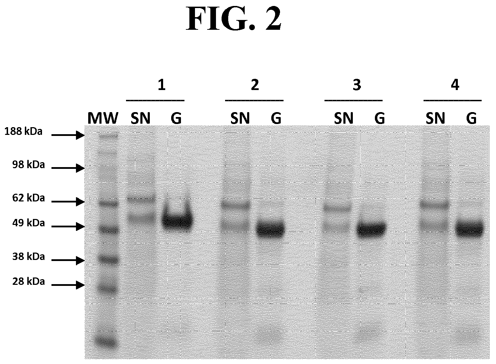

**FIG. 2****:** SDS-PAGE analysis of Protein G capture-elution mode chromatography fractions (Protein G Sepharose™ 4 Fast Flow resin). (1) Fc IGHG1. (2) Fc 113. (3) Fc 133. (4) Fc H435R/Y436F. (MW) molecular weight markers as indicated. (SN) cell culture supernantant. (G) elution from Protein G.

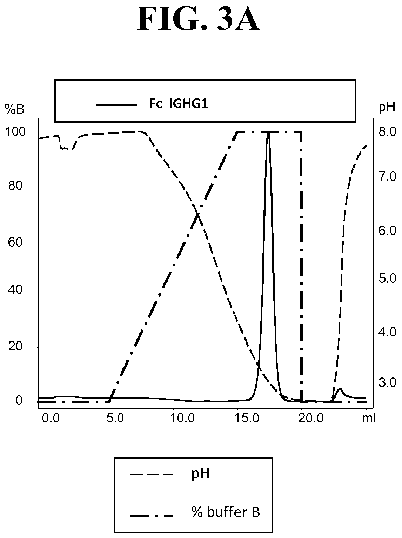

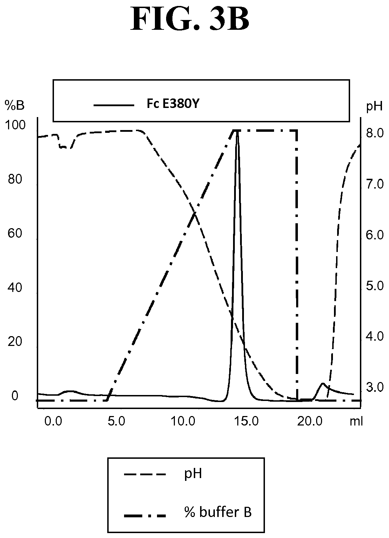

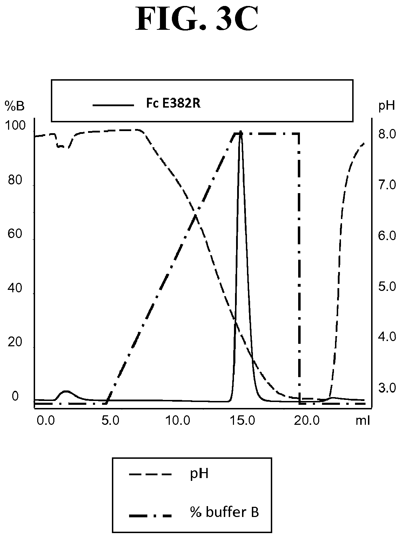

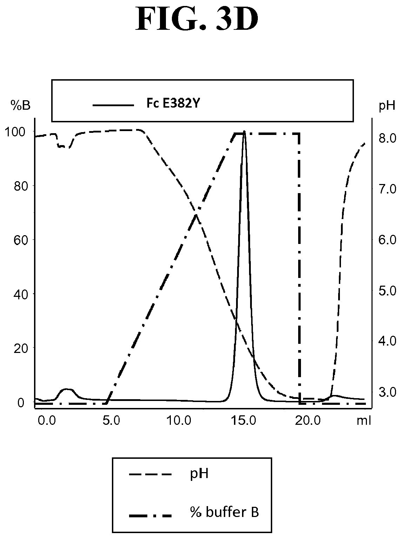

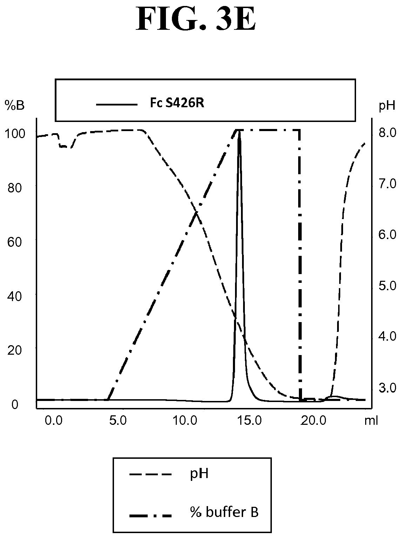

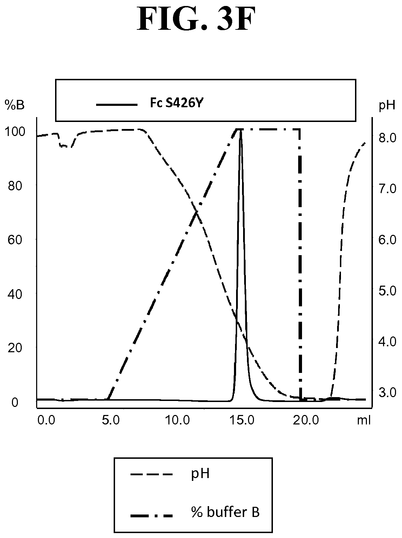

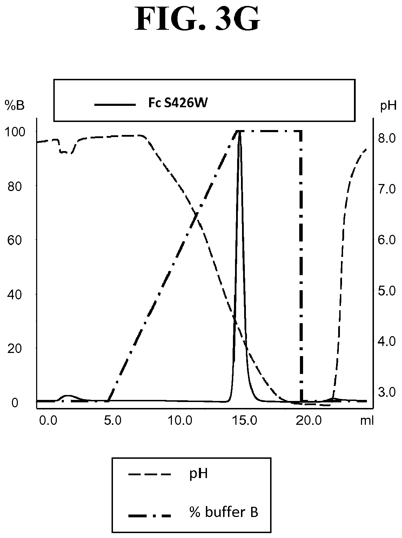

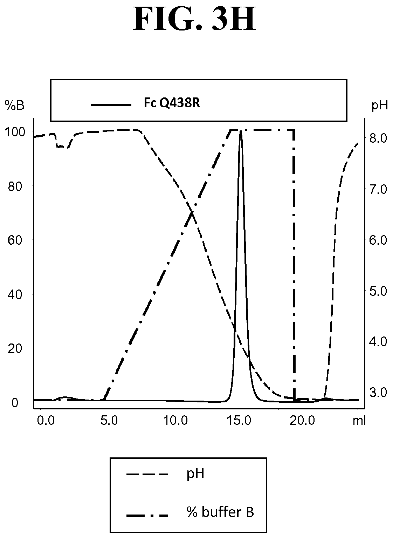

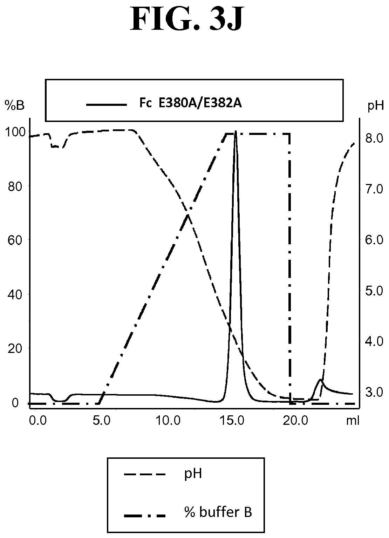

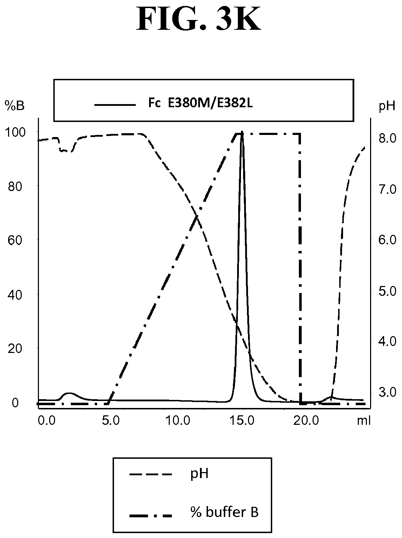

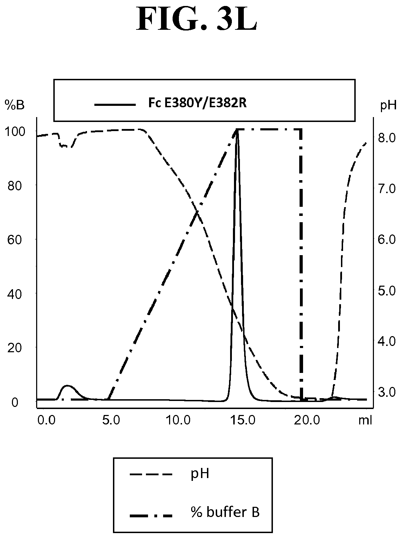

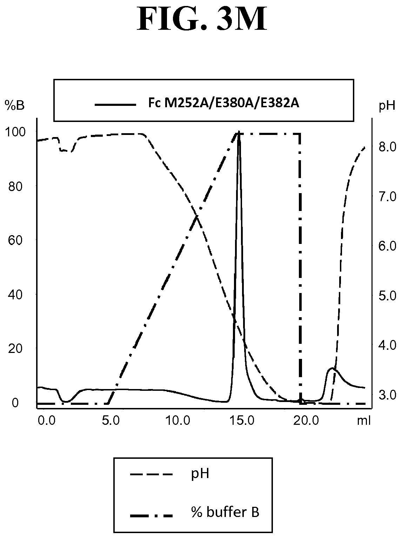

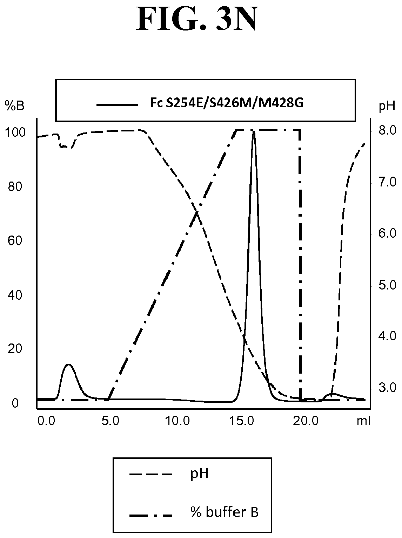

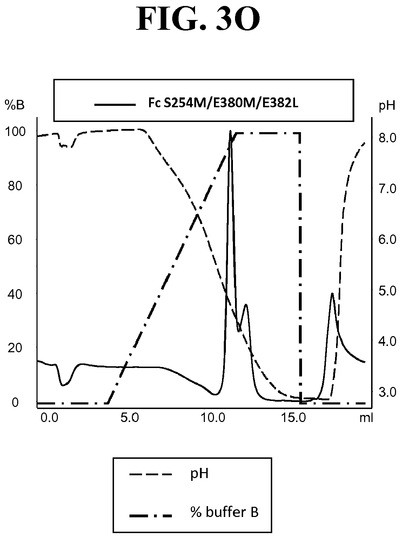

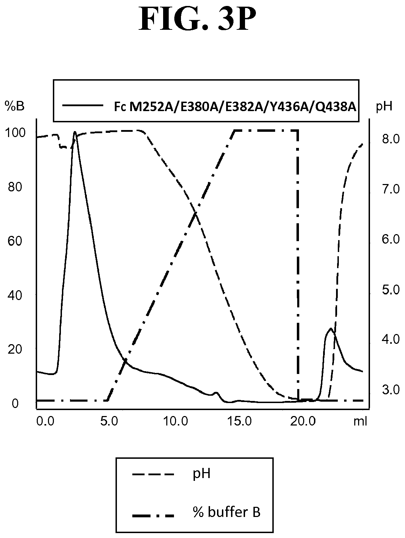

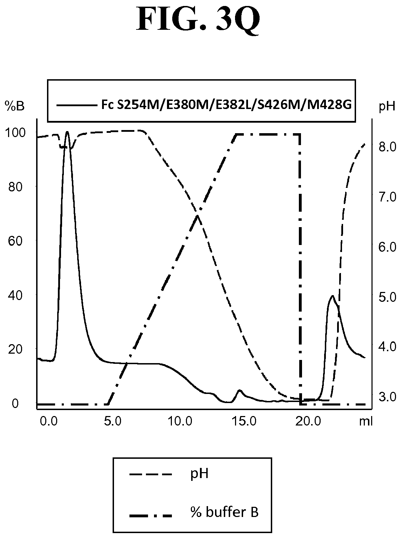

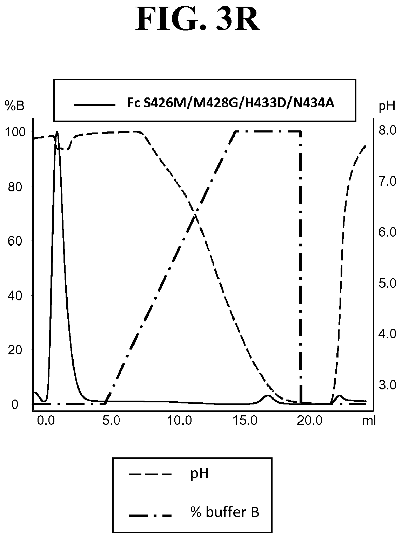

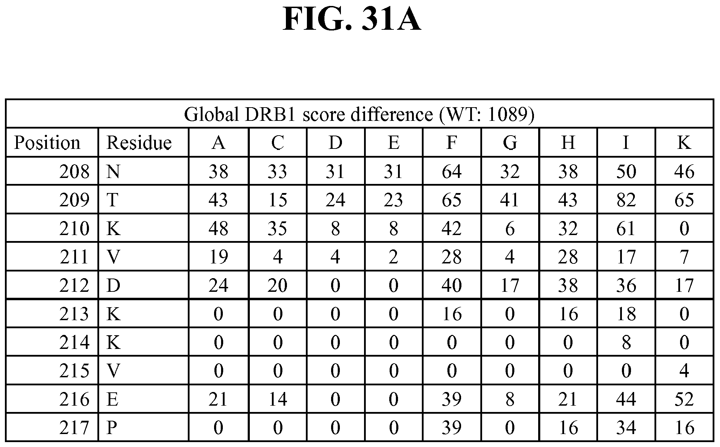

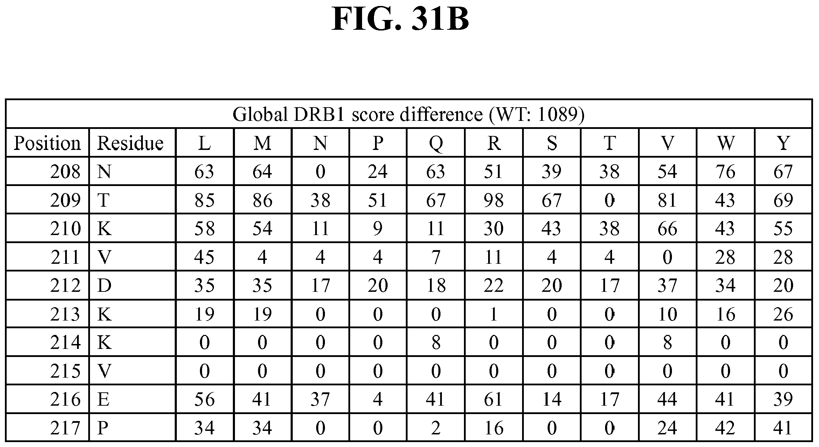

**FIG. 3A****-R:** Protein G gradient mode chromatography traces (HiTrap™ Protein G HP column). Plots of absorbance at 280 nm *vs*. total volume of mobile phase are shown as solid line. Plots of mobile phase pH and percentage of eluent buffer (B) present in mobile phase are shown as dashed and dotted-dashed lines, respectively. FIG. 3A: Fc IGHG1. FIG. 3B: Fc E380Y. FIG. 3C: Fc E382R. FIG. 3D: Fc E382Y. FIG. 3E: Fc S426R. FIG. 3F: Fc S426Y. FIG. 3G: Fc S426W. FIG. 3H: Fc Q438R. FIG. 31: Fc Q438Y. FIG. 3J: Fc E380A/E382A. FIG. 3K: Fc E380M/E382L. FIG. 3L: Fc E380Y/E382R. FIG. 3M: Fc M252A/E380A/ E382A. FIG. 3N: Fc S254E/S426M/M428G. FIG. 3O: Fc S254M/E380M/E382L. FIG. 3P: Fc M252A/E380A/E383A/Y436A/Q438A. FIG. 3Q: Fc S254M/E380M/E382L/S426M/ M428G. FIG. 3R: Fc S426M/M428G/H433D/N434A.

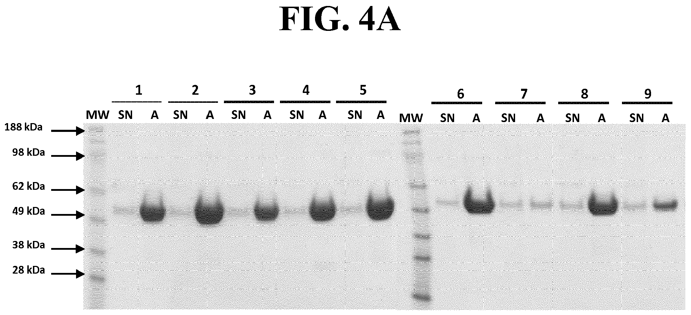

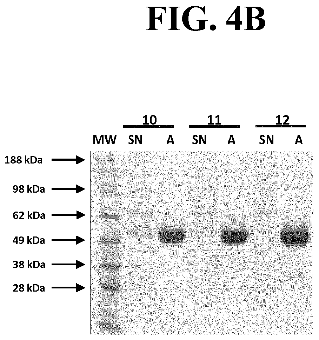

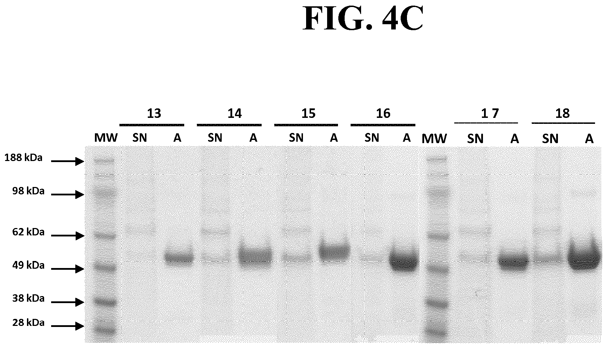

**FIG. 4A****-C:** SDS-PAGE analysis of Protein A capture-elution mode chromatography fractions (MabSelect SuRe™ Protein A resin). FIG. 4A: (1) Fc IGHG1, (2) Fc E380Y, (3) Fc E382R, (4) Fc E382Y, (5) Fc E380Y/E382R, (6) Fc Q438R, (7) Fc S426W, (8): Fc S426R, and (9) Fc S426Y. FIG. 4B: (10) Fc Q438Y, (11) Fc S254E/S426M/M428G, and (12) Fc E380M/E382L. FIG. 4C: (13) Fc S254M/E380M/E382L, (14) Fc E380A/E382A, (15) Fc M252A/E380A/E382A, (16) Fc S254M/E380M/E382L/S426M/M428G, (17) Fc M252A/E380A/E382A/Y436A/Q438A, and (18) Fc S426M/M428G/H433D/N434A. FIG. 4A-C: (MW) molecular weight markers as indicated. (SN) cell culture supernatant. (A) elution from Protein A.

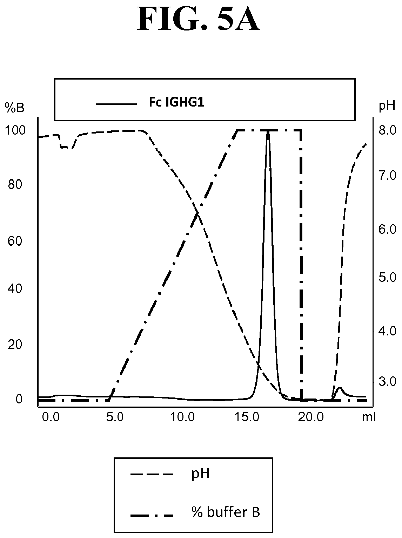

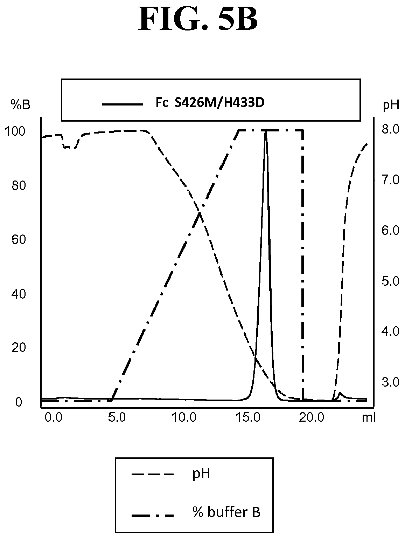

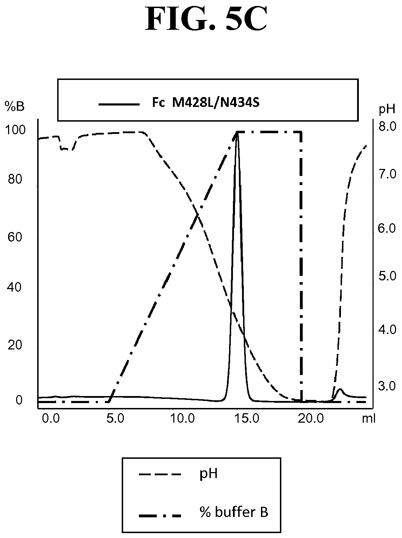

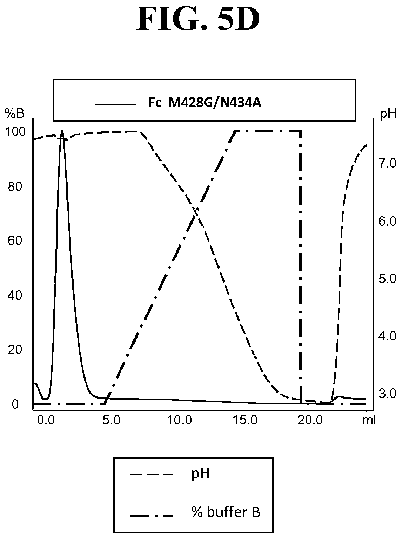

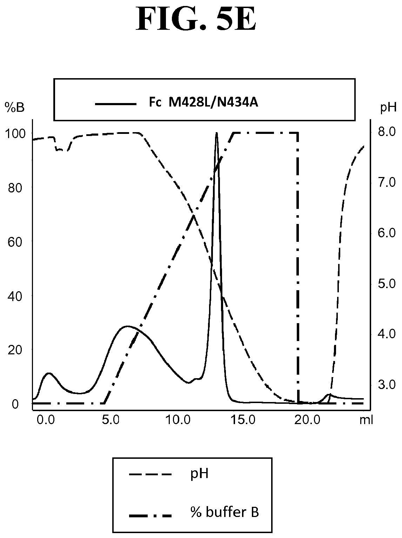

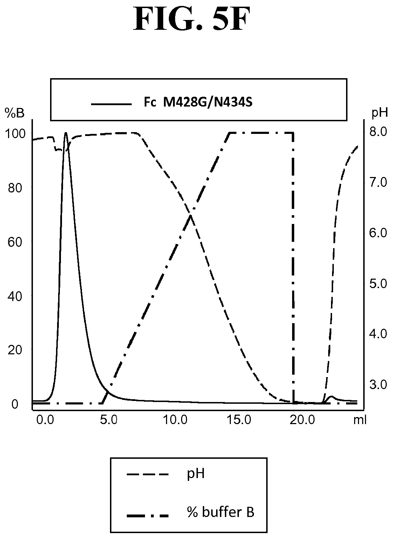

**FIG. 5A****-F:** Protein G gradient mode chromatography traces (HiTrap™ Protein G HP column). Plots of absorbance at 280 nm vs. total volume of mobile phase are shown as solid line. Plots of mobile phase pH and percentage of eluent buffer (B) present in mobile phase are shown as dashed and dotted-dashed lines, respectively. FIG. 5A: Fc IGHG1. FIG. 5B: Fc S426M/H433D. FIG. 5C: Fc M428L/N434S. FIG. 5D: Fc M428G/N434A. FIG. 5E: Fc M428L/N434A. FIG. 5F: M428G/N434S.

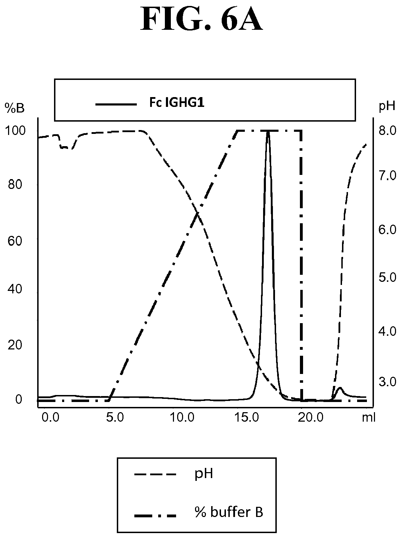

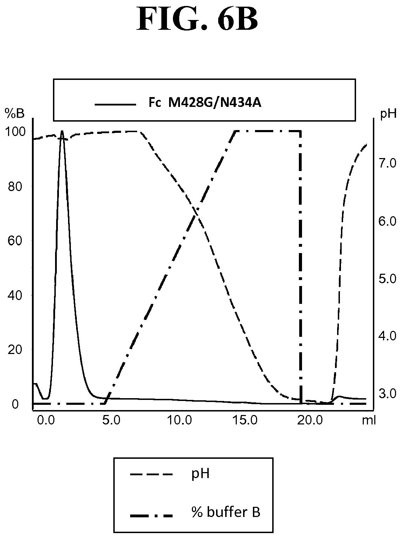

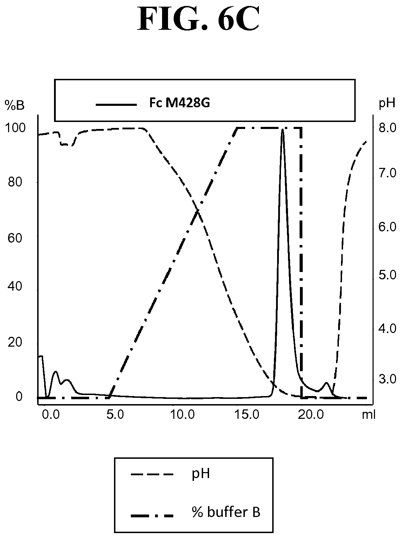

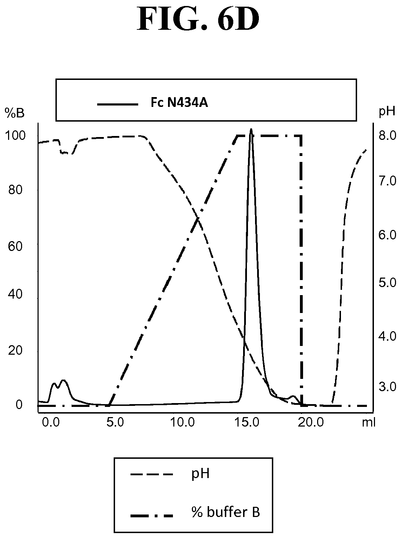

**FIG. 6A****-D:** Protein G gradient mode chromatography traces (HiTrap™ Protein G HP column). Plots of absorbance at 280 nm *vs*. total volume of mobile phase are shown as solid line. Plots of mobile phase pH and percentage of eluent buffer (B) present in mobile phase are shown as dashed and dotted-dashed lines, respectively. FIG. 6A: Fc IGHG1. FIG. 6B: Fc M428G /N434A. FIG. 6C: Fc M428G. FIG. 6D: Fc N434A.

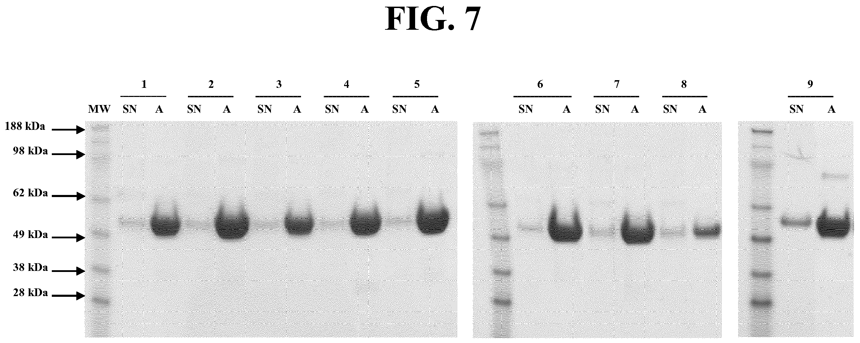

**FIG. 7****:** SDS-PAGE analysis of Protein A capture-elution mode chromatography fractions (MabSelect SuRe™ Protein A resin). (1) Fc IGHG1. (2) Fc M428G/N434A. (3) Fc S426M/M428G/ H433D/N434A. (4) Fc M248L/N434S. (5) Fc M428G/N434S. (6) Fc M248L/N434A. (7) Fc S426M/H433D. (8) Fc M248G. (9) Fc N434A. (MW) molecular weight markers as indicated. (SN) cell culture supernantant. (A) elution from Protein A.

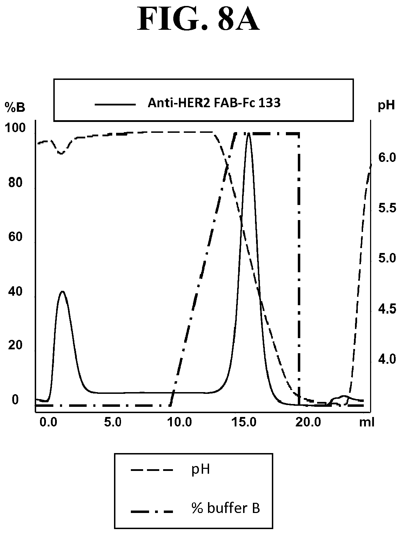

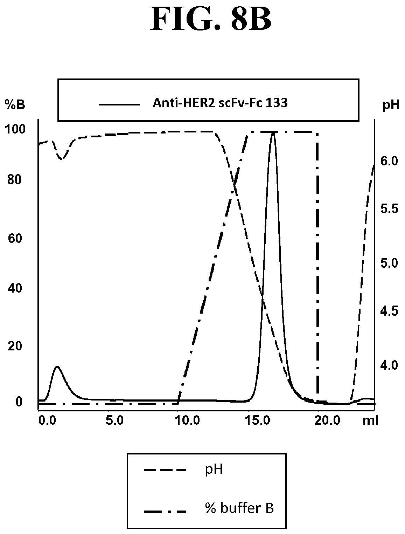

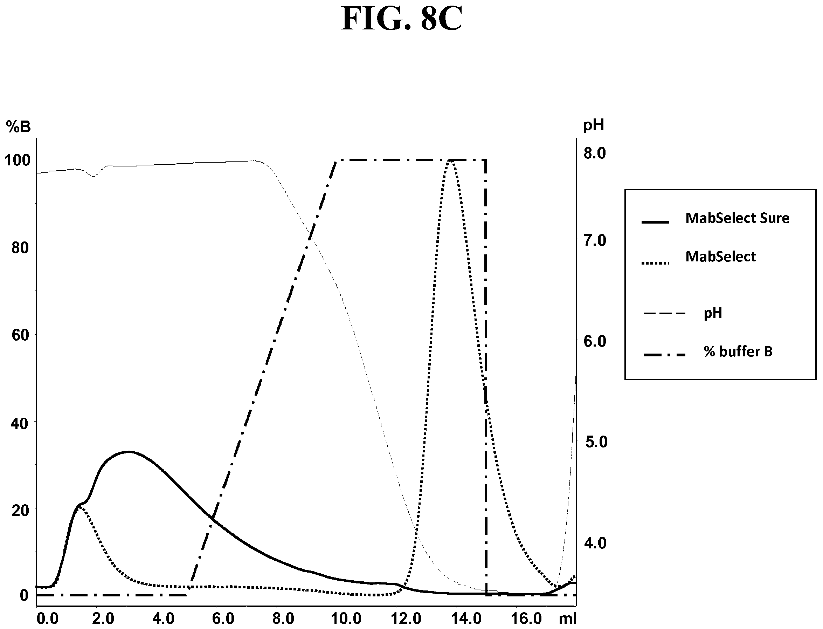

**FIG. 8A****-C:** Protein A gradient mode chromatography traces. Plots of absorbance at 280 nm *vs*. total volume of mobile phase are shown as solid line. Plots of mobile phase pH and percentage of eluent buffer (B) present in mobile phase are shown as dashed and dotted-dashed lines, respectively. FIG. 8A: anti-HER2 FAB-Fc 133 (HiTrap™ MabSelect SuRe™ Protein A column). FIG. 8B: anti-HER2 scFv-Fc 133 (HiTrap™ MabSelect SuRe™ Protein A column). FIG. 8C: anti-HER2 FAB (HiTrap™ MabSelect SuRe™ Protein A column and HiTrap™ MabSelect™ Protein A column).

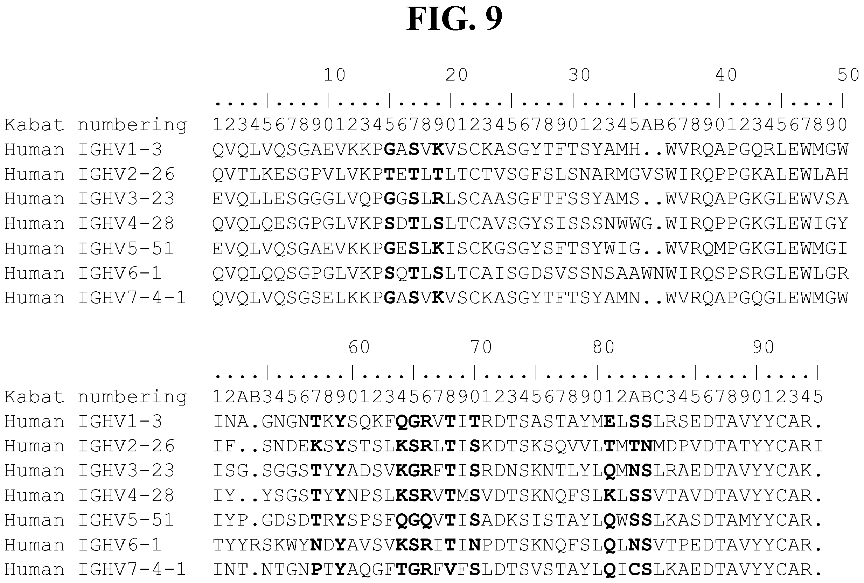

**FIG. 9****:** Representative amino acid sequences for each of the seven known human VH framework subclasses. Sequences were aligned according to the Kabat numbering. Positions interacting with the domain D of Protein A are shown in bold.

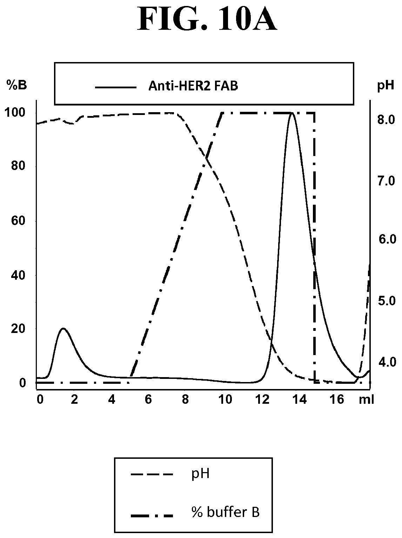

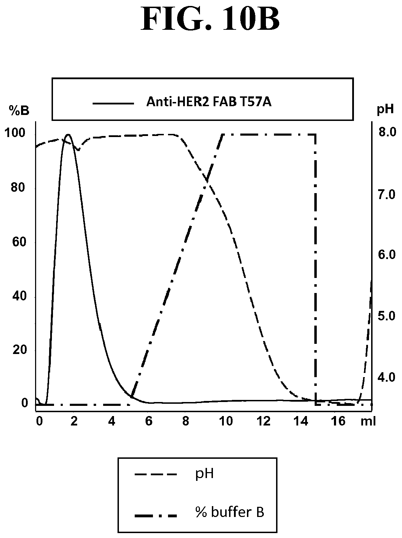

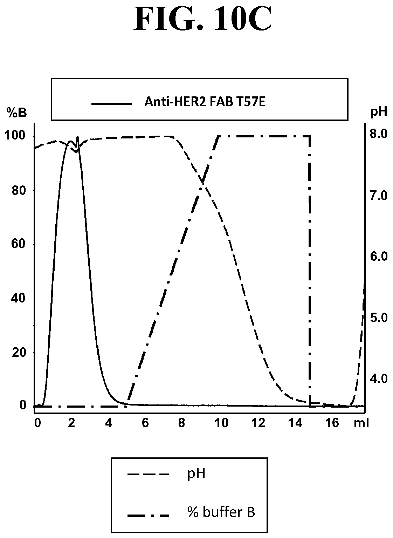

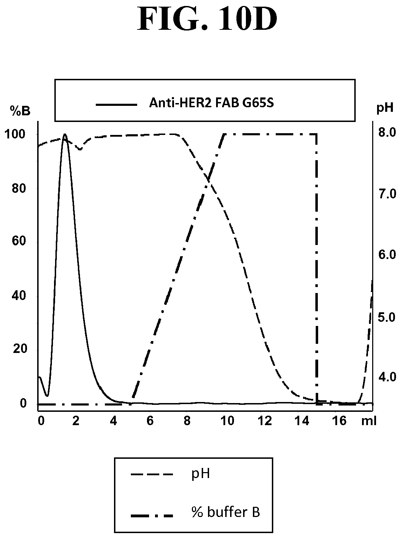

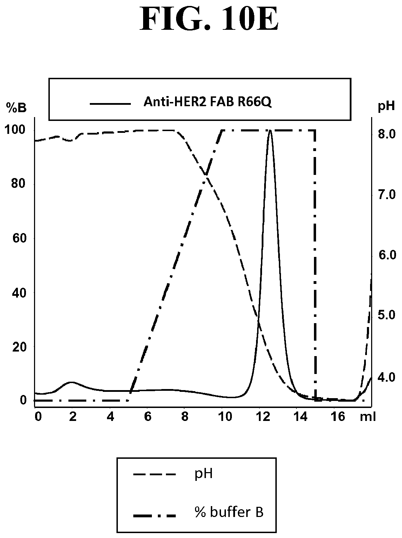

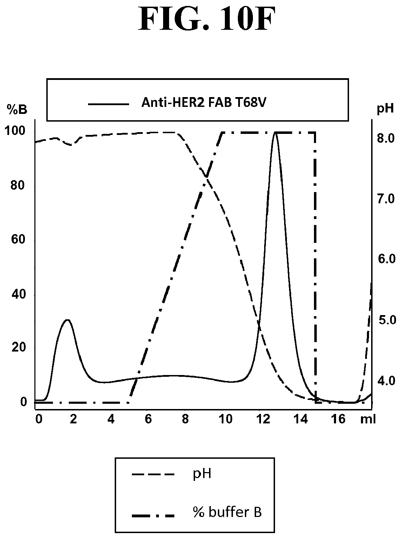

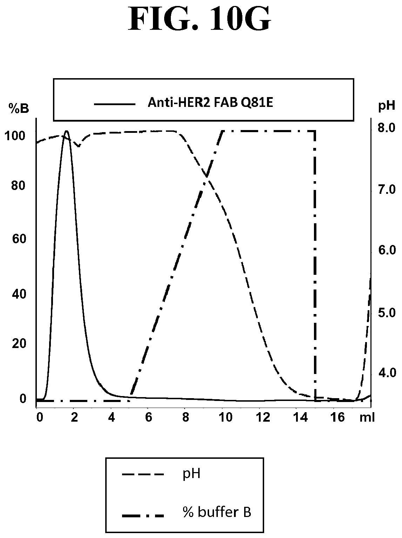

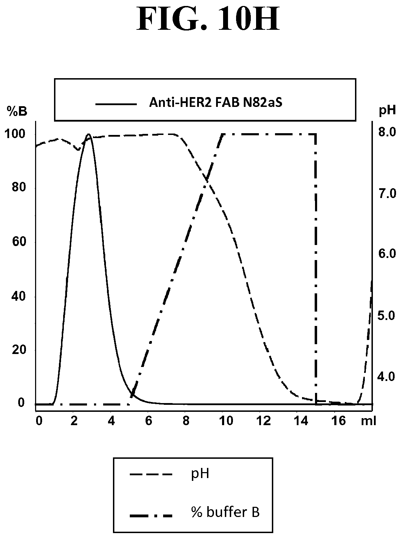

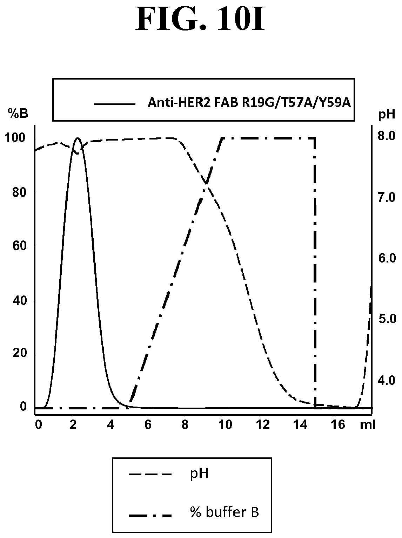

**FIG. 10A****-I:** Protein A gradient mode chromatography traces (HiTrap™ MabSelect™ Protein A column). Plots of absorbance at 280 nm vs. total volume of mobile phase are shown as solid line. Plots of mobile phase pH and percentage of eluent buffer (B) present in mobile phase are shown as dashed and dotted-dashed lines, respectively. FIG. 10A: anti-HER2 FAB. FIG. 10B: anti-HER2 FAB T57A. FIG. 10C: anti-HER2 FAB T57E. FIG. 10D: anti-HER2 FAB G65S. FIG. 10E: anti-HER2 FAB R66Q. FIG. 10F: anti-HER2 FAB T68V. FIG. 10G: anti-HER2 FAB Q81E. FIG. 10H: anti-HER2 FAB N82aS. FIG. 10I: anti-HER2 FAB R19G/T57A/Y59A.

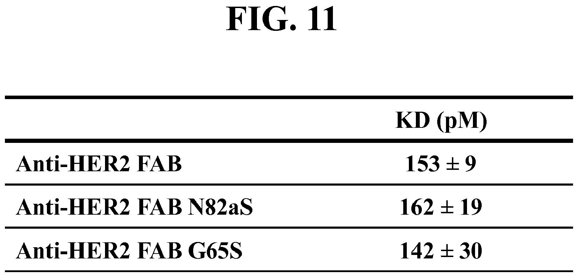

**FIG. 11****:** Equilibrium dissociation constants (KD) of selected anti-HER2 FAB variants for the HER2 antigen.

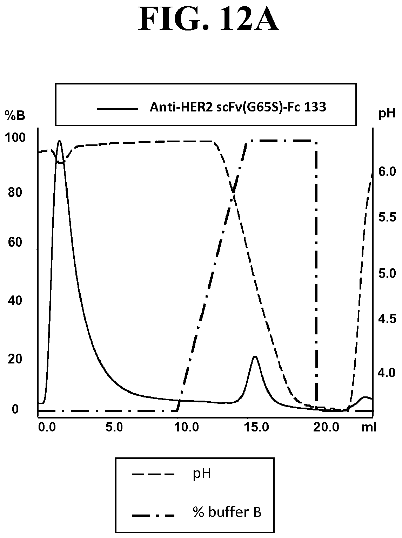

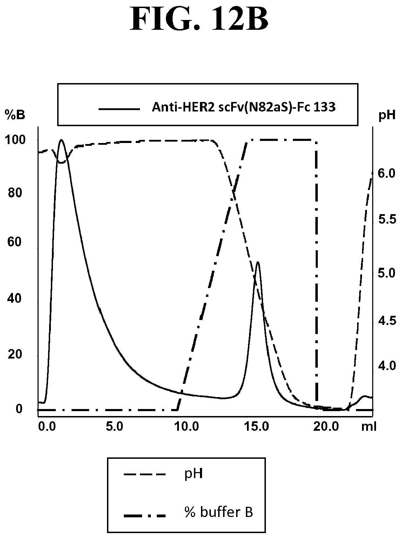

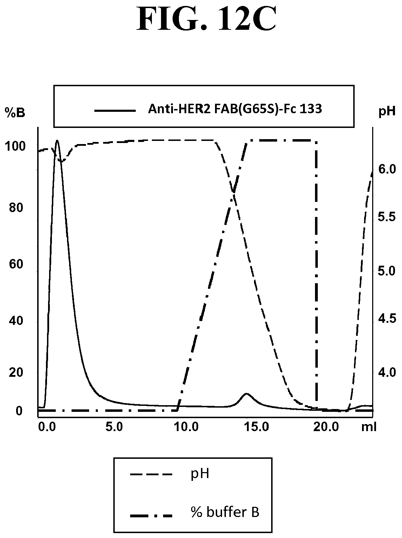

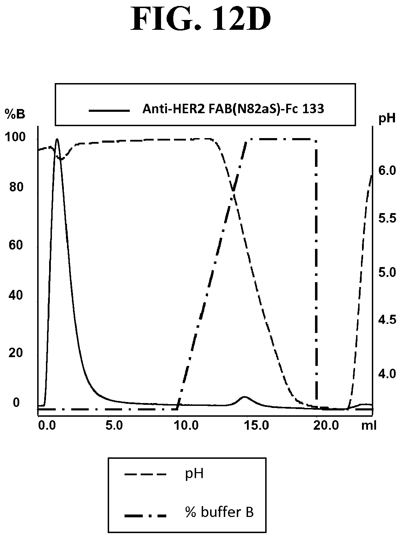

**FIG. 12A****-D:** Protein A gradient mode chromatography traces (HiTrap™ MabSelect SuRe™ Protein A column). Plots of absorbance at 280 nm *vs*. total volume of mobile phase are shown as solid line. Plots of mobile phase pH and percentage of eluent buffer (B) present in mobile phase are shown as dashed and dotted-dashed lines, respectively. FIG. 12A: anti-HER2 scFv(G65S)-Fc 133. FIG. 12B: anti-HER2 scFv(N82aS)-Fc 133. FIG. 12C: anti-HER2 FAB(G65S)-Fc 133. FIG. 12D: anti-HER2 FAB(N82aS)-Fc 133.

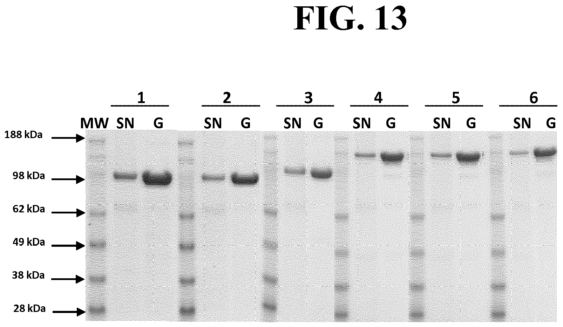

**FIG. 13****:** SDS-PAGE analysis of Protein G capture-elution mode chromatography fractions (Protein G Sepharose™ 4 Fast Flow resin). (1) anti-HER2 scFv(N82aS)-Fc 133. (2) anti-HER2 scFv(G65S)-Fc 133. (3) anti-HER2 scFv-Fc 133. (4) anti-HER2 FAB(G65S)-Fc 133. (5) anti-HER2 FAB(N82aS)-Fc 133. (6) anti-HER2 FAB-Fc 133. (MW) molecular weight markers as indicated. (SN) cell culture supernantant. (G) elution from Protein G.

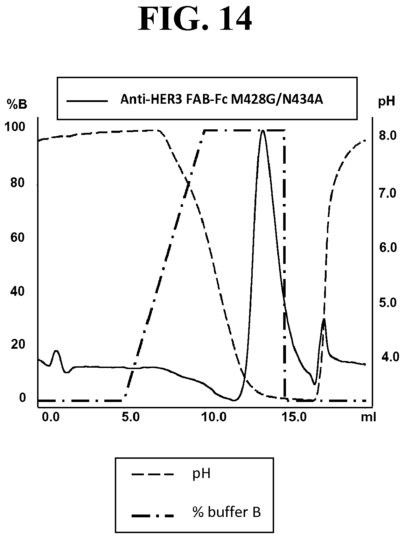

**FIG. 14****:** Protein G gradient mode chromatography traces of anti-HER3 FAB-Fc M428G/N434A (HiTrap™ Protein G HP column). Plot of absorbance at 280 nm *vs*. total volume of mobile phase is shown as solid line. Plots of mobile phase pH and percentage of eluent buffer (B) present in mobile phase are shown as dashed and dotted-dashed lines, respectively.

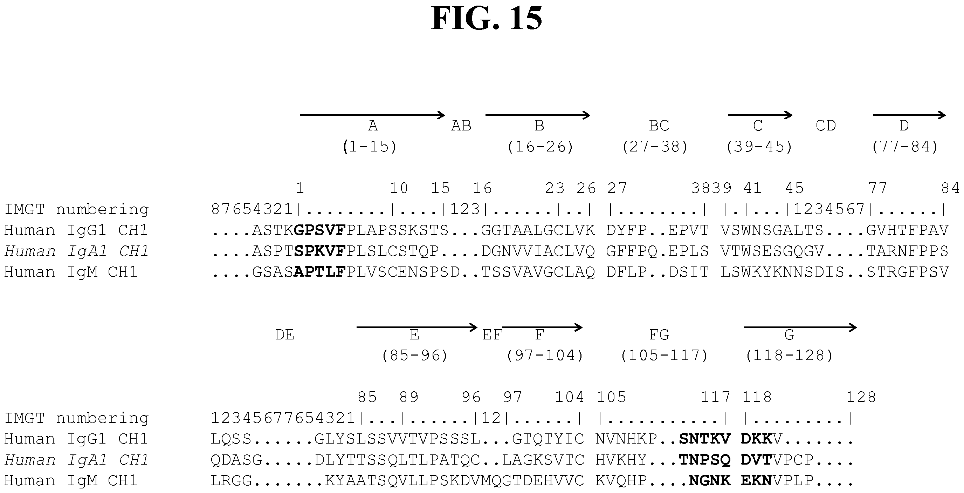

**FIG. 15****:** Sequences of human IGHM, IGHA1 and IGHG1 CH1 domains; the IMGT® numbering is used. Residues involved in the binding to domain III of Protein G are shown in bold.

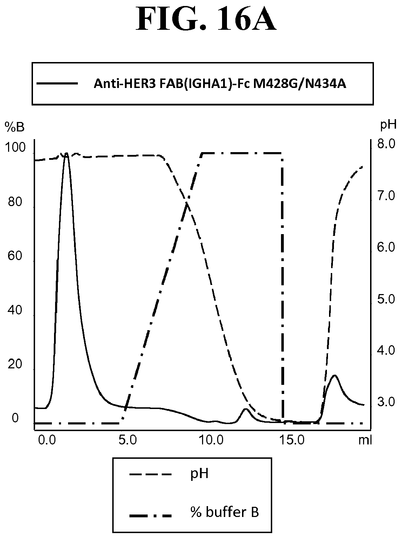

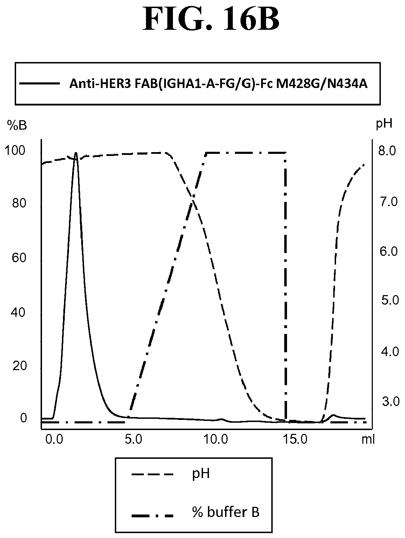

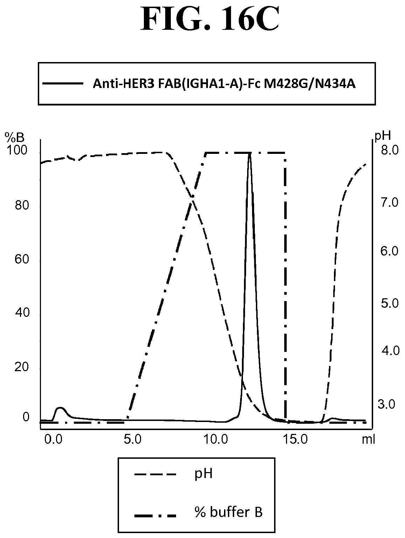

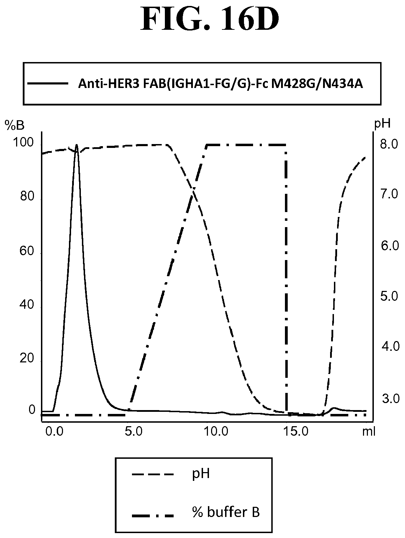

**FIG. 16A****-D:** Protein G gradient mode chromatography traces (HiTrap™ Protein G HP column). Plots of absorbance at 280 nm vs. total volume of mobile phase are shown as solid line. Plots of mobile phase pH and percentage of eluent buffer (B) present in mobile phase are shown as dashed and dotted-dashed lines, respectively. FIG. 16A: anti-HER3 FAB(IGHA1)-Fc M428G/N434A. FIG. 16B: anti-HER3 FAB(IGHA1-A-FG/G)-Fc M428G/N434A. FIG. 16C: anti-HER3 FAB(IGHA1-A)-Fc M428G/N434A. FIG. 16D: anti-HER3 FAB(IGHA1-FG/G)-Fc M428G/N434A.

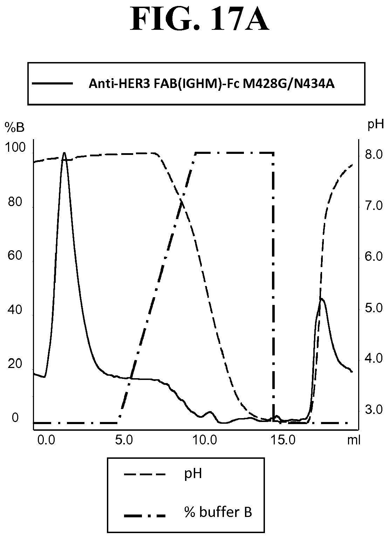

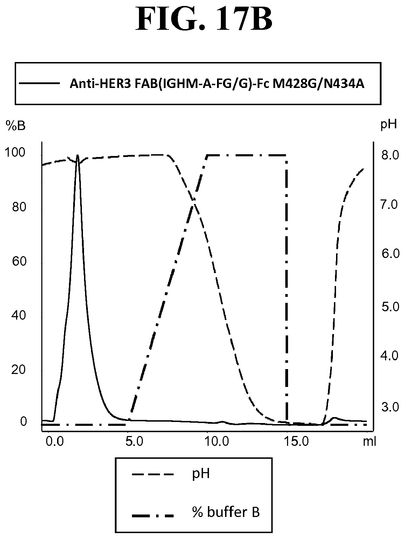

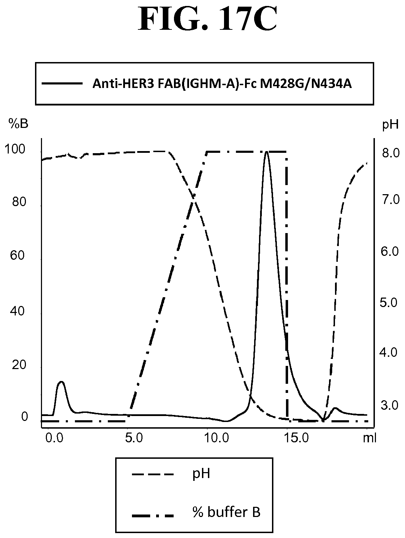

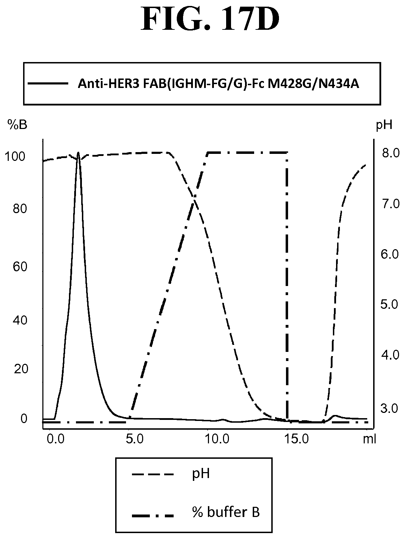

**FIG. 17A****-D:** Protein G gradient mode chromatography traces (HiTrap™ Protein G HP column). Plots of absorbance at 280 nm vs. total volume of mobile phase are shown as solid line. Plots of mobile phase pH and percentage of eluent buffer (B) present in mobile phase are shown as dashed and dotted-dashed lines, respectively. FIG. 17A: anti-HER3 FAB(IGHM)-Fc M428G/N434A. FIG. 17B: anti-HER3 FAB(IGHM-A-FG/G)-Fc M428G/N434A. FIG. 17C: anti-HER3 FAB(IGHM-A)-Fc M428G/N434A. FIG. 17D: anti-HER3 FAB(IGHM-FG/G)-Fc M428G/N434A.

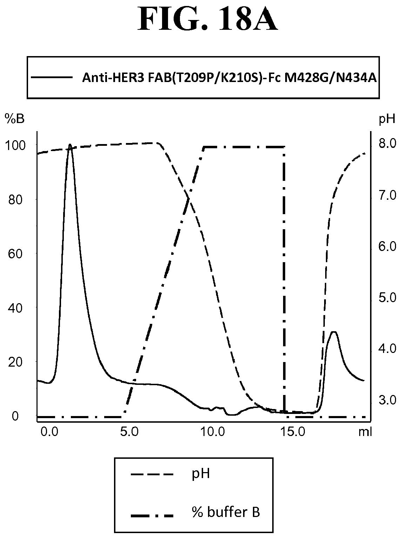

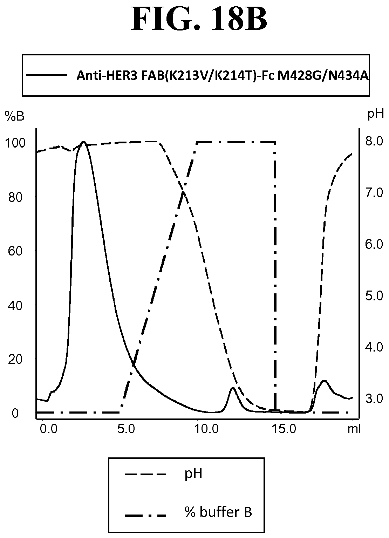

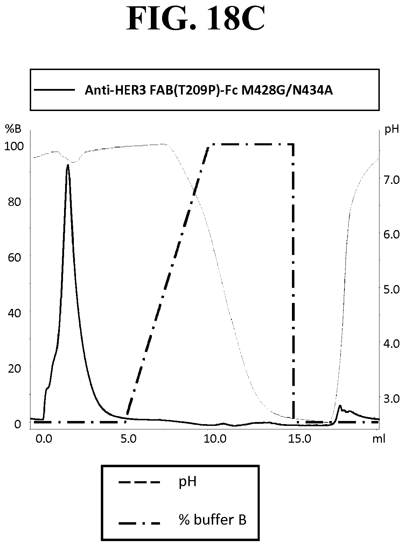

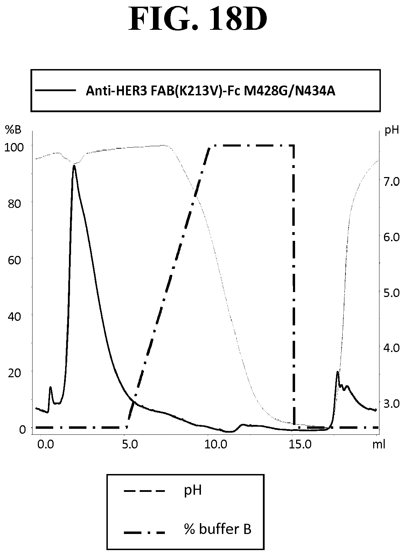

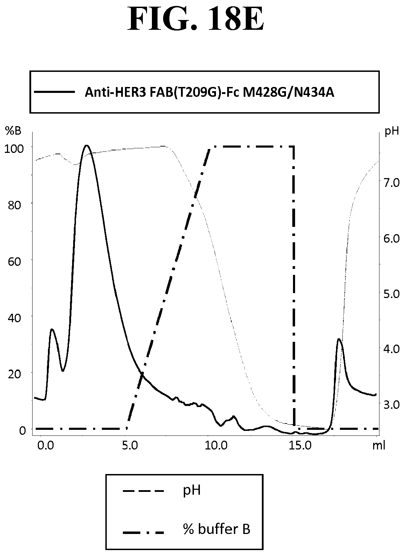

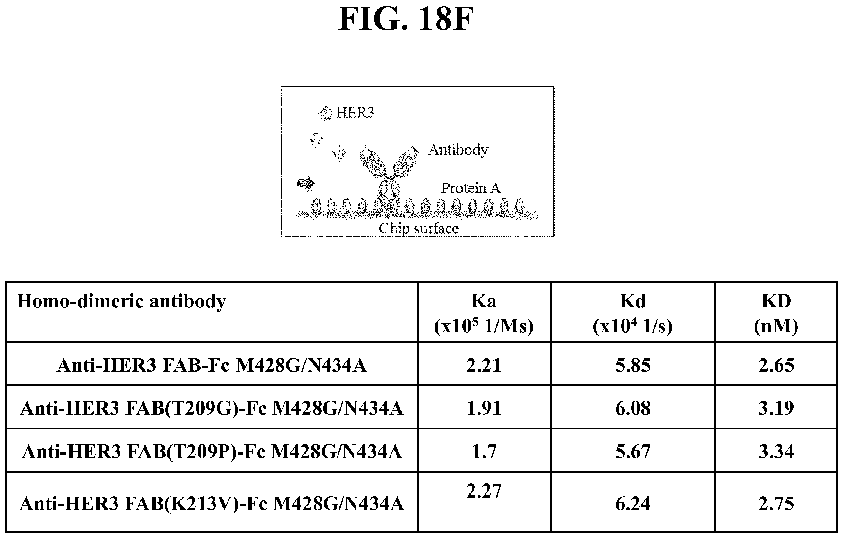

**FIG. 18A****-E:** Protein G gradient mode chromatography traces (HiTrap™ Protein G HP column). Plots of absorbance at 280 nm vs. total volume of mobile phase are shown as solid line. Plots of mobile phase pH and percentage of eluent buffer (B) present in mobile phase are shown as dashed and dotted-dashed lines, respectively. FIG. 18A: anti-HER3 FAB(T209P/K210S)-Fc M428G/N434A. FIG. 18B: anti-HER3 FAB(K213V/K214T)-Fc M428G/N434A. FIG. 18C: anti-HER3 FAB(T209P)-Fc M428G/N434A. FIG. 18D: Anti-HER3 FAB(K213V)-Fc M428G/N434A. FIG. 18E: Anti-HER3 FAB(T209G)-Fc M428G/N434A. FIG. 18F: Determination of the KD measurement for the anti-HER3 antibody variants.

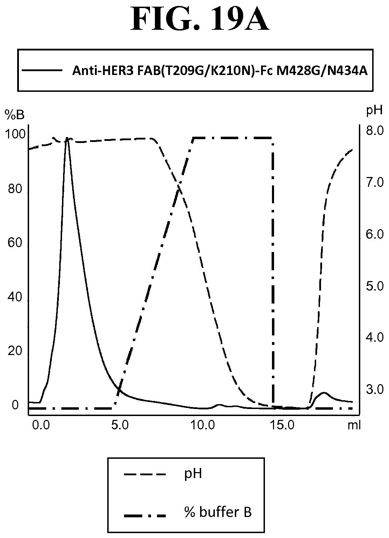

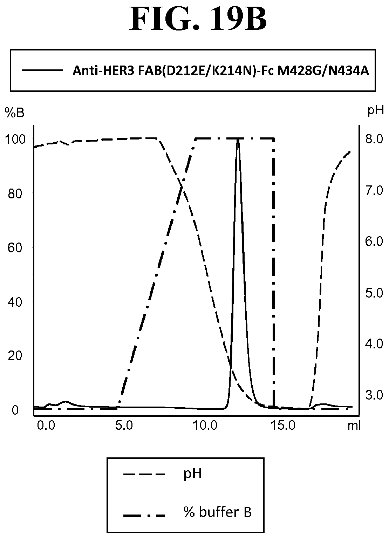

**FIG. 19A****-B:** Protein G gradient mode chromatography traces (HiTrap™ Protein G HP column). Plots of absorbance at 280 nm vs. total volume of mobile phase are shown as solid line. Plots of mobile phase pH and percentage of eluent buffer (B) present in mobile phase are shown as dashed and dotted-dashed lines, respectively. FIG. 19A: anti-HER3 FAB(T209G/K210N)-Fc M428G/N434A. FIG. 19B: anti-HER3 FAB(D212E/K214N)-Fc M428G/N434A.

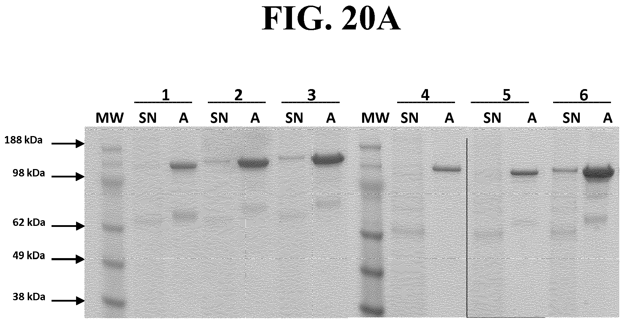

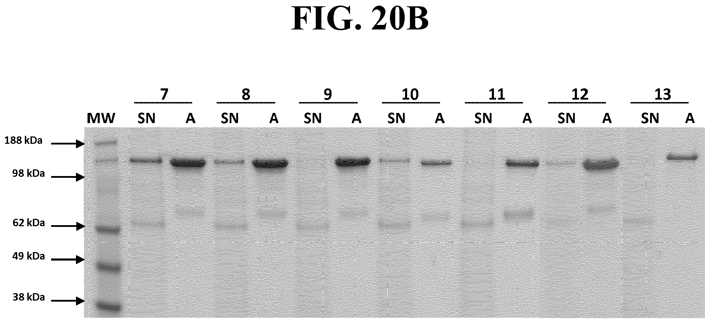

**FIG. 20****:** SDS-PAGE analysis of Protein A capture-elution mode chromatography fractions (MabSelect SuRe™ Protein A resin). FIG. 20A: (1) anti-HER3 FAB-Fc M428G/N434A, (2) anti-HER3 FAB(IGHA1)-Fc M428G/N434A, (3) anti-HER3 FAB(IGHM)-Fc M428G/N434A, (4) anti-HER3 FAB(IGHA1-A-FG/G)-Fc M428G/N434A, (5) anti-HER3 FAB(IGHA1-FG/G)-Fc M428G/N434A, and (6) anti-HER3 FAB(IGHA1-A)-Fc M428G/N434A. FIG. 20B: (7) anti-HER3 FAB(IGHM-A-FG/G)-Fc M428G/N434A, (8) anti-HER3 FAB(IGHM-FG/G)-Fc M428G/N434A, (9) anti-HER3 FAB(IGHM-A)-Fc M428G/N434A, (10) anti-HER3 FAB(K213V/K214T)-Fc M428G/N434A, (11) anti-HER3 FAB(T209G/K210N)-Fc M428G/N434A, (12) anti-HER3 FAB(T209P/K210S)-Fc M428G/N434A, and (13) anti-HER3 FAB(D212E/K214N)-Fc M428G/N434A. FIG. 20A & FIG. 20 B: (MW) molecular weight markers as indicated. (SN) cell culture supernatant. (A) elution from Protein A.

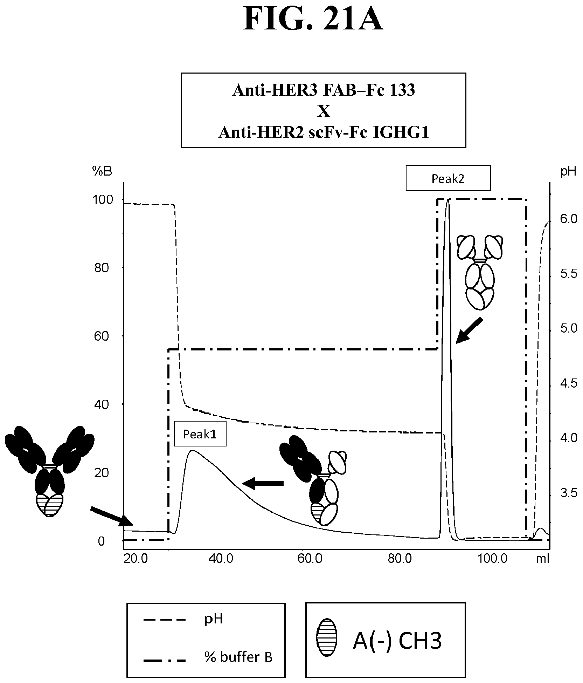

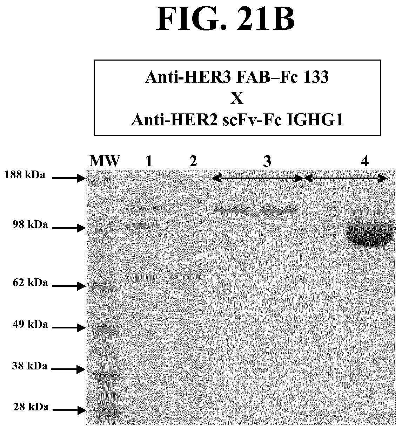

**FIG. 21A****:** Protein A gradient mode chromatography trace of anti-HER3 FAB-Fc 133 x anti-HER2 scFv-Fc IGHG1 hetero-dimer (HiTrap™ MabSelect SuRe™ Protein A column). Plot of absorbance at 280 nm vs. total volume of mobile phase is shown as solid line. Plots of mobile phase pH and percentage of eluent buffer (B) present in mobile phase are shown as dashed and dotted-dashed lines, respectively. **FIG. 21B****:** SDS-PAGE analysis of chromatography fractions from trace shown in FIG. 21A. (MW) molecular weight marker as indicated. (1) cell culture supernatant. (2) flow-through. (3) peak 1. (4) peak 2.

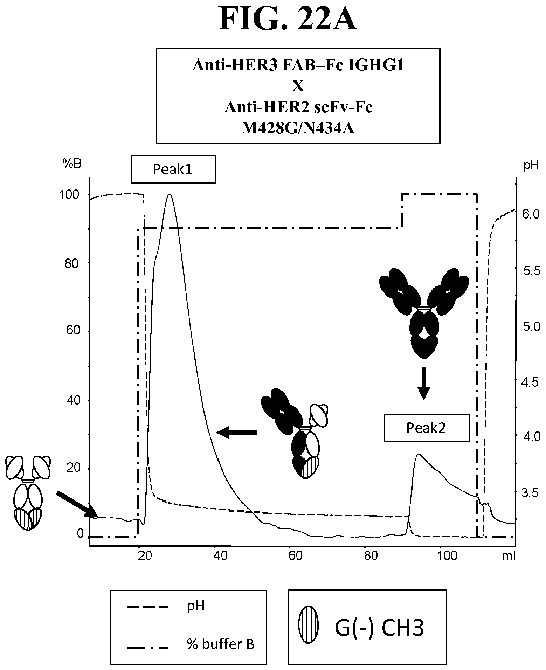

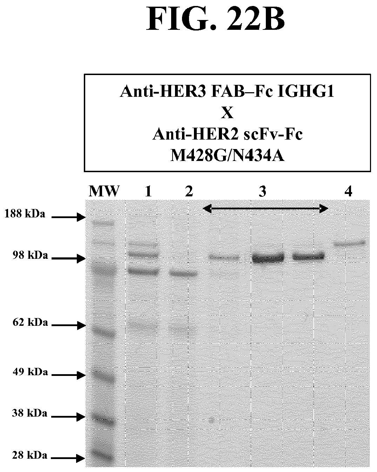

**FIG. 22A****:** Protein G gradient mode chromatography trace of anti-HER3 FAB-Fc IGHG1 x anti-HER2 scFv-Fc M428G/N434A hetero-dimer (HiTrap™ Protein G HP column). Plot of absorbance at 280 nm vs. total volume of mobile phase is shown as solid line. Plots of mobile phase pH and percentage of eluent buffer (B) present in mobile phase are shown as dashed and dotted-dashed lines, respectively. **FIG. 22B****:** SDS-PAGE analysis of chromatography fractions from trace shown in FIG. 22A. (MW) molecular weight marker as indicated. (1) cell culture supernatant. (2) flow-through. (3) peak 1. (4) peak 2.

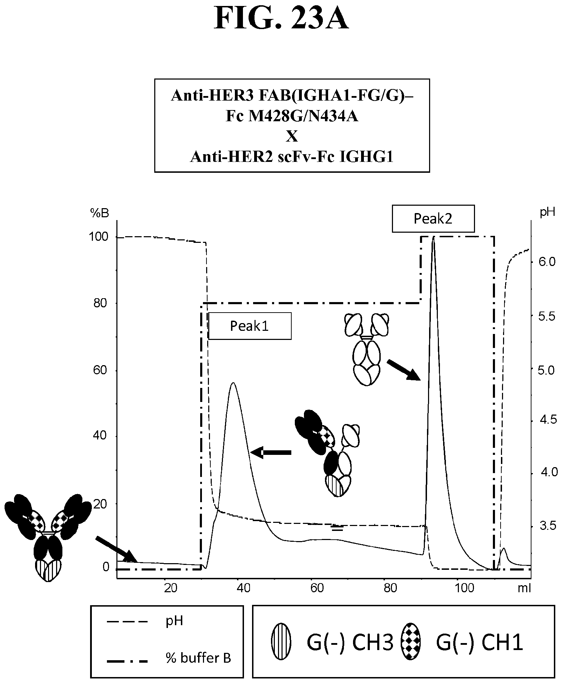

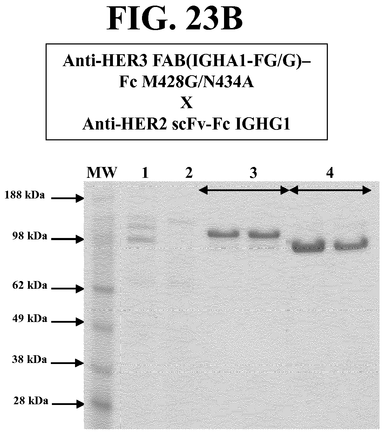

**FIG. 23A****:** Protein G gradient mode chromatography trace of anti-HER3 FAB(IGHA1-FG/G)-Fc M428G/N434A x anti-HER2 scFv-Fc IGHG1 hetero-dimer (HiTrap™ Protein G HP column). Plot of absorbance at 280 nm vs. total volume of mobile phase is shown as solid line. Plots of mobile phase pH and percentage of eluent buffer (B) present in mobile phase are shown as dashed and dotted-dashed lines, respectively. **FIG. 23B****:** SDS-PAGE analysis of chromatography fractions from trace shown in FIG. 23A. (MW) molecular weight marker as indicated. (1) cell culture supernatant. (2) flow-through. (3) peak 1. (4) peak 2.

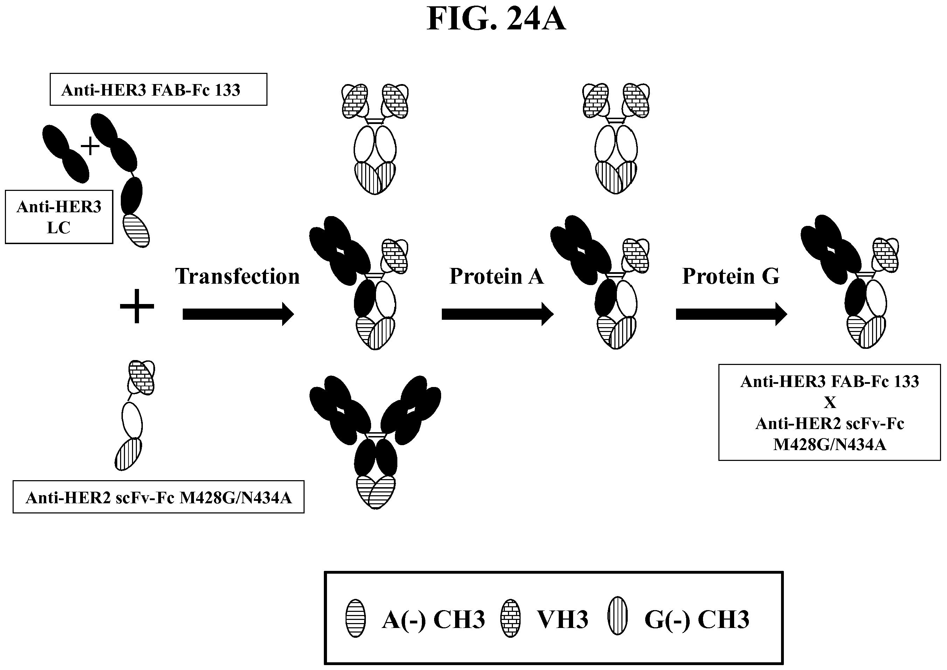

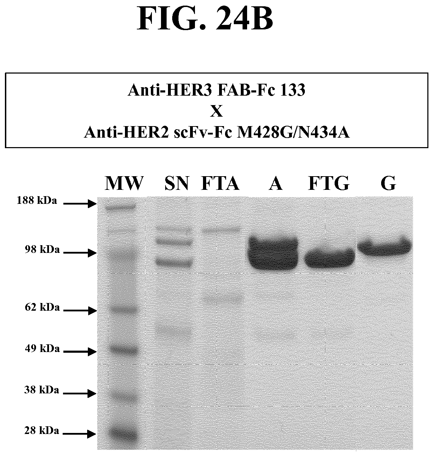

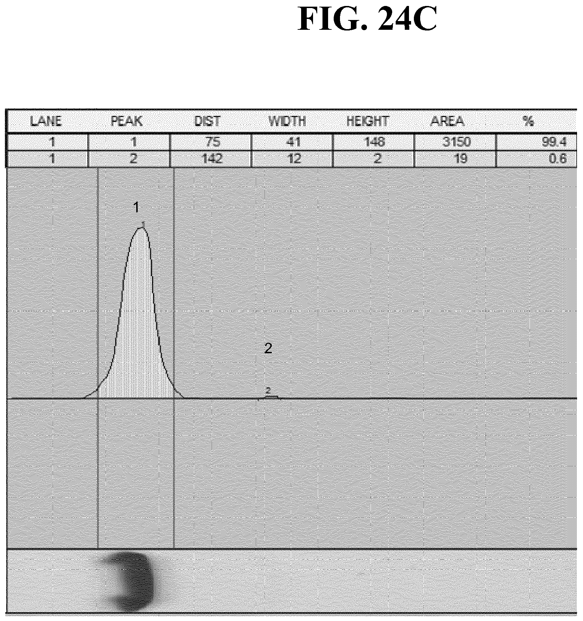

**FIG. 24A****:** Purification scheme of anti-HER3 FAB-Fc 133 x anti-HER2 scFv-Fc M428G/N434A hetero-dimer using a combination of Protein A and Protein G capture-elution mode chromatography (HiTrap™ MabSelect SuRe™ Protein A column and HiTrap™ Protein G HP column). **FIG. 24B****:** SDS-PAGE analysis of the Protein A and Protein G steps performed according to the purification scheme shown in FIG. 24A. (MW) molecular weight marker as indicated. (SN) cell culture supernatant. (FTA) flow-through from Protein A capture-elution step. (A) elution from Protein A capture-elution step. (FTG) flow-through from Protein G capture-elution step. (A) elution Protein G capture-elution step. **FIG. 24C****:** Scanning densitometry analysis assessing the relative proportion of anti-HER3 FAB-Fc 133 x anti-HER2 scFv-Fc M428G/N434A hetero-dimer after Protein A and G capture-elution purification (4-12% SDS Tris-glycine polyacrylamide gel).

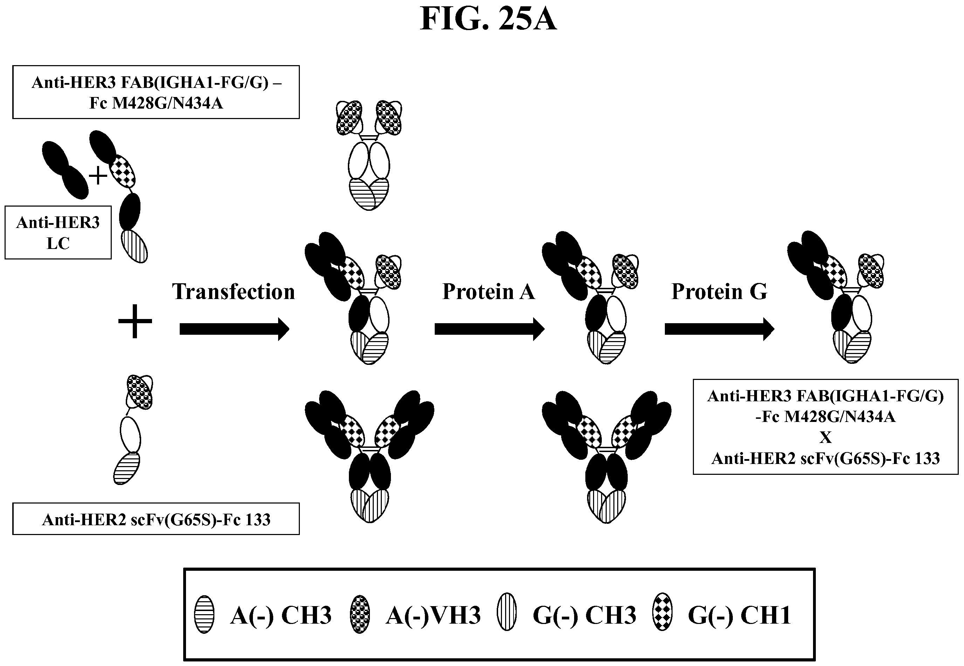

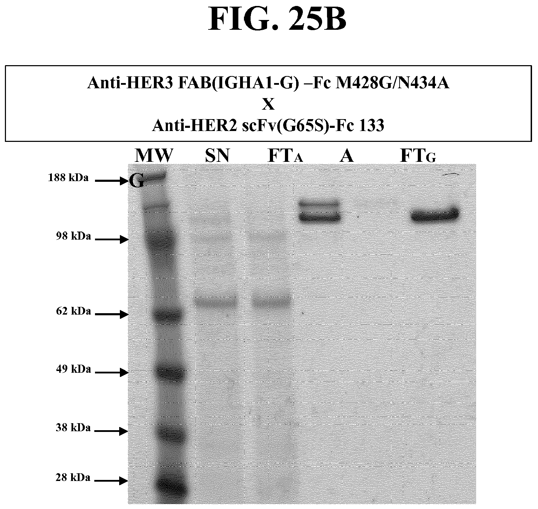

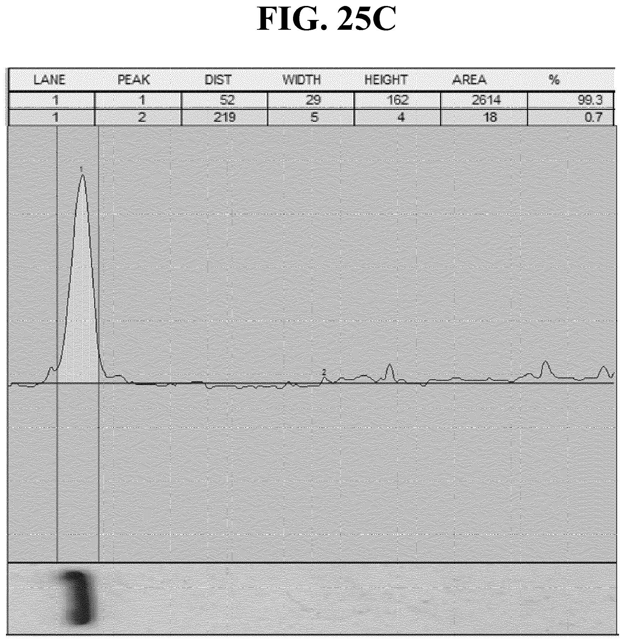

**FIG. 25A****:** Purification scheme of anti-HER3 FAB(IGHA1-FG/G)-Fc M428G/N434A x anti-HER2 scFv(G65S)-Fc 133 hetero-dimer using a combination of Protein A and Protein G capture-elution mode chromatography (HiTrap™ MabSelect SuRe™ Protein A column and HiTrap™ Protein G HP column). **FIG. 25B****:** SDS-PAGE analysis of the Protein A and Protein G steps performed according to the purification scheme shown in FIG. 25A: (MW) molecular weight marker as indicated. (SN) cell culture supernatant. (FTA) flow-through from Protein A capture-elution step. (A) elution from Protein A capture-elution step. (FTG) flow-through from Protein G capture-elution step. (A) elution from Protein G capture-elution step. **FIG. 25C****:** Scanning densitometry analysis assessing the relative proportion of anti-HER3 FAB(IGHA1-FG/G)-Fc M428G/N434A x anti-HER2 scFv(G65S)-Fc 133 hetero-dimer after Protein A and G capture-elution purification (4-12% SDS Tris-glycine polyacrylamide gel).

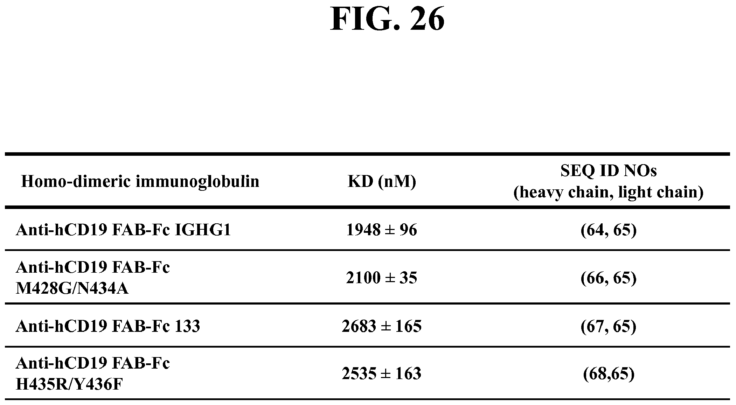

**FIG. 26****:** Equilibrium dissociation constants (KD) of selected anti-hCD19 FAB-Fc variants for human FcRn.

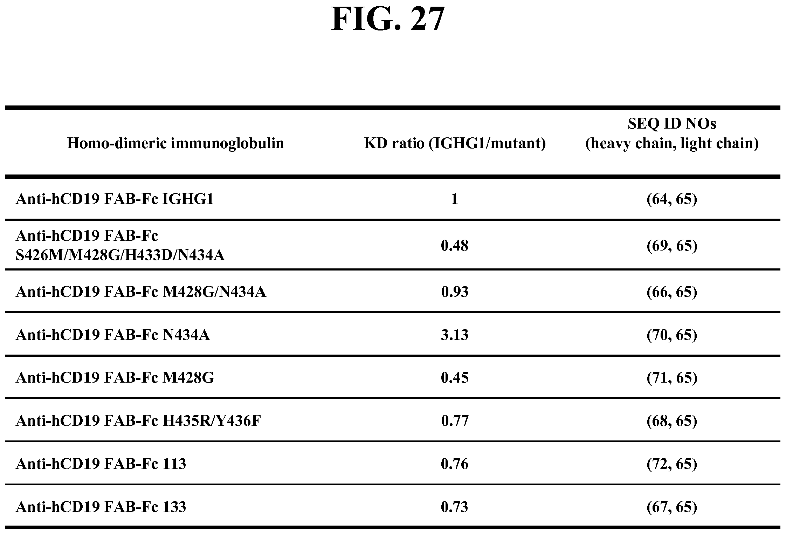

**FIG. 27****:** Equilibrium dissociation constants (KD) of selected anti-hCD19 FAB-Fc variants for human FcRn expressed as relative ratios to the unmodified anti-hCD19 FAB-Fc IGHG1 control.

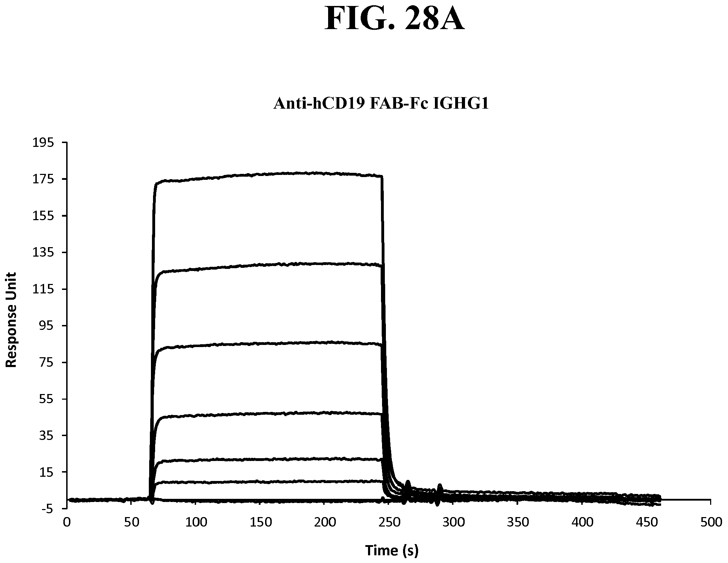

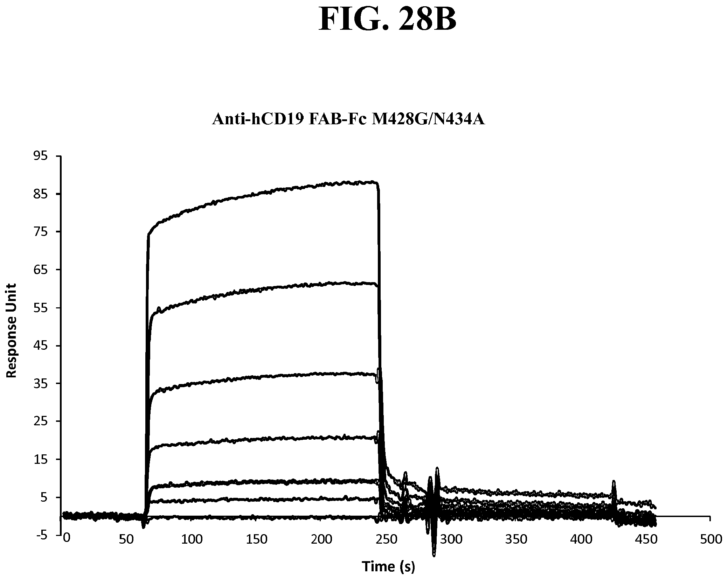

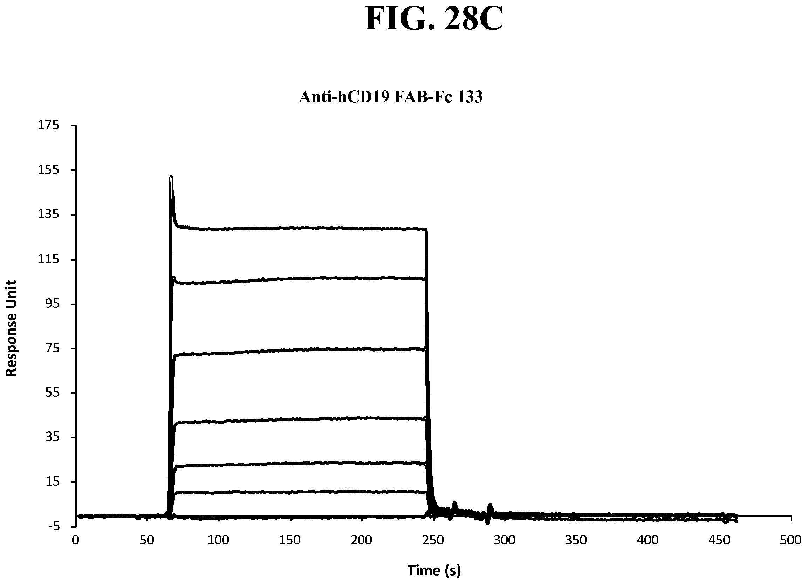

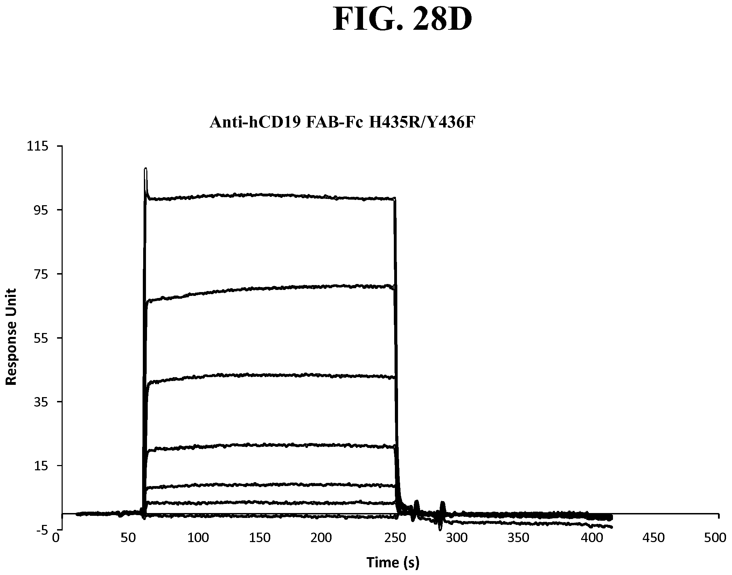

**FIG. 28 A-D****:** Surface Plasmon Resonance measurements of selected anti-hCD19 FAB-Fc variants for the human FcRn (as indicated). Data are expressed as number of response units (abbreviated RU; Y axis) vs. time (X axis). FIG. 28A: Anti-hCD19 FAB-Fc IGHG1; FIG. 28B: Anti-hCD19 FAB-Fc M428G/N434A; FIG. 28C: Anti-hCD19 FAB-Fc 133; FIG. 28D: Anti-hCD19 FAB-Fc H435R/Y436F.

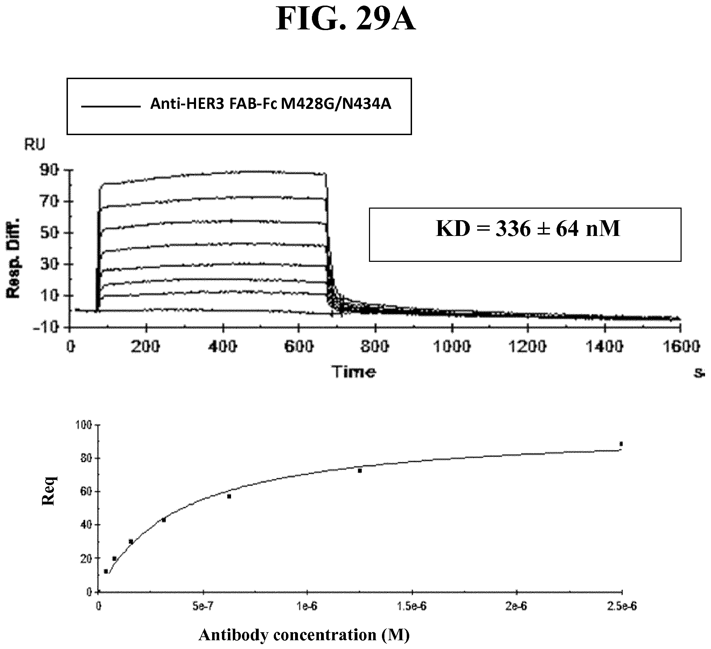

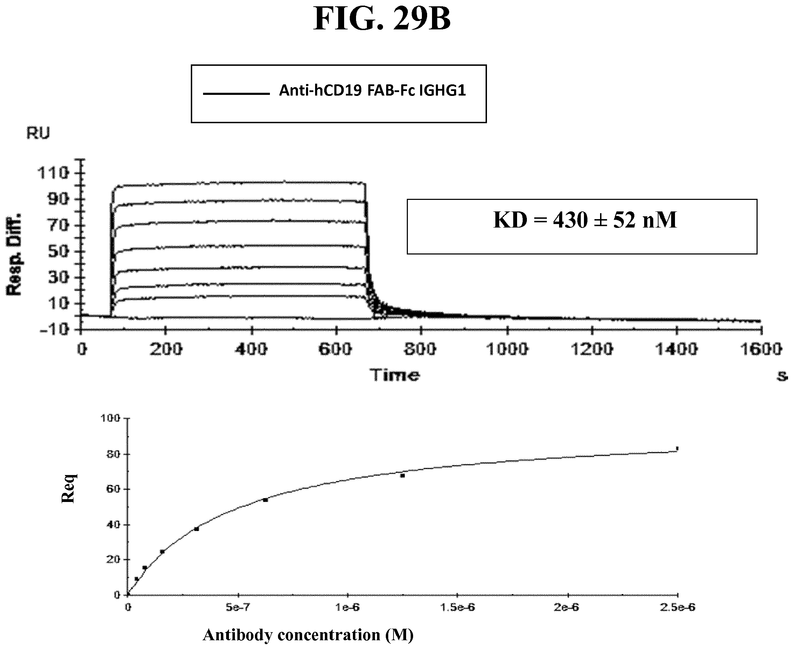

**FIG. 29A****:** Upper plot shows one Surface Plasmon Resonance measurement of anti-HER3 FAB-Fc M428G/N434A for the human FcyR3a. Data are expressed as number of response units (abbreviated RU; Y axis) vs. time (X axis). Mean KD value calculated from three independent experiments is shown. Lower plot shows calculated Req value against antibody concentration based on upper plot and from which KD value is determined. **FIG. 29B****:** Upper plot shows one Surface Plasmon Resonance measurement of anti-hCD19 FAB-Fc IGHG1 for the human FcyR3a. Data are expressed as number of response units (abbreviated RU; Y axis) vs. time (X axis). Mean KD value calculated from three independent experiments is shown. Lower plot shows calculated Req value against antibody concentration based on upper plot and from which KD value is determined.

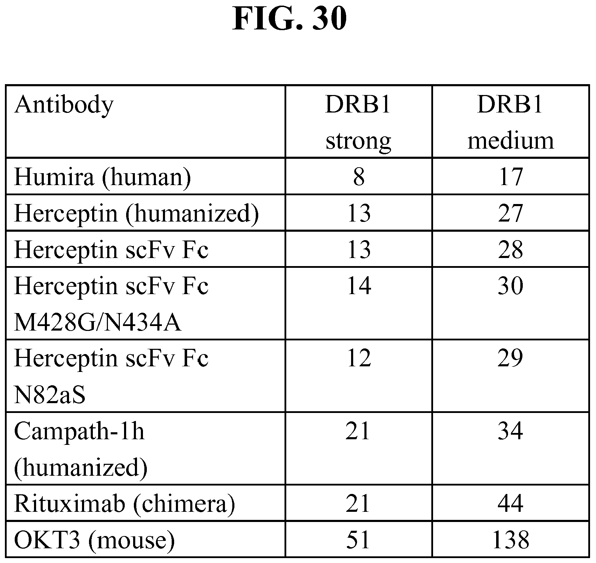

**FIG. 30****:** Table showing Epibase™ immunogenicity results for substitutions M428G and N434A and substitution N82aS. Counts of strong and medium binding to the DRB1 allotype group are shown. Results for a selection of therapeutic antibodies are also shown.
**FIG. 31A****-B:** Tables showing Epibase™ immunogenicity results for substitutions T209G, T209P, and K213V. For each position, the global DRB1 score difference is shown for every possible substitution.

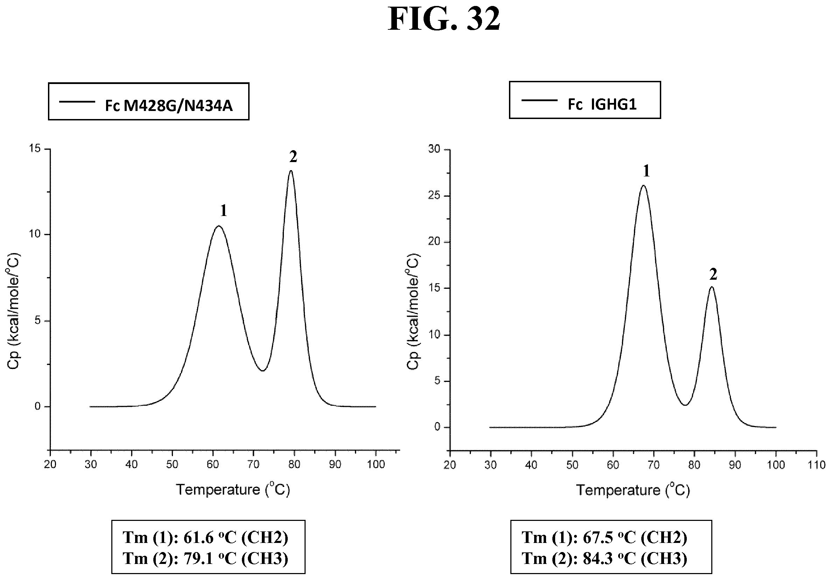

**FIG. 32****:** Thermo-stability measurements of Fc M428G/N434A and Fc IGHG1 using differential scanning calorimetry. Data are expressed as excess molar heat capacity (abbreviated Cp [kcal/mol/°C]; Y axis) vs. temperature (°C; X axis).

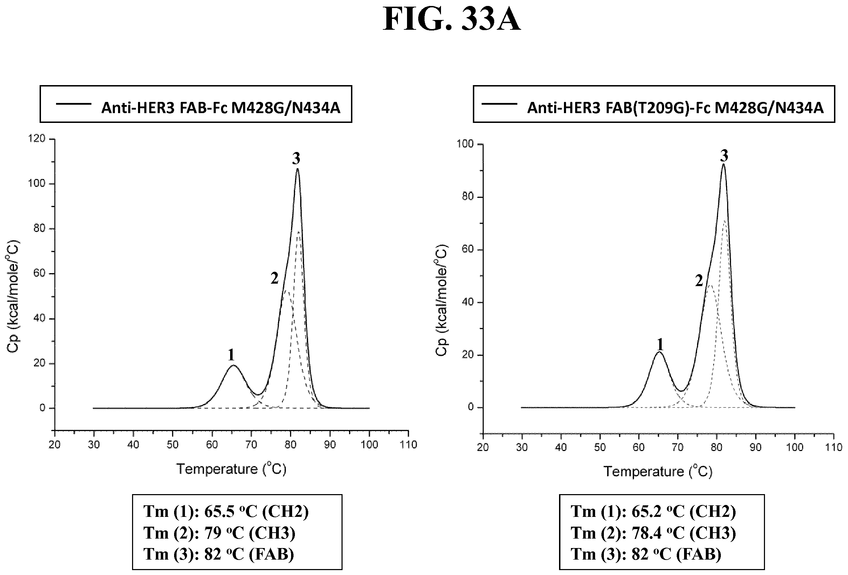

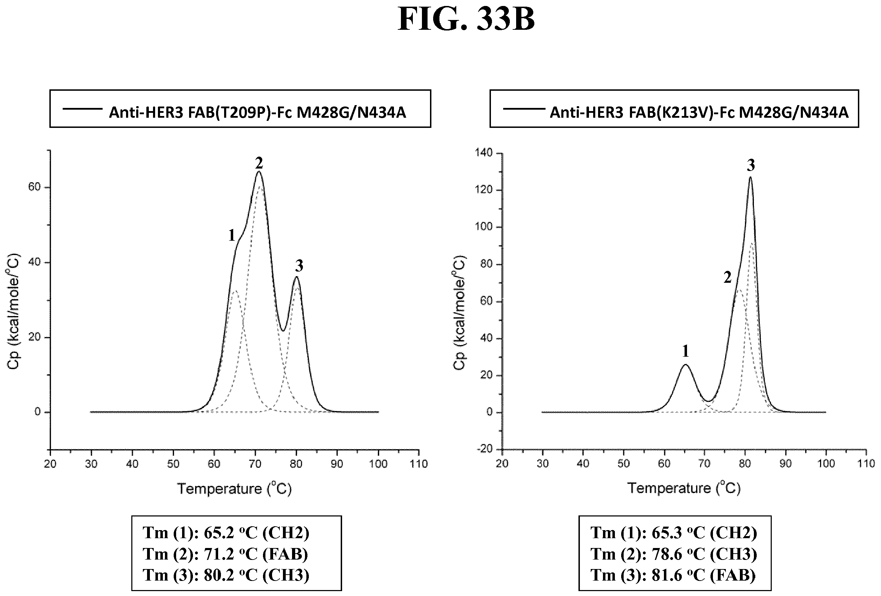

**FIG. 33A****-B:** Thermo-stability measurements using differential scanning calorimetry. Data are expressed as excess molar heat capacity (abbreviated Cp [kcal/mol/°C]; Y axis) vs. temperature (°C; X axis). FIG. 33A: Anti-HER3 FAB-Fc M428G/N434A and anti-HER3 FAB(T209G)-Fc M428G/N434A. FIG. 33B: Anti-HER3 FAB(T209P)-Fc M428G/N434A and anti-HER3 FAB(K213V)-Fc M428G/N434A.

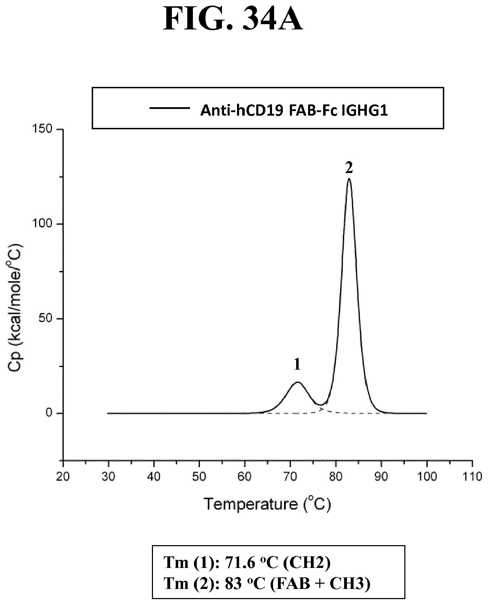

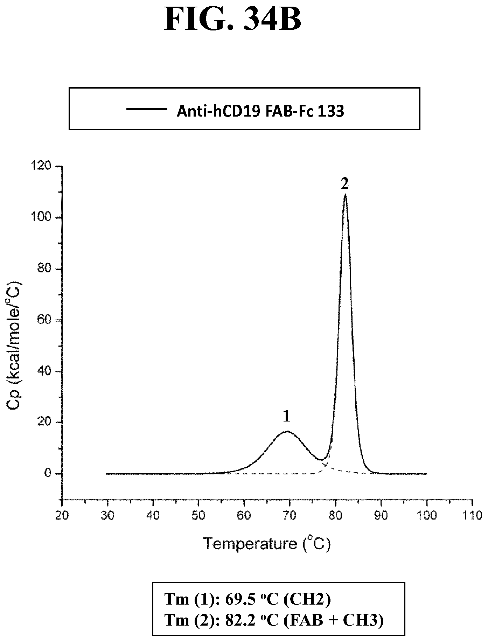

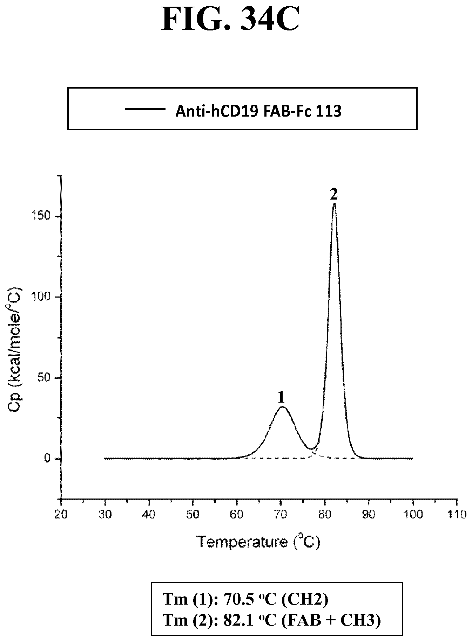

**FIG. 34A****-C:** Thermo-stability measurements using differential scanning calorimetry. Data are expressed as excess molar heat capacity (abbreviated Cp [kcal/mol/°C]; Y axis) vs. temperature (°C; X axis). FIG. 34A: Anti-hCD19 FAB-Fc IGHG1. FIG. 34B: Anti-hCD19 FAB-Fc 133. FIG. 34C: Anti-hCD19 FAB-Fc 113.

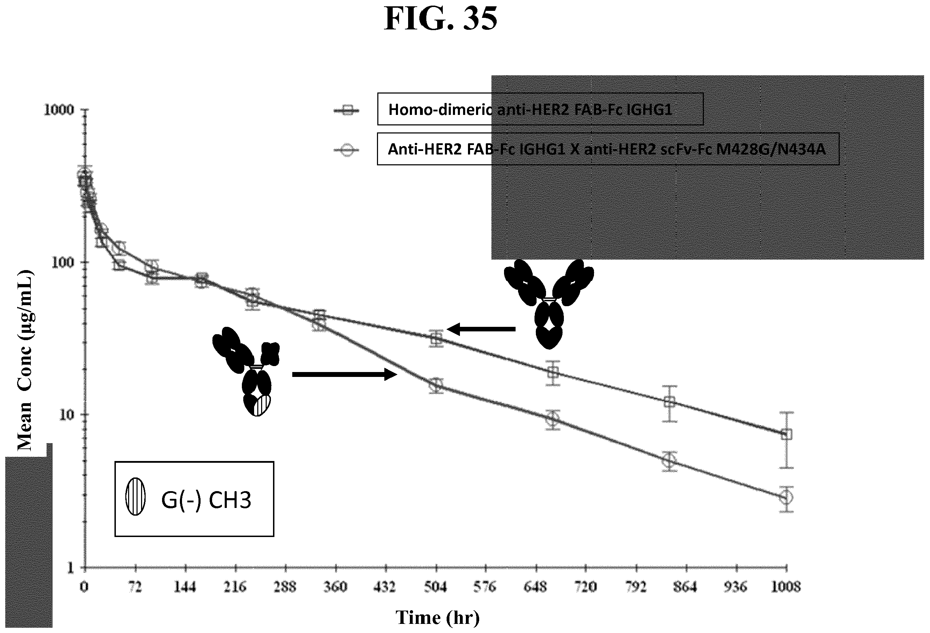

**FIG. 35****:** Semi-logarithmic plasma concentration-time profiles after intravenous administration (bolus) of homo-dimeric anti-HER2 FAB-Fc IGHG1 or hetero-dimeric anti-HER2 FAB-Fc IGHG1 X anti-HER2 scFv-Fc M428G/N434A immunoglobulins to female Sprague-Dawley rats. Results are expressed as mean ± SD from four rats. Data are expressed as mean serum concentration (abbreviated Mean Conc, µg/ml; Y axis) vs. time (hours, X axis).

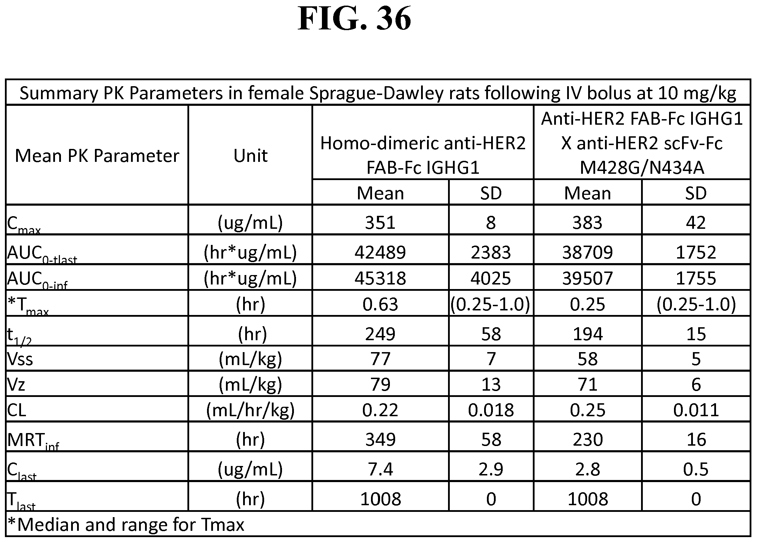

**FIG. 36****:** Table showing summary PK Parameters in female Sprague-Dawley rats following IV bolus at 10 mg/kg of homo-dimeric anti-HER2 FAB-Fc IGHG1 or hetero-dimeric anti-HER2 FAB-Fc IGHG1 X anti-HER2 scFv-Fc M428G/N434A immunoglobulins. (t_{1/2}) corresponds to immunoglobulin elimination half-life.

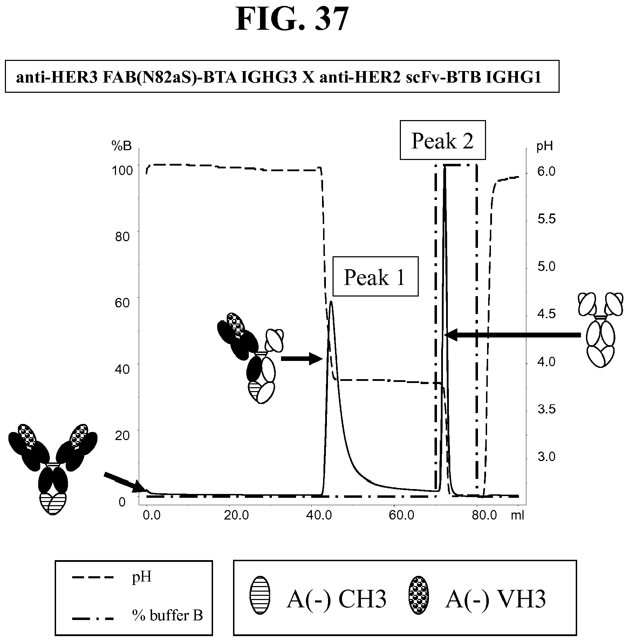

**FIG. 37****:** Protein A gradient mode chromatography trace of anti-HER3 FAB(N82aS)-BTA IGHG3 X anti-HER2 scFv-BTB IGHG1 hetero-dimer (HiTrap™ MabSelect SuRe™ Protein A column). Plot of absorbance at 280 nm vs. total volume of mobile phase is shown as solid line. Plots of mobile phase pH and percentage of eluent buffer (B) present in mobile phase are shown as dashed and dotted-dashed lines, respectively.

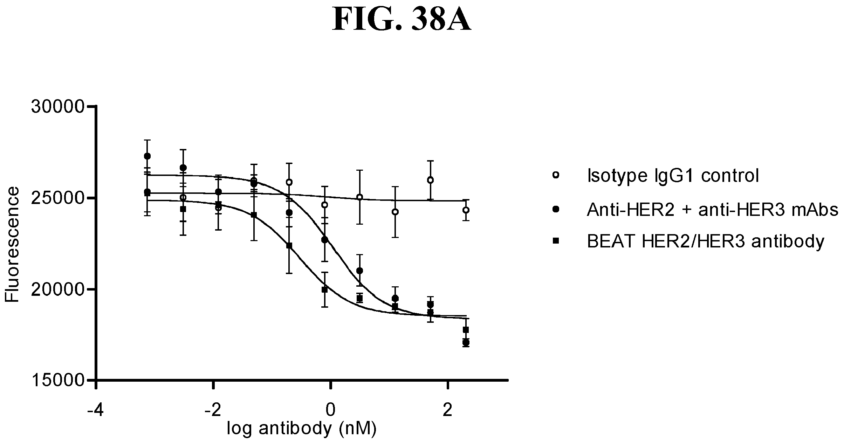

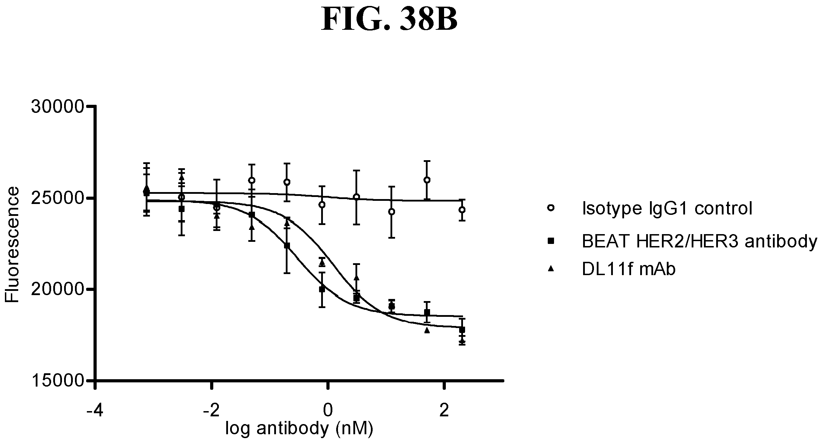

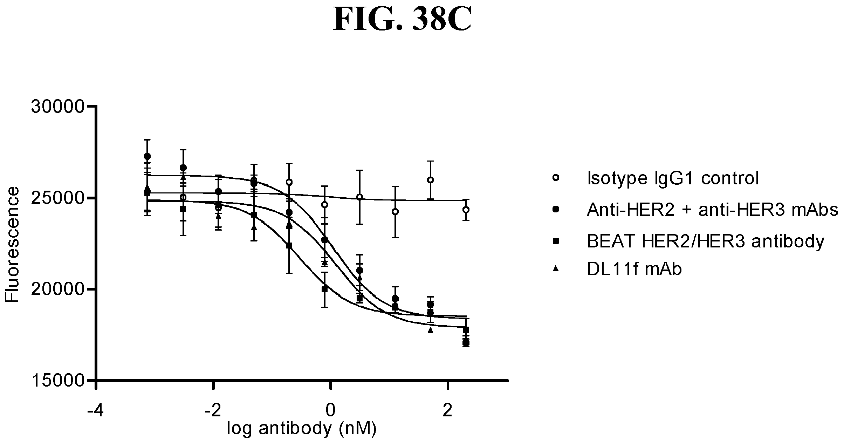

**FIG. 38A****-C:** Calu-3 cell proliferation assay. Calu-3 cells in the presence of 3 nM heregulin beta were treated with serial dilutions of antibodies in the presence of 1% serum containing growth medium. Cell proliferation was measured after 3 days using alamarBlue® staining. Results are expressed in semi-logarithmic antibody concentration vs. fluorescence units (excitation at 540 nm, emission at 620 nm). IgG1 isotype control is indicated as a negative control of inhibition of cell proliferation. FIG. 38A: BEAT HER2/HER3 antibody and equimolar mixture of anti-HER2 and anti-HER3 antibodies. FIG. 38B: BEAT HER2/HER3 antibody and DL11f antibody (anti-EGFR and anti-HER3 bispecific antibody). FIG. 38C: BEAT HER2/HER3 antibody, equimolar mixture of anti-HER2 and anti-HER3 antibodies, and DL11f antibody.

### Detailed Description of the Invention

The present invention relates generally to novel hetero-dimeric immunoglobulin variants, which have reduced or eliminated binding to protein A, protein G or both protein A and protein G. Also encompassed in the present invention are methods for the selective purification of hetero-dimeric immunoglobulins.

For purposes of interpreting this specification, the following definitions will apply and whenever appropriate, terms used in the singular will also include the plural and vice versa. It is to be understood that the terminology used herein is for the purpose of describing particular embodiments only, and is not intended to be limiting.

The terms "polypeptide" and "protein" refer to a polymer of amino acid residues wherein amino acids are combined via peptide bonds to form a chain of amino acids that have been linked together via dehydration synthesis. Polypeptides and proteins can be synthesized through chemical synthesis or recombinant expression and are not limited to a minimum amino acid length.

In accordance with the invention, the group of polypeptides comprises "proteins" as long as the proteins consist of a single polypeptide chain. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or hetero-dimers, homo- or hetero-trimers etc. An example for a hetero-multimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art. Furthermore, for purposes of the present invention, a "polypeptide" refers to a protein which includes modifications, such as deletions, additions and substitutions (which can be conservative in nature) to the native sequence. These modifications may be deliberate, as through site-directed mutagenesis, or may be accidental, such as through mutations of hosts which produce the proteins or errors due to PCR amplification.

The term "immunoglobulin" as referred to herein can be used interchangeably with the term "antibody". Immunoglobulin includes full-length antibodies and any antigen binding fragment or single chains thereof. Immunoglobulins can be homo-dimeric or hetero-dimeric. Immunoglobulins and specifically naturally occurring antibodies are glycoproteins which exist as one or more copies of a Y-shaped unit, composed of four polypeptide chains. Each "Y" shape contains two identical copies of a heavy (H) chain, and two identical copies of a light (L) chain, named as such by their relative molecular weights. Each light chain pairs with a heavy chain, and each heavy chain pairs with another heavy chain. Covalent interchain disulfide bonds and non covalent interactions link the chains together. Immunoglobulins and specifically naturally occurring antibodies contain variable regions, which are the two copies of the antigen binding site. Papain, a proteolytic enzyme splits the "Y" shape into three separate molecules, two so called "Fab" or "FAB" fragments (Fab = fragment antigen binding), and one so called "Fc" fragment or "Fc region" (Fc = fragment crystallizable). A Fab fragment consists of the entire light chain and part of the heavy chain. The heavy chain contains one variable region (VH) and either three or four constant regions (CHI, CH2, CH3, and CH4, depending on the antibody class or isotype). The region between the CH1 and CH2 regions is called the hinge region and permits flexibility between the two Fab arms of the Y-shaped antibody molecule, allowing them to open and close to accommodate binding to two antigenic determinants separated by a fixed distance. The "hinge region" as referred to herein is a sequence region of 6-62 amino acids in length, only present in IgA, IgD, and IgG, which encompasses the cysteine residues that bridge the two heavy chains. The heavy chains of IgA, IgD, and IgG each have four regions, i.e. one variable region (VH) and three constant regions (CH1-3). IgE and IgM have one variable and four constant regions (CH1-4) on the heavy chain. The constant regions of the immunoglobulins may mediate the binding to host tissues or factors, including various cells of the immune system (e.g., effector cells) and the first component (C1q) of the complement system classical pathway. Each light chain is usually linked to a heavy chain by one covalent disulfide bond. Each light chain contains one variable region (VL) and one light chain constant region. The light chain constant region is a kappa light chain constant region designated herein as IGKC or is a lambda light chain constant region designated herein as IGLC. IGKC is used herein equivalently to Cκ or CK and has the same meaning. IGLC is used herein equivalently to Cλ or CL and has the same meaning. The term "an IGLC region" as used herein refer to all lambda light chain constant regions e.g. to all lambda light chain constant regions selected from the group consisting of IGLC1, IGLC2, IGLC3, IGLC6, and IGLC7. The VH and VL regions can be further subdivided into regions of hypervariability, termed complementarity determining regions (CDR), interspersed with regions that are more conserved, termed framework regions (FR or FW). Each VH and VL is composed of three CDRs and four FRs, arranged from amino- terminus to carboxy-terminus in the following order: FR1, CDR1, FR2, CDR2, FR3, CDR3, FR4. The variable regions of the heavy and light chains contain an epitope- binding region that interacts with an antigen.

The term "full length antibody" as used herein includes the structure that constitutes the natural biological form of an antibody, including variable and constant regions. For example, in most mammals, including humans and mice, the full length antibody of the IgG class is a tetramer and consists of two identical pairs of two immunoglobulin chains, each pair having one light and one heavy chain, each light chain comprising immunoglobulin regions VL and a light chain constant region, and each heavy chain comprising immunoglobulin regions VH, CH1 (Cγ1), CH2 (Cγ2), CH3 (Cγ3), and CH4 (Cγ4), depending on the antibody class or isotype). In some mammals, for example in camels and llamas, IgG antibodies may consist of only two heavy chains, each heavy chain comprising a variable region attached to the Fc region.

Antibodies are grouped into classes, also referred to as isotypes, as determined genetically by the constant region. Human constant light chains are classified as kappa (CK) and lambda (Cλ) light chains. Heavy chains are classified as mu (µ), delta (δ), gamma (γ), alpha (α), or epsilon (ε), and define the antibody's isotype as IgM, IgD, IgG, IgA, and IgE, respectively. Thus, "isotype" as used herein is meant any of the classes and/or subclasses of immunoglobulins defined by the chemical and antigenic characteristics of their constant regions. The known human immunoglobulin isotypes are IGHG1 (IgG1), IGHG2 (IgG2), IGHG3 (IgG3), IGHG4 (IgG4), IGHA1 (IgA1), IGHA2 (IgA2), IGHM (IgM), IGHD (IgD), and IGHE (IgE). The so-called human immunoglobulin pseudo-gamma IGHGP gene represents an additional human immunoglobulin heavy constant region gene which has been sequenced but does not encode a protein due to an altered switch region (Bensmana M et al., (1988) Nucleic Acids Res, 16(7): 3108). In spite of having an altered switch region, the human immunoglobulin pseudo-gamma IGHGP gene has open reading frames for all heavy constant regions (CH1-CH3) and hinge. All open reading frames for its heavy constant regions encode protein regions which align well with all human immunoglobulin constant regions with the predicted structural features. This additional pseudo-gamma isotype is referred herein as IgGP or IGHGP. Other pseudo immunoglobulin genes have been reported such as the human immunoglobulin heavy constant region epsilon P1 and P2 pseudo-genes (IGHEP1 and IGHEP2). The IgG class is the most commonly used for therapeutic purposes. In humans this class comprises subclasses IgG1, IgG2, IgG3, and IgG4. In mice this class comprises subclasses IgG1, IgG2a, IgG2b, IgG2c and IgG3.

The term "Immunoglobulin fragments" as used herein include, but is not limited to, (i) a region including for example a CH1, a CH2 or a CH3 region, (ii) the Fab fragment consisting of VL, VH, CL or CK and CH1 regions, including Fab' and Fab'-SH, (ii) the Fd fragment consisting of the VH and CH1 regions, (iii) the dAb fragment (Ward ES et al., (1989) Nature, 341(6242): 544-6) which consists of a single variable region (iv) F(ab')₂ fragments, a bivalent fragment comprising two linked Fab fragments (v) single chain Fv molecules (scFv), wherein a VH region and a VL region are linked by a peptide linker which allows the two regions to associate to form an antigen binding site (Bird RE et al., (1988) Science, 242(4877): 423-6; Huston JS et al., (1988) Proc Natl Acad Sci U S A, 85(16): 5879-83), (vi) "diabodies" or "triabodies", multivalent or multispecific fragments constructed by gene fusion (Holliger P et al., (1993) Proc Natl Acad Sci U S A, 90(14): 6444-8; Tomlinson I & Holliger P, (2000) Methods Enzymol, 326:461-79), (vii) scFv, diabody or region antibody fused to an Fc region and (viii) scFv fused to the same or a different antibody.

The term "variable region" refers to the regions or domains that mediates antigen-binding and defines specificity of a particular antibody for a particular antigen. In naturally occurring antibodies, the antigen-binding site consists of two variable regions that define specificity: one located in the heavy chain, referred herein as heavy chain variable region (VH) and the other located in the light chain, referred herein as light chain variable region (VL). In humans, the heavy chain variable region (VH) can be divided into seven subgroups VH1, VH2, VH3, VH4, VH5, VH6 and VH7. In some cases, specificity may exclusively reside in only one of the two regions as in single-domain antibodies from heavy-chain antibodies found in camelids. The V regions are usually about 110 amino acids long, and consist of relatively invariant stretches of amino acid sequence called framework regions (FRs or "non-CDR regions") of 15-30 amino acids separated by shorter regions of extreme variability called "hypervariable regions" that are 7-17 amino acids long. The variable domains of native heavy and light chains comprise four FRs, largely adopting a beta-sheet configuration, connected by three hypervariable regions, which form loops. The hypervariable regions in each chain are held together in close proximity by FRs and, with the hypervariable regions from the other chain, contribute to the formation of the antigen binding site of antibodies (see Kabat EA *et al., supra.).* The term "hypervariable region" as used herein refers to the amino acid residues of an antibody which are responsible for antigen binding. The hypervariable region generally comprises amino acid residues from a "complementary determining region" or "CDR", the latter being of highest sequence variability and/or involved in antigen recognition. For all variable regions numbering is according to Kabat (Kabat EA *et al., supra.*)*.*

A number of CDR definitions are in use and are encompassed herein. The Kabat definition is based on sequence variability and is the most commonly used (Kabat EA *et al., supra.*)*.* Chothia refers instead to the location of the structural loops (Chothia & Lesk J. (1987) Mol. Biol. 196:901-917). The AbM definition is a compromise between the Kabat and the Chothia definitions and is used by Oxford Molecular's AbM antibody modelling software (Martin ACR et al., (1989) PNAS USA 86:9268-9272; Martin ACR et al., (1991) Methods Enzymol. 203:121-153; Pedersen JT et al., (1992) Immunomethods 1:126-136; Rees AR et al., (1996) In Sternberg M.J.E. (ed.), Protein Structure Prediction. Oxford University Press, Oxford, 141-172). The contact definition has been recently introduced (MacCallum RM et al., (1996) J. Mol. Biol. 262:732-745) and is based on an analysis of the available complex structures available in the Protein Databank. The definition of the CDR by IMGT®, the international ImMunoGeneTics information system® (http://www.imgt.org) is based on the IMGT numbering for all immunoglobulin and T cell receptor V-REGIONs of all species (IMGT®, the international ImMunoGeneTics information system®; Lefranc MP et al., (1999) Nucleic Acids Res. 27(1):209-12; Ruiz M et al., (2000) Nucleic Acids Res. 28(1):219-21; Lefranc MP (2001) Nucleic Acids Res. 29(1):207-9; Lefranc MP (2003) Nucleic Acids Res. 31(1):307-10; Lefranc MP et al., (2005) Dev. Comp. Immunol. 29(3):185-203; Kaas Q et al., (2007) Briefings in Functional Genomics & Proteomics, 6(4):253-64). All Complementarity Determining Regions (CDRs) as referred to in the present invention, are defined preferably as follows (numbering according to Kabat EA *et al., supra*):
LCDR1: 24-34
LCDR2: 50-56
LCDR3: 89-98
HCDR1: 26-35
HCDR2: 50-65
HCDR3: 95-102
The "non-CDR regions" of the variable domain are known as framework regions (FR). The "non-CDR regions" of the VL region as used herein comprise the amino acid sequences: 1-23 (FR1), 35-49 (FR2), 57-88 (FR3), and 99-107 (FR4).
The "non-CDR regions" of the VH region as used herein comprise the amino acid sequences: 1-25 (FR1), 36-49 (FR2), 66-94 (FR3), and 103-113 (FR4).

The CDRs of the present invention may comprise "extended CDRs" which are based on the aforementioned definitions and have variable domain residues as follows: LCDR1: 24-36, LCDR2: 46-56, LCDR3:89-97, HCDR1: 26-35, HCDR2:47-65, HCDR3: 93-102. These extended CDRs are numbered as well according to Kabat *et al., supra.* The "non-extended CDR region" of the VL region as used herein comprise the amino acid sequences: 1-23 (FR1), 37-45 (FR2), 57-88 (FR3), and 98- approximately 107 (FR4). The "non-extended CDR region" of the VH region as used herein comprise the amino acid sequences: 1-25 (FR1), 37-46 (FR2), 66-92 (FR3), and 103- approximately 113 (FR4).

The term "Fab" or "FAB" or "Fab region" or "FAB region" as used herein includes the polypeptides that comprise the VH, CH1, VL, and light chain constant immunoglobulin regions. Fab may refer to this region in isolation, or this region in the context of a full length antibody or antibody fragment.

The term "Fc" or "Fc region", as used herein includes the polypeptide comprising the constant region of an antibody heavy chain excluding the first constant region immunoglobulin region. Thus Fc refers to the last two constant region immunoglobulin regions of IgA, IgD, and IgG, and the last three constant region immunoglobulin regions of IgE and IgM, and the flexible hinge N-terminal to these regions. For IgA and IgM, Fc may include the J chain. For IgG, Fc comprises immunoglobulin regions Cgamma2 and Cgamma3 (Cy2 and Cy3) and the hinge between Cgamma1 (Cyl) and Cgamma2 (Cy2). Although the boundaries of the Fc region may vary, the human IgG heavy chain Fc region is usually defined to comprise residues C226 or P230 to its carboxyl-terminus, wherein the numbering is according to the EU index. Fc may refer to this region in isolation or this region in the context of an Fc polypeptide, for example an antibody.

The term "immunoglobulin constant region" as used herein refers to immunoglobulin or antibody heavy chain constant regions from human or animal species and encompasses all isotypes. Preferably, immunoglobulin constant regions are of human origin and are selected from the group consisting of, but not limited to: IGHG1 CH1, IGHG2 CH1, IGHG3 CH1, IGHG4 CH1, IGHA1 CH1, IGHA2 CH1, IGHE CH1, IGHEP1 CH1, IGHM CH1, IGHD CH1, IGHGP CH1, IGHG1 CH2, IGHG2 CH2, IGHG3 CH2, IGHG4 CH2, IGHA1 CH2, IGHA2 CH2, IGHE CH2, IGHEP1 CH2, IGHM CH2, IGHD CH2, IGHGP CH2, IGHG1 CH3, IGHG2 CH3, IGHG3 CH3, IGHG4 CH3, IGHA1 CH3, IGHA2 CH3, IGHE CH3, IGHEP1 CH3, IGHM CH3, IGHD CH3, IGHGP CH3, IGHE CH4 and IGHM CH4. Prefered "immunoglobulin constant regions" are selected from the group consisting of human IGHE CH2, IGHM CH2, IGHG1 CH3, IGHG2 CH3, IGHG3 CH3, IGHG4 CH3, IGHA1 CH3, IGHA2 CH3, IGHE CH3, IGHM CH3, IGHD CH3 and IGHGP CH3. More prefered "immunoglobulin constant regions" are selected from the group consisting of human IGHG1 CH3, IGHG2 CH3, IGHG3 CH3, IGHG4 CH3, IGHA1 CH3, IGHA2 CH3, IGHE CH3, IGHM CH3, IGHD CH3 and IGHGP CH3.

The term "epitope binding region" includes a polypeptide or a fragment thereof having minimal amino acid sequence to permit the specific binding of the immunoglobulin molecule to one or more epitopes. Naturally occurring antibodies have two epitope binding regions which are also known as antigen binding or combining sites or paratopes. Epitope binding regions in naturally occurring antibodies are confined within the CDR regions of the VH and/or VL domains wherein the amino acid mediating epitope binding are found. In addition to naturally occurring antibodies, artificial VH domains or VL domains or fragments thereof and combinations thereof can be engineered to provide epitope binding regions (Holt LJ et al., (2003) Trends Biotechnol, 21(11): 484-490; Polonelli L et al., (2008) PLoS ONE, 3(6): e2371). Examples of non immunoglobulin based epitope binding regions can be found in artificial protein domains used as "scaffold" for engineering epitope binding regions (Binz HK et al., (2005) Nat Biotechnol, 23(10): 1257-1268) or peptide mimetics (Murali R & Greene MI (2012) Pharmaceuticals, 5(2): 209-235). Preferably the term 'epitope binding region' includes the combination of one or more heavy chain variable domains and one or more complementary light chain variable domains which together forms a binding site which permits the specific binding of the immunoglobulin molecule to one or more epitopes.

As used herein, the term "epitope" includes a fragment of a polypeptide or protein or a non-protein molecule having antigenic or immunogenic activity in an animal, preferably in a mammal, and most preferably in a human. An epitope having immunogenic activity is a fragment of a polypeptide or protein that elicits an antibody response in an animal. An epitope having antigenic activity is a fragment of a polypeptide or protein to which an antibody or polypeptide specifically binds as determined by any method well-known to one of skill in the art, for example by immunoassays. Antigenic epitopes need not necessarily be immunogenic. Preferably, the term "epitope" as used herein refers to a polypeptide sequence of at least about 3 to 5, preferably about 5 to 10 or 15, and not more than about 1,000 amino acids (or any integer there between), which define a sequence that by itself or as part of a larger sequence, binds to an antibody generated in response to such sequence. There is no critical upper limit to the length of the fragment, which may comprise nearly the full-length of the protein sequence, or even a fusion protein comprising one or more epitopes. An epitope for use in the subject invention is not limited to a polypeptide having the exact sequence of the portion of the parent protein from which it is derived. Thus the term "epitope" encompasses sequences identical to the native sequence, as well as modifications to the native sequence, such as deletions, additions and substitutions (generally conservative in nature).The epitopes of protein antigens are divided into two categories, conformational epitopes and linear epitopes, based on their structure and interaction with the epitope binding site (Goldsby R et al., (2003) "Antigens (Chapter 3)" Immunology (Fifth edition ed.), New York: W. H. Freeman and Company. pp. 57-75, ISBN 0-7167-4947-5). A conformational epitope is composed of discontinuous sections of the antigen's amino acid sequence. These epitopes interact with the paratope based on the 3-D surface features and shape or tertiary structure of the antigen. Most epitopes are conformational. By contrast, linear epitopes interact with the paratope based on their primary structure. A linear epitope is formed by a continuous sequence of amino acids from the antigen.

The term "hetero-dimeric immunoglobulin" or "hetero-dimeric fragment" or "hetero-dimer" or "hetero-dimer of heavy chains"as used herein includes an immunoglobulin molecule or part of comprising at least a first and a second polypeptide, like a first and a second region, wherein the second polypeptide differs in amino acid sequence from the first polypeptide. Preferably, a hetero-dimeric immunoglobulin comprises two polypeptide chains, wherein the first chain has at least one non identical region to the second chain, and wherein both chains assemble, i.e. interact through their non-identical regions. More preferably the hetero-dimeric immunoglobulin, has binding specificity for at least two different ligands, antigens or binding sites, i.e. is bispecific. Hetero-dimeric immunoglobulin as used herein includes but is not limited to full length bispecific antibodies, bispecifc Fab, bispecifc F(ab')₂, bispecific scFv fused to an Fc region, diabody fused to an Fc region and domain antibody fused to an Fc region.

The term "homo-dimeric immunoglobulin" or "homo-dimeric fragment" or "homo-dimer" or "homo-dimer of heavy chains" as used herein includes an immunoglobulin molecule or part of comprising at least a first and a second polypeptide, like a first and a second region, wherein the second polypeptide is identical in amino acid sequence to the first polypeptide. Preferably, a homo-dimeric immunoglobulin comprises two polypeptide chains, wherein the first chain has at least one identical region to the second chain, and wherein both chains assemble, i.e. interact through their identical regions. Preferably, a homo-dimeric immunoglobulin fragment comprises at least two regions, wherein the first region is identical to the second region, and wherein both regions assemble, i.e. interact through their protein-protein interfaces.

For all immunoglobulin constant regions included in the present invention, numbering can be according to the IMGT® (IMGT®; *supra*).

For all human CH1, CH2, CH3 immunoglobulin heavy chain constant regions selected from the group consisting of IGHG1, IGHG2, IGHG3, and IGHG4, numbering can be according to the "EU numbering system" (Edelman GM et al., (1969) Proc Natl Acad Sci USA, 63(1): 78-85). A complete correspondence for the human CH1, hinge, CH2 and CH3 constant regions of IGHG1 can be found at the IMGT database (IMGT®; *supra*).

For the human kappa immunoglobulin light chain constant region (IGKC), numbering can be according to the "EU numbering system" (Edelman GM et al., *supra*). A complete correspondence for the human CK region can be found at IMGT database (IMGT®; *supra*)*.*

For the human lambda immunoglobulin light chain constant regions (IGLC1, IGLC2, IGLC3, IGLC6, and IGLC7), numbering can be according to the "Kabat numbering system" (Kabat EA *et al., supra*)*.* A complete correspondence for human IGLC regions can be found at the IMGT database (IMGT®; *supra*).

The human IGHG1 immunoglobulin heavy chain constant regions as referred to herein have the following region boundaries: CH1 region (EU numbering: 118-215), Hinge γ1 region (EU numbering: 216-230), CH2 region (EU numbering: 231-340), and CH3 region (EU numbering: 341-447). The human CK region referred herein spans residues 108 to 214 (EU numbering). The human IGLC1, IGLC2, IGLC3, IGLC6, and IGLC7 regions referred herein span residues 108-215 (Kabat numbering).

The terms "amino acid" or "amino acid residue" as used herein includes natural amino acids as well as non-natural amino acids. Preferably natural amino acids are included.

The term "modification" or "amino acid modification" herein includes an amino acid substitution, insertion, and/or deletion in a polypeptide sequence. The terms "substitution" or "amino acid substitution" or "amino acid residue substitution" as used herein refers to a substitution of a first amino acid residue in an amino acid sequence with a second amino acid residue, whereas the first amino acid residue is different from the second amino acid residue i.e. the substituted amino acid residue is different from the amino acid which has been substituted. For example, the substitution R94K refers to a variant polypeptide, in which the arginine at position 94 is replaced with a lysine. For example 94K indicates the substitution of position 94 with a lysine. For the purposes herein, multiple substitutions are typically separated by a slash or a comma. For example, "R94K/L78V" or "R94K, L78V" refers to a double variant comprising the substitutions R94K and L78V. By "amino acid insertion" or "insertion" as used herein is meant the addition of an amino acid at a particular position in a parent polypeptide sequence. For example, insert -94 designates an insertion at position 94. By "amino acid deletion" or "deletion" as used herein is meant the removal of an amino acid at a particular position in a parent polypeptide sequence. For example, R94- designates the deletion of arginine at position 94.

In certain embodiments, the terms "decrease", "reduce", or "reduction" in binding to Protein A refers to an overall decrease of at least 25%, 30%, 40%, 50%, 60%, 70%, 80%, 85%, 90%, 95%, 97%, or 99% up to 100% (elimination) in the binding of a modified immunoglobulin or fragment thereof to Protein A detected by standard art known methods such as those described herein, as compared to a parental i.e. unmodified immunoglobulin or wild-type IgG or an IgG having the wild-type human IgG Fc region. In certain embodiments these terms alternatively may refer to an overall decrease of 10-fold (i.e. 1 log), 100-fold (2 logs), 1,000-fold (or 3 logs), 10,000-fold (or 4 logs), or 100,000-fold (or 5 logs).

The terms "eliminate", "abrogate", "elimination" or "abrogation" of binding to Protein A refers to an overall decrease of 100% in the binding of a modified immunoglobulin or fragment thereof to Protein A i.e. a complete loss of the binding of a modified immunoglobulin or fragment thereof to Protein A, detected by standard art known methods such as those described herein, as compared to a parental i.e. unmodified immunoglobulin or wild-type IgG or an IgG having the wild-type human IgG Fc region.

Similarly, the terms "decrease", "reduce", or "reduction" in binding to Protein G refers to an overall decrease of at least 25%, 30%, 40%, 50%, 60%, 70%, 80%, 85%, 90%, 95%, 97%, or 99% up to 100% (elimination) in the binding of a modified immunoglobulin or fragment thereof to Protein G detected by standard art known methods such as those described herein, as compared to a parental i.e. unmodified immunoglobulin or wild-type IgG or an IgG having the wild-type human IgG Fc region. In certain embodiments these terms alternatively may refer to an overall decrease of 10-fold (i.e. 1 log), 100-fold (2 logs), 1,000-fold (or 3 logs), 10,000-fold (or 4 logs), or 100,000-fold (or 5 logs).

The terms "eliminate", "abrogate", "elimination" or "abrogation" of binding to Protein G refers to an overall decrease of 100% in the binding of a modified immunoglobulin or fragment thereof to Protein G i.e. a complete loss of the binding of a modified immunoglobulin or fragment thereof to Protein G, detected by standard art known methods such as those described herein, as compared to a parental i.e. unmodified immunoglobulin or wild-type IgG or an IgG having the wild-type human IgG Fc region.

Similarly, the terms "decrease", "reduce", or "reduction" in binding to an affinity reagent refers to an overall decrease of at least 25%, 30%, 40%, 50%, 60%, 70%, 80%, 85%, 90%, 95%, 97%, or 99% up to 100% (elimination) in the binding of a modified immunoglobulin or fragment thereof to the affinity reagent detected by standard art known methods such as those described herein, as compared to a parental, i.e. unmodified immunoglobulin or wild-type IgG or an IgG having the wild-type human IgG Fc region. In certain embodiments these terms alternatively may refer to an overall decrease of 10-fold (i.e. 1 log), 100-fold (2 logs), 1,000-fold (or 3 logs), 10,000-fold (or 4 logs), or 100,000-fold (or 5 logs).

The terms "eliminate" , "abrogate", "elimination" or "abrogation" of binding to an affinity reagent refers to an overall decrease of 100% in the binding of a modified immunoglobulin or fragment thereof to the affinity reagent i.e. a complete loss of the binding of a modified immunoglobulin or fragment thereof to the affinity reagent detected by standard art known methods such as those described herein, as compared to a parental, i.e. unmodified immunoglobulin or wild-type IgG or an IgG having the wild-type human IgG Fc region.

"Bispecific antibodies" are monoclonal antibodies that have binding specificities for at least two different antigens. In certain embodiments, the bispecific antibodies are bispecific antibodies with one or more amino acid modifications in the VH region relative to the parental antibody. In certain embodiments, bispecific antibodies may be human or humanized antibodies. Bispecific antibodies may also be used to localize cytotoxic agents to cells which express a target antigen. These antibodies possess a target-antigen-binding arm and an arm which binds a cytotoxic agent, such as, e.g., saporin, anti-interferon-a, vinca alkaloid, ricin A chain, methotrexate or radioactive isotope hapten. Bispecific antibodies can be prepared as full length antibodies or antibody fragments. Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, (1983) Nature, 305: 537-40). Because of the random assortment of immunoglobulin heavy and light chains, these hybridomas (quadromas) produce a potential mixture of different antibody molecules, of which only one has the correct bispecific structure. The purification of the correct molecule, which is usually done by affinity chromatography steps, is rather cumbersome, and the product yields are low. Similar procedures are disclosed in WO 93/08829 and in Traunecker et al., (1991) EMBO J, 10: 3655-9. According to a different approach, antibody variable regions with the desired binding specificities (antibody-antigen combining sites) are fused to immunoglobulin constant region sequences. The fusion, for example, is with an immunoglobulin heavy chain constant region, comprising at least part of the hinge, CH2, and CH3 regions. In certain embodiments, the first heavy-chain constant region (CH1), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US4,676,980), and for treatment of HIV infection (WO91/00360, WO92/00373, and EP03089). Heteroconjugate antibodies may be made using any convenient cross-linking method. Suitable cross-linking agents are well known in the art (see US4,676,980), along with a number of cross-linking techniques. Antibodies with more than two valencies are also contemplated. For example, trispecific antibodies can be prepared (see Tutt A et al. (1991) J. Immunol. 147: 60-9).

In some embodiments the present disclosure provides a bispecific hetero-dimeric immunoglobulin or fragment thereof or a bispecific full-length antibody which binds to antigens selected from within the groups of: tumor antigens, cytokines, vascular growth factors and lympho-angiogenic growth factors. Preferably, the bispecific hetero-dimeric immunoglobulin or fragment thereof or the bispecific antibody binds to antigens selected from the group consisting of: HER1, HER2, HER3, EGFR, CD3, CD19, CD20, EpCAM, IgE and VLA-2. Preferably the bispecific hetero-dimeric immunoglobulin or fragment thereof or the bispecific antibody binds to HER2 and HER3. Preferably the bispecific hetero-dimeric immunoglobulin or fragment thereof or the bispecific antibody binds to CD3 and EpCAM or CD3 and HER2. Preferably the bispecific hetero-dimeric immunoglobulin or fragment thereof or the bispecific antibody binds to CD19 and IgE or CD20 and IgE.

The term "bacterial surface protein" includes a protein anchored or embedded in the solvent accessible surface of bacteria which binds to naturally occurring immunoglobulins or fragments thereof and/or artificial immunoglobulins or fragments thereof such as engineered variable domains or Fab fragments or Fc regions and the like. In another aspect the bacterial surface protein can be released as a soluble variant. Furthermore, for purposes of the present invention, "bacterial surface protein" includes a protein which includes modifications, such as deletions, additions and substitutions (which can be conservative in nature) to the native sequence and which retains binding to naturally occurring immunoglobulins or fragments thereof and/or artificial immunoglobulins or fragments thereof.

Examples of known bacterial surface proteins which interact with immunoglobulins are found in Gram-positive bacteria wherein these proteins serve as means for bacteria to establish residence at unique locations or evade the immune system. Several bacterial surface proteins that bind immunoglobulins or fragments thereof have been used in the analysis, purification and preparation of antibodies, or in other diagnostic and biological research applications. Bacterial surface proteins that bind immunoglobulins or fragments thereof include but are not limited to following examples:
*Protein A:* Protein A is a cell wall component produced by several strains of *Staphylococcus aureus* which consists of a single polypeptide chain. The Protein A gene product consists of five homologous repeats attached in a tandem fashion to the pathogen's cell wall. The five domains are approximately 58 amino acids in length and denoted EDABC, each exhibiting immunoglobulin binding activity (Tashiro M & Montelione GT (1995) Curr. Opin. Struct. Biol., 5(4): 471-481). The five homologous immunoglobulin binding domains fold into a three-helix bundle. Each domain is able to bind proteins from many mammalian species, most notably IgGs (Hober S et al., (2007) J. Chromatogr. B Analyt. Technol. Biomed. Life Sci., 848(1): 40-47). Protein A binds the heavy chain of most immunoglobulins within the Fc region but also within the Fab region in the case of the human VH3 family (Jansson B et al, (1998) FEMS Immunol. Med. Microbiol., 20(1): 69-78). Protein A binds IgG from various species including human, mouse, rabbit, and guinea pig but does not bind human IgG3 (Hober S *et al.,* (2007) *supra).* The inability of human IgG3 to bind Protein A can be explained by the H435R and Y436F substitutions in the human IgG3 Fc region (EU numbering, Jendeberg et al., (1997) J. Immunol. Methods, 201(1): 25-34). Besides IgG, Protein A also interacts with IgM and IgA.

The capacity of Protein A to bind antibodies with such high affinity is the driving motivation for its industrial scale use in biologic pharmaceuticals. Protein A used for production of antibodies in bio-pharmaceuticals is usually produced recombinantly in *E. coli* and functions essentially the same as native Protein A (Liu HF et al., (2010) MAbs, 2(5): 480-499).
Most commonly, recombinant Protein A is bound to a stationary phase chromatography resin for purification of antibodies. Optimal binding occurs at pH8.2, although binding is also good at neutral or physiological conditions (pH 7.0-7.6). Elution is usually achieved through pH shift towards acidic pH (glycine-HCl, pH2.5-3.0). This effectively dissociates most protein-protein and antibody-antigen binding interactions without permanently affecting protein structure. Nevertheless, some antibodies and proteins are damaged by low pH, and it is best to neutralize immediately after recovery by addition of 1/10th volume of alkaline buffer such as 1 M Tris-HCl, pH 8.0 to minimize the duration of time in the low-pH condition.
There are various commercially available Protein A chromatography resins. The main differences between these media are the support matrix type, Protein A ligand modification, pore size and particle size. The differences in these factors give rise to differences in compressibility, chemical and physical robustness, diffusion resistance and binding capacity of the adsorbents (Hober S *et al.,* (2007), *supra).* Examples of Protein A chromatography resins include but are not limited to the MabSelect SuRe™ Protein A resin and MabSelect™ Protein A resin from GE Healthcare as used in examples.

*Protein G:* Protein G is a bacterial cell wall protein isolated from group C and G *Streptococci.* DNA sequencing of native Protein G isolated from different *Streptococci* identified immunoglobulin binding domains as well as sites for albumin and cell surface binding. Depending on the strain both the immunoglobulin binding region and the albumin binding region consist of 2-3 independently folding units (Tashiro M & Montelione GT (1995) Curr. Opin. Struct. Biol., 5(4): 471-481). Protein G from strain G148 consists of 3 albumin and immunoglobulin binding domains respectively denoted ABD1, ABD2, and ABD3, and C1, C2, and C3 (Olsson A et al., (1987) Eur. J. Biochem., 168(2): 319-324.). Each immunoglobulin binding domain denoted C1, C2, and C3 is approximately 55 residues and separated by linkers of about 15 residues. All experimentally solved 3D structures of Protein G immunoglobulin binding domains show a highly compact globular structure without any disulfide bridges or tightly bound prosthetic groups (Sauer-Eriksson AE et al., (1995) Structure, 3(3): 265-278; Derrick JP & Wigley DB (1992) Nature, 359(6397): 752-754; Derrick JP & Wigley DB (1994) J. Mol. Biol., 243(5): 906-918; Lian LY et al., (1994) Nat. Struct. Biol., 1(6): 355-357). The structure comprises a four-stranded beta-sheet made up of two anti-parallel beta-hairpins connected by an alpha-helix.

Streptococcus strains from groups C and G show binding to all human subclasses of IgG including IgG3 in contrast to Protein A. Protein G also binds to several animal IgG including mouse, rabbit, and sheep (Bjorck L & Kronvall G (1984) J. Immunol., 133(2): 969-974; Akerstrom B et al., (1985) J. Immunol., 135(4): 2589-2592; Akerstrom B & Bjorck L (1986) J. Biol. Chem., 261(22): 10240-10247; Fahnestock SR et al., (1986) J. Bacteriol., 167(3): 870-880). Hence, Protein G exhibits a broader binding spectrum to subclasses of different species compared to Protein A.

In addition, Protein G binds to the Fab portion of IgGs with high affinity. The structure of the binding domain of streptococcal Protein G has been determined both alone (by NMR, Lian LY *et al.,* (1994) *supra),* and in complex with an IgG1 Fab (by x-ray crystallography, Derrick JP & Wigley DB (1992) *supra* and Derrick JP & Wigley DB (1994) *supra*)*.* All experimentally solved 3D structures showed a binding within the CH1 domain of IgG heavy chains.

Similarly to Protein A, recombinant Protein G produced in *E. coli* is routinely used to purify antibodies. The albumin and cell surface binding domains have been eliminated from recombinant Protein G to reduce non specific binding and, therefore, can be used to separate IgG from crude samples. Similarly to Protein A, recombinant Protein G is bound to a stationary phase chromatography resin for purification of antibodies. Optimal binding occurs at pH 5, although binding is also good at pH 7.0-7.2; as for Protein A, elution is also achieved through pH shift towards acidic pH (glycine-HCl, pH2.5-3.0). Examples of Protein G chromatography resins include but are not limited to the Protein G Sepharose™ 4 Fast Flow resin and HiTrap™ Protein G HP column from GE Healthcare as used in the Examples.

*Protein L:* Protein L is an immunoglobulin binding protein that was originally derived from the bacteria *Peptostreptococcus magnus,* but is now produced recombinantly (Bjorck L (1988) J. Immunol., 140(4): 1194-1197; Kastern W et al., (1992) J. Biol. Chem., 267(18): 12820-12825). Protein L has the unique ability to bind through kappa light chain interactions without interfering with an antibody's antigen binding site (Nilson BH et al., (1993) J. Immunol. Methods, 164(1): 33-40). This gives Protein L the ability to bind a wider range of immunoglobulin classes and subclasses than other antibody binding protein. Protein L will bind to all classes of immunoglobulins (IgG, IgM, IgA, IgE and IgD). Protein L will also bind single chain variable fragments (scFv) and Fab fragments (Nilson BH *et al.,* (1993) *supra*; Bottomley SP et al., (1995) Bioseparation, 5(6): 359-367). Protein L binds the human variable domains of kappa I, III, and IV subclasses and mouse kappa I subclass (Nilson BH *et al.,* (1992) *supra).* Examples of Protein L chromatography resins include but are not limited to the Protein L resin from Genescript as used in examples.

*M1 Protein & Protein H:* M1 Protein and Protein H are surface proteins simultaneously present at the surface of certain strains of *Streptococcus pyogenes.* Protein H has affinity for the Fc region of IgG (Akesson P et al., (1990) Mol. Immunol., 27(6): 523-531; Akesson P et al., (1994) Biochem. J., 300 (Pt 3): 877-886). Protein H binds to the Fc region of IgGs from human, monkeys and rabbits (Akesson P *et al.,* (1990), *supra;* Frick IM et al., (1995) EMBO J., 14(8): 1674-1679). M Proteins are also known to bind fibrinogen (Kantor FS (1965) J Exp Med, 121: 849-859), and previous work has demonstrated that M1 Protein from the API strain also has affinity for albumin and polyclonal IgG (Schmidt KH & Wadstrom T (1990) Zentralbl. Bakteriol., 273(2): 216-228).

Bacterial surface proteins are examples of affinity reagents. Other examples include but are not limited to artificially made proteins such as antibodies and fragments thereof such as: KappaSelect and LambdaFabSelect affinity resins from GE Healthcare (Glattbrugg, Switzerland) or CaptureSelect™ IgG-CH1 from Invitrogen AG (Basel, Switzerland).

The term "human FcRn" includes the human hetero-dimeric protein consisting of the IgG receptor FcRn large subunit p51 (also referred to as IgG Fc fragment receptor transporter alpha chain or FcRn transmembrane alpha chain; UniProt database accession number P55899) non covalently associated with beta2-microglobulin (UniProt database accession number P61769). Human FcRn is a MHC class I-related receptor for IgG and its expression has been identified in a variety of cell types which include epithelial cells, endothelial cells, macrophages and dendritic cells in rodents and humans of all ages (Roopenian DC & Akilesh S (2007) Nat. Rev. Immunol., 7(9): 715-725). Human FcRn plays a role in adult salvage of IgGs through its occurrence in the pathway of endocytosis in endothelial cells (Tesar DB & Bjorkman PJ (2010) Curr. Opin. Struct. Biol., 20(2): 226-233). FcRn receptors located in the acidic endosomes bind and recycle internalized IgGs to the cell surface. IgGs are released from FcRn receptors at the basic pH of blood, thereby escaping lysosomal degradation. This mechanism provides an explanation for the greater half-life of IgGs in the blood compared to other isotypes. FcRn forms a 2:1 complex with immunoglobulin molecules, i.e., two FcRn molecules bind to one Fc region or each of the two heavy chains from the Fc region binds one molecule of FcRn (Roopenian DC & Akilesh S (2007) *supra).* FcRn binds to the Fc region of IgGs at the junction between the CH2 and CH3 domains. Critical to the function of FcRn is its pH-dependent binding of IgG: at pH6.0, FcRn binds IgG, whereas IgG binding to FcRn is not detectable at pH 7.5. The strict pH dependence of the FcRn/IgG interaction suggests involvement of the imidazole side chains of histidines, which deprotonate over the pH range of 6.0-6.5. Mutation of the surface accessible histidine residues at positions 310 and 435 of the CH2 and CH3 domains severely reduced or eliminated IgG binding to FcRn. Human FcRn, Protein A and Protein G binding sites overlap in the Fc region of human IgGs (Nezlin R & Ghetie V (2004) Advances in Immunology, Academic Press. Volume 82: 155-215).

The term "chromatography" refers to protein liquid chromatography and includes fast protein liquid chromatography (FPLC) which is a form of liquid chromatography that is often used to analyze or purify mixtures of proteins. As in other forms of chromatography, separation is possible because the different components of a mixture have different affinities for two materials, a moving fluid (the mobile phase) which passes through a porous solid (the stationary phase). In FPLC, the mobile phase is an aqueous solution, or "buffer". The buffer flow rate can be operated under gravity flow or controlled by a positive-displacement pump which is normally kept at a constant rate, while the composition of the buffer can be varied by drawing fluids in different proportions from two or more external reservoirs. The stationary phase is a resin composed of beads, usually of cross-linked agarose, packed into a cylindrical glass or plastic column. FPLC resins are available in a wide range of bead sizes and surface ligands depending on the application.

In the most common FPLC strategies, ion exchange or affinity chromatography, a resin is chosen so that the protein of interest will bind to the resin while in buffer A (the running buffer) but become dissociated and return to solution in buffer B (the elution buffer). A mixture containing one or more proteins of interest is dissolved in 100% buffer A and loaded onto the column. The proteins of interest bind to the resin while other components are carried out in the buffer. The total flow rate of the buffer is kept constant; however, the proportion of buffer B (the "elution" buffer) can be increased from 0% to 100% in a gradual or stepwise manner according to a programmed change in concentration (the "gradient"). At some point during this process each of the bound proteins dissociates and appears in the effluent. Typical laboratory FPLC detection systems consist of one or two high-precision pumps, a control unit, a column, a detection system and a fraction collector. Although it is possible to operate the system manually, the components are normally linked to a personal computer or, in older units, a microcontroller. When operating with these semi-automated FLPC systems (for example when using the AKTA systems from GE healthcare), the effluent usually passes through two detectors which measure salt concentration (by conductivity) and protein concentration (by absorption of ultraviolet light at a wavelength of 280nm). Plots recording absorption of ultraviolet vs. total volume of spent mobile phase provide a visual representation of the purification process. These plots are termed chromatograms or chromatography traces. As each protein is eluted it appears in the effluent as a "peak" in protein concentration (and also as a graphical "peak" in the so-called chromatogram or chromatography trace) which can be collected for further use.

FPLC was developed and marketed in Sweden by Pharmacia in 1982 (now GE Healthcare) and was originally called fast performance liquid chromatography to contrast it with HPLC or high-performance liquid chromatography. FPLC is generally applied only to proteins; however, because of the wide choice of resins and buffers it has broad application. In contrast to HPLC the buffer pressure used is relatively low, typically less than 5 bar, but the flow rate is relatively high, typically 1-5 ml/min. FPLC can be readily scaled from analysis of milligrams of mixtures in columns with a total volume of 5ml or less to industrial production of kilograms of purified protein in columns with volumes of many litres. Eluted protein or mixtures thereof can be further analyzed by different analytical techniques, e.g. by SDS-PAGE, mass spectrometry and other known analytical techniques known in the art.

The process of "Affinity chromatography" involves the use of an affinity reagent as ligands which are cross-linked to the stationary phase and that have binding affinity to specific molecules or a class of molecules. Ligands can be bio-molecules, like protein ligands or can be synthetic molecules. Both types of ligand tend to have good specificity. The most commonly used protein ligand in production is the affinity reagent Protein A. In affinity chromatography when the solution (for example a crude cell supernatant containing a protein of interest) is loaded onto to the column the target protein is usually adsorbed while allowing contaminants (other proteins, lipids, carbohydrates, DNA, pigments, etc.) to pass through the column. The adsorbent itself is normally packed in a chromatography column; though the adsorption stage can be performed by using the adsorbent as a stirred slurry in batch binding mode. The next stage after adsorption is the wash stage, in which the adsorbent is washed to remove residual contaminants. The bound protein is then eluted in a semi-pure or pure form. Elution is normally achieved by changing the buffer or salt composition so that the protein can no longer interact with the immobilized ligand and is released. In some instances the protein of interest may not bind the affinity resin and affinity chromatography is directed at binding unwanted contaminants and the unbound fraction is therefore collected to isolate the protein of interest. Affinity chromatography can be performed in a fixed bed or a fluidised bed.

The term "gradient mode chromatography" refers to a chromatography method wherein the proportion of the "elution" buffer (buffer B) is increased from 0% to 100% in a gradual or stepwise manner.

The terms "capture-elution mode chromatography" or "capture-elution purification mode" or "capture-elution purification" refers to a chromatography method wherein the proportion of the "elution" buffer (buffer B) is not increased from 0% to 100% in a gradual or stepwise manner but rather directly applied at a 100% after capture and optionally a wash step with running buffer (buffer A).

### Purification of hetero-dimeric immunoglobulins

One of the most common methods for producing a bispecific antibody is to express two distinct antibodies in a single cell. Such a method gives rise to multiple species as the heavy chains of the distinct antibodies form both homo- and hetero-dimers. Since it is only the hetero-dimers that are required, these need to be separated from the mixture of homo- and hetero-dimers. The present invention provides a highly efficient method for the separation of hetero-dimeric immunoglobulins from a mixture of homo-and hetero-dimers by utilizing conventional Protein A and Protein G affinity chromatography.

As a first step (Example 1), the substitutions that would eliminate protein A or protein G binding were designed and tested in homo-dimeric immunoglobulin Fc fragments wherein both monomers carried the substitutions. New substitutions that reduce or eliminate binding to Protein G were selected and reduced to a minimal number of two.

In a second step (Example 2), the substitutions that reduce or eliminate binding to Protein A or G in homo-dimeric Fc fragments were assayed in homo-dimeric immunoglobulins based on FAB or scFv fragments. This allowed the identification of significant bottlenecks for both techniques. It was found that the presence of a variable heavy chain domain of the VH3 subclass within the heavy chain which has substitutions for reduced or no binding to Protein A, hampers any differential affinity methods based on Protein A; while the presence of a gamma CH1 constant domain within the heavy chain which has substitutions for reduced or no binding to Protein G, hampers the new differential affinity method based on Protein G. Solutions to these major impediments were found in the forms of framework substitutions that reduce or eliminate Protein A binding to the VH3 subclass for the differential affinity methods based on Protein A, and CH1 based substitutions that reduce or eliminate Protein G binding to the gamma CH1 domains for the new differential affinity method based on Protein G.

In a last step (Example 3), both Protein A and G differential affinity methods were used on their own and in combination with the aforementioned solutions described above to successfully purify hetero-dimeric immunoglobulins. More importantly, the Protein A and G differential affinity methods were combined and shown to enable the purification of hetero-dimeric immunoglobulins without the need of gradient elution when used sequentially, solely relying on two sequential capture-and-direct-elution chromatographic steps.
Binding to the human FcRn protects immunoglobulins from degradation and increase immunoglobulins' half-life, it therefore essential that mutations made in the Fc region that would eliminate the binding to Protein A or G did not disrupt binding to FcRn.
From Surface Plasmon Resonance (SPR) measurements (Example 4), it was found that the substitutions used in the new differential affinity method based on Protein G as shown herein, allowed for >90% retention of human FcRn binding while the previously described differential affinity methods based on protein A only retained about 75% of human FcRn binding. Making the new differential affinity method based on Protein G a technique of choice when developing hetero-dimeric immunoglobulins for human therapy. Substitutions that abrogate Protein G binding in the Fc region of immunoglobulins had no impact on Fc binding to the human FcγR3a.

Examples 5 to 7 characterise substitutions that abrogate Protein G binding in the Fc region and CH1 region of gamma immunoglobulins. Example 8 shows the design and functional testing of a therapeutic hetero-dimeric immunoglobulin based on the present invention.

### Examples

### Methods:

### General methods

### Construction of expression vectors

Mutations were introduced in cDNA coding sequences by standard overlap-PCR technique using appropriate cDNA templates. PCR products were digested with the HindIII and NotI DNA restriction enzymes, purified and ligated in a modified pcDNA3.1 plasmid (Invitrogen AG, Basel, Switzerland) carrying a CMV promoter and a Bovine Hormone poly-adenylation previously digested with the same DNA restriction enzymes . Light chains were independently ligated in the same expression vector. In all expression-vectors, secretion was driven by the murine VJ2C leader peptide.

### Expression of recombinant antibodies and fragments thereof

For transient expression, equal quantities of each engineered chains vectors were co-transfected into suspension-adapted HEK-EBNA cells (ATCC-CRL-10852) using Polyethyleneimine (PEI). Typically, 100ml of cells in suspension at a density of 0.8-1.2 million cells per ml is transfected with a DNA-PEI mixture. When recombinant expression vectors encoding each engineered chain genes are introduced into the host cells, the immunoglobulin construct is produced by further culturing the cells for a period of 4 to 5 days to allow for secretion into the culture medium (EX-CELL 293, HEK293-serum-free medium (Sigma, Buchs, Switzerland), supplemented with 0.1% pluronic acid, 4mM glutamine, and 0.25µg/ml geneticin). Cell-free culture supernatants containing the secreted immunoglobulins were prepared by centrifugation followed by filtration, and used for further analysis.

### Example 1 methods: Purification and testing of Fc fragment abrogated for Protein A or G binding

### Capture-elution mode chromatography

Supernatants were conditioned with 0.1 volume (V) of 1M Tris-HCl pH8.0 prior purification. Protein G Sepharose™ 4 Fast Flow (Protein A binding site mutants) or MabSelect SuRe™ resin (Protein G binding site mutants) (both from GE Healthcare Europe GmbH, Glattbrugg, Switzerland; catalogue numbers 17-0618-01 and 17-5438-01, respectively) were respectively added to conditioned supernatants. Mixtures were incubated overnight at 4°C. After incubation, bound proteins were washed with 10CVs of PBS pH7.4, eluted with 4 column volumes (CVs) of 0.1M Glycine pH3.0 and neutralised with 0.1V of 1M Tris-HCl pH8.0. Supernatant, flow through and elution fractions were analysed under non reduced conditions by SDS-PAGE (NuPAGE Bis-Tris 4-12% acrylamide, Invitrogen AG, Basel, Switzerland).

### Gradient mode chromatography

Post production, cell-culture supernatants containing homo-dimeric Fc variants were first purified in capture-elution mode chromatography using Protein G Sepharose™ 4 Fast Flow (Protein A binding site mutants) or MabSelect SuRe™ Protein A resin (Protein G binding site mutants) (see below, both resins from GE Healthcare Europe GmbH; catalogue numbers 17-0618-01 and 17-5438-01, respectively). Eluted material from capture-elution mode chromatography were subsequently loaded onto a 1ml HiTrap™ MabSelect SuRe™ Protein A column (Protein A binding site mutants) or a 1ml HiTrap™ Protein G HP column (Protein G binding site mutants). Both columns were pre-equilibrated in 0.2M phosphate citrate buffer pH8.0 and operated on an ÄKTApurifier™ chromatography system (both from GE Healthcare Europe GmbH; catalogue numbers 11-0034-93 and 17-0404-01, respectively) at a flow rate of 1ml/min. Elutions were performed with a pH linear gradient combining various amounts of two buffers (running buffer (A): 0.2M phosphate citrate buffer pH8.0 and elution buffer (B): 0.04M phosphate citrate buffer pH3.0 (Example 1.1) or 0.02M phosphate citrate buffer pH2.6 (Example1.2). The linear gradient went from 0% B to 100% B in five column volumes (CVs) (Example 1.1) or in ten CVs (Example 1.2). Eluted fractions were neutralised with 0.1V of 1M Tris-HCl pH8.0. Supernatant, flow through and elution fractions were analysed under non reduced conditions by SDS-PAGE (NuPAGE Bis-Tris 4-12% acrylamide, Invitrogen AG, Basel, Switzerland).

### Example 2 methods: Purification and testing of homo-dimeric immunoglobulins abrogated for Protein A or G binding

### Example 2.1: Homo-dimeric immunoglobulins abrogated for Protein A binding

### Purification and testing of FAB fragments abrogated for Protein A binding.

Post production, cell culture supernatants were conditioned with 0.1V of 1M Tris-HCl pH8.0. Protein L resin (Genescript, Piscataway, USA) was added to the conditioned supernatant and incubated overnight at 4°C. After incubation, bound proteins were washed with ten CVs of PBS pH7.4, eluted with 4CVs of 0.1M Glycine pH3.0, and finally neutralised with 0.1V of 1M Tris-HCl pH8.0. To assess Protein A binding, Protein L purified FAB were injected on a 1ml HiTrap MabSelect™ column (GE Healthcare Europe GmbH, Glattbrugg, Switzerland) at pH8.0 (Citric acid/Na₂HPO₄ buffer). Elution was performed with a pH linear gradient combining various amounts of two buffers (running buffer (A): 0.2 M phosphate citrate buffer pH8.0 and elution buffer (B): 0.04 M phosphate citrate buffer pH3.0). The linear gradient went from 0% B to 100% B in 5CVs. Eluted fractions were neutralised with 0.1V of 1M Tris-HC1pH8.0. Supernatant, flow through and elution fractions were analysed under non reduced conditions by SDS-PAGE (NuPAGE Bis-Tris 4-12% acrylamide, Invitrogen AG, Basel, Switzerland).

### SPR testing of FAB fragments abrogated for Protein A binding

cDNA encoding the human HER2 extracellular region fused to an IGHG1 Fc fragment was cloned into an expression vector similar to the heavy and light expression vectors described above and transiently transfected in HEK293E cells using the PEI method (see PCT publication No: WO12/131555). Supernatants were conditioned with 0.1V of 1 M Tris-HCl pH8.0 and the antigen purified by Protein A capture-elution chromatography as described in Example 1. For SPR experiments, a monoclonal mouse anti-human IgG (Fc) antibody sensor chip was used, this allowed for the capture the Fc fused recombinant HER2 antigen in the correct orientation (Human Antibody Capture Kit, catalogue number BR-1008-39, GE Healthcare Europe GmbH). Measurements were recorded on a BIAcore™ 2000 instrument (GE Healthcare Europe GmbH, Glattbrugg, Switzerland). Different dilutions of anti-HER2 FAB (50, 25, 12.5, 6.25, 3.13, 1.57, 0.78, 0.39nM) were injected over the sensor chip for 4min at 30µl/min. For each measurement, after seven minutes of dissociation, a 3M MgCl₂ solution was injected for 1min at 30 µl/min for regeneration. Data (sensorgram: fc2-fc1) were fitted with a 1:1 Langmuir. To account for the experimental variations in captured HER2-Fc at the beginning of each measurement, the Rmax value was set to local in all fits. Measurements were performed in duplicate, and included zero-concentration samples for referencing. Both Chi2 and residual values were used to evaluate the quality of a fit between the experimental data and individual binding models.

### Purification and testing of VH3 based homo-dimeric immunoglobulins abrogated for Protein A binding in their Fc and VH3 domains.

Gradient mode chromatography and capture-elution mode chromatography were performed according to the procedure described for Example 1.

### Example 2.2: Homo-dimeric immunoglobulins abrogated for Protein G binding Chromatography

Gradient mode chromatography and capture-elution mode chromatography were performed according to the procedure described for Example 1.

### SPR testing of FAB fragments abrogated for Protein G binding

cDNA encoding the human HER3 extracellular region (UniProt accession number: P21860 (ERBB3_HUMAN) residues 20-632, SEQ ID NO: 73, referred herein as HER3 antigen; UniProt Consortium (2013) Nucleic Acids Res., 41(Database issue):D43-7; http://www.uniprot.org/) fused to the amino acid sequence SAHHHHHHHH (SEQ ID NO: 100) was cloned into an expression vector similar to the heavy and light chain expression vectors described above and transiently transfected in HEK293E cells using PEI. Post production, cell-free supernatants were prepared, filtered sterilized, conditioned with 0.1 volume of 1 M Tris-HCl pH 8 and purified by Ni²⁺-NTA affinity chromatography (GE Healthcare Europe GmbH, Cat. No: 17-5318-02).

For SPR experiments, antibody variants were captured on a protein-A coupled CM5 research grade sensor chip (chip: GE Healthcare Europe GmbH; Cat. No: BR-1000-14; Protein A Sigma, Cat. No: P7837) with the recombinant HER3 antigen used as analyte. Measurements were run as follows: (capture) 150 RUs of antibody, (flow rate) 30 µl/min HBS-P buffer, (regeneration) glycine pH 1.5, (injection) 5 min, (dissociation) 8 min, (HER3 antigen concentration injected) 50, 25, 10, 5, 1, and 0.5 nM, (data fit) 1:1 binding without mass transfer. To account for the experimental variations in captured antibody at the beginning of each measurement, the Rmax value was set to local in all fits. Measurements were performed in triplicates, and included zero-concentration samples for referencing. Both Chi2 and residual values were used to evaluate the quality of a fit between the experimental data and individual binding models.

### Example 3 methods: Purification and testing of hetero-dimeric immunoglobulins abrogated for Protein A or G binding

### Examples 3.1 and 3.2: One step purification of hetero-dimeric immunoglobulins using Protein A or G

Post production, cell culture supernatants were adjusted to pH6.0 with 0.1V of 0.2M NaH₂PO₄ and loaded on 1ml HiTrap™ MabSelect SuRe™ column (Example 3.1) or on a 1ml HiTrap™ Protein G HP column (Example 3.2) at 1ml/min. After loading, bound proteins were washed extensively with 0.125M phosphate citrate buffer pH6.0. Elution was performed using two isocratic gradients combining two buffers (running buffer (A): 0.125M phosphate citrate buffer pH6.0 and elution buffer (B): 0.04M phosphate citrate buffer pH3.0). The hetero-dimeric immunoglobulin was eluted with the first isocratic gradient for 70CVs which varied as follows: 55% B in example 3.1, 90%B and 80%B in the first and second instances shown in Example 3.2. The non-abrogated homo-dimeric molecule was eluted in the second isocratic gradient at 100%B for 20CVs in all examples. Eluted fractions were neutralised with 0.1V of 1M Tris-HCl pH8.0. Supernatant, flow through and elution fractions were analysed under non reduced conditions by SDS-PAGE (NuPAGE Bis-Tris 4-12% acrylamide, Invitrogen AG, Basel, Switzerland).

### Example 3.3: Sequential purification of hetero-dimeric immunoglobulins using Protein A and Protein G.

Post production, cell culture supernatant was adjusted to pH6.0 with 0.1V of 0.2M NaH₂PO₄ and loaded on 1ml HiTrap MabSelect SuRe™ column at 1ml/min. After loading, bound proteins were washed extensively with 0.125M phosphate citrate buffer pH6.0. The hetero-dimeric immunoglobulin and the homo-dimeric immunoglobulin with no Protein G binding site were eluted with 10CVs of 0.04M phosphate citrate buffer pH3.0. Fractions containing the hetero- and homo-dimer mixture were pooled and further diluted with 10Vs of 0.125M phosphate citrate buffer pH6.0. The diluted mixture was then loaded on 1ml HiTrap Protein G HP column (GE Healthcare Europe GmbH, Glattbrugg, Switzerland) and bound proteins were extensively washed with 0.125M phosphate citrate buffer pH6.0. Hetero-dimeric immunoglobulins were eluted with 10CVs of 0.04M phosphate citrate buffer pH3.0. Eluted fractions were neutralised with 0.1V of 1M Tris-HCl pH8.0. Supernatant, flow through and elution fractions were analysed under non reduced conditions by SDS-PAGE (NuPAGE Bis-Tris 4-12% acrylamide, Invitrogen AG, Basel, Switzerland).

### Example 4 methods: SPR experiments on human FcRn and Fc gamma receptor 3a

### SPR experiments on human FcRn

Briefly, recombinant human FcRn was expressed in CHO-S cells. cDNA encoding the human FcRn alpha chain and beta2-microglobulin protein (UniProt accession numbers: P55899 (IgG receptor FcRn large subunit p51) residues 24-297and P61769 (Beta-2-microglobulin) residues 21-119, respectively) were cloned into two separate mammalian expression vectors containing puromycin resistance gene. CHO-S cells were stably co-transfected using PEI method described previously and stable clones were selected by their growth in presence of 7.5µg/ml puromycin. Growth medium was PowerCHO2 (Lonza Ltd, Basel, Switzerland). Prior purification, post-production supernatants were conditioned with 0.2M NaH₂PO₄, 0.1M NaCl pH6.0 in order to adjust pH to 6.0. FcRn was purified using human IgG sepharose6 Fast flow (GE Healthcare Europe GmbH, Glattbrugg, Switzerland) and eluted with PBS pH7.4. Measurements were recorded on a BIAcore™ 2000 instrument (GE Healthcare Europe GmbH, Glattbrugg, Switzerland). Each Immunoglobulin variant was immobilized on a CM5 sensor chip (GE Healthcare Europe GmbH, Glattbrugg, Switzerland) via amine coupling using a standard protocol provided by the manufacturer to reach an approximate response of 1500 RUs. FcRn binds to the Fc region of immunoglobulins at acidic pH in endosomes (pH6.0), but exhibits no binding at the basic pH of blood (pH 7.4) therefore all the measurement were made using a 20 mM sodium phosphate buffer pH6.0 - 0.1M NaCl (running buffer). Different dilutions of human FcRn (6000, 3000, 1500, 750, 375, 187.5, 93.8, 46.9nM) were injected for 3min at 10µl/min. After 3min of dissociation, PBS pH7.4 was injected for 1min at 30µl/min for surface regeneration. KD values were determined using a steady state affinity model. Equilibrium constants were determined by fitting the steady-state response versus the concentration of human FcRn over a range of concentrations to a 1:1 binding model (stoichiometry (n) = 1). Measurements were performed in triplicate, and included zero-concentration samples for referencing. Both Chi2 and residual values were used to evaluate the quality of a fit between the experimental data and individual binding models.

### SPR experiments on human Fc gamma receptor 3a

Human Fc gamma receptor 3a (abbreviated FcyR3a, UniProt accession number: P08637 (FCG3A_HUMAN) residues 17-192) was cloned and expressed similarly to the HER3 antigen described above. Purification was performed on Ig-G sepharose chromatography (GE Healthcare Europe GmbH, Cat. No: 17-0969-01) with an elution step at 0.1 M glycine pH 3. FcyR3a was further purified by gel filtration (SUPERDEX 75 10/300 GL, GE Healthcare Europe GmbH, Cat. No: 17-5174-01) to remove traces of IgG contaminants. Measurements were run as follows: (chip) CM5 chip coupled with 17000 RUs of antibody, (flow rate) 10 µl/min HBS-P, (regeneration) none, (injection) 8 min, (dissociation) 10 min, (FcyR3a concentration injected) 2500, 1250, 625, 312, 156, 78, and 39 nM, (data fit) steady state affinity.

### Example 5 methods: Immunogenicity prediction of Protein A and G abrogating substitutions

The predicted immunogenicity of the Protein A and Protein G abrogating mutations was investigated using Lonza's Epibase platform™ (Lonza, Applied Protein Services, Cambridge, UK).

### Example 6 methods: Thermo-stability analysis of Protein A and G abrogating substitutions

Thermo-stabilities of immnunoglobulins were compared by calorimetry. Measurements were carried out on a VP-DSC differential scanning microcalorimeter (MicroCal-GE Healthcare Europe GmbH). The cell volume was 0.128 ml, the heating rate was 1°C/min, and the excess pressure was kept at 64 p.s.i. All protein fragments were used at a concentration of 1-0.5 mg/ml in PBS (pH 7.4). The molar heat capacity of each protein was estimated by comparison with duplicate samples containing identical buffer from which the protein had been omitted. The partial molar heat capacities and melting curves were analysed using standard procedures. Thermograms were baseline corrected and concentration normalised before being further analysed using a Non-Two State model in the software Origin v7.0 (MicroCal-GE Healthcare Europe GmbH).

### Example 7 methods: Pharmacokinetic analysis of Protein G abrogating substitutions

Pharmacokinetics analyses were conducted in female Sprague Dawley rats. Each group contained four rats. Rats received 10 mg/kg of antibody by intravenous bolus injection. Blood samples were collected at 0.25h, 1h, 4h, 6h, and at 1, 2, 4, 7, 10, 14, 21, 28, 35 and 42 days post injection.

Serum levels of antibodies were determined by sandwich ELISA. HER2 antigen was coated onto 96-well ELISA plates at a concentration of 2 µg/ml and incubated overnight at 4°C.

After the plates were blocked with BSA, serum samples, reference standards (11 serial dilutions) and quality control samples were added to the plate and incubated for one hour at room temperature. After washing to remove unbound antibody, peroxidase-conjugated goat anti-human IgG_F(ab')₂ fragment specific detection antibody (Jackson Immunoresearch, distributor: MILAN ANALYTICA AG, Rheinfelden, Switzerland, Cat No: 109-035-006) was added and developed by standard colorimetric tetramethylbenzidine substrate (TMB, Pierce-Thermo Fisher Scientific-Perbio Science S.A., Lausanne, Switzerland, Cat. No.: 34021) according to manufacturer's recommendation. Absorbance values at 450 nm were recorded on a plate reader and the concentrations of antibody in serum samples were calculated using the reference standard curve generated in the sample plate utilizing four parametric regression model. The pharmacokinetics parameters were evaluated by non-compartment analysis using WinNonlin™ version 5.3 (Pharsight Corporation, Mountain View, CA, USA).

### Example 8 methods: Functional analysis of Protein A substitutions

### Phage display library construction and screening

Anti-HER3 antibodies can be isolated from antibody phage display libraries. To this aim, a scFv phage display library was screened. The library used herein was from synthetic origin with a diversity restricted to the CDR-H3 and CDR-L3 of the variable heavy and light chain, respectively. The library construction followed the protocol from Silacci M. et al. (2005, Proteomics, 5(9): 2340-50) with some modifications as described below.

The antibody scaffold used for the library was based on the heavy chain variable germline domain DP47 assembled with the light chain variable germline domain DPK22. A flexible linker based on G4S peptide repeats (GGGGSGGGGSGGGAS; SEQ ID NO: 94) was used for assembling the two variable domains. Five sub-libraries were cloned each resulting from the assembly of one heavy chain variable germline domain DP47 having a CDR-H3 with the following sequence K(X)nFDY (Kabat residues 94-102) wherein X is a random naturally occurring amino acid and n is 5 or 6 or 7 or 8 or 9, corresponding to a variable heavy chain domain with a CDR-H3 length of 8 or 9 or 10 or 11 or 12 residues, respectively with a pool of variable light chain domains resulting from the assembly of 3 different light chain variable germline domain DPK22 having a CDR-L3 with one of the following sequence CQQXGXXPXTF (SEQ ID NO: 96) or CQQXXGXPXTF (SEQ ID NO: 97) or CQQXXXXPXTF (SEQ ID NO: 98) (Kabat residues 88-98) wherein X is a random naturally occurring amino acid. Each sub-library of DP47-DPK22 scFv fragments had diversity between 1x10e9 and 3.7x10e9, once combined the five sub-libraries reached a total diversity of 1.05x10e10.

ScFv fragments recognizing human HER3 were isolated from the synthetic phage display library described above in a series of repeated selection cycles on recombinantly derived human HER3 antigen (see Methods section above). Methods to screen antibody phage display libraries are known (Viti F. et al., (2000) Methods Enzymol., 326: 480-505). Briefly, the immobilised antigen which had been previously coated on plastic immunotubes (overnight in PBS at a concentration of 20 µg/ml) was incubated with the library; tubes were washed with PBS/0.1% Tween 20. Bound phages were eluted with triethylamine and rescued as described by Silacci M. *et al., supra.* This selection process was repeated three times. Over one thousand clones from the second and third rounds of selection were expressed and analysed by ELISA against the target antigen. Positive clones were subjected to DNA sequencing and some of the unique clones were further analysed for their ability to bind cell lines expressing human HER3.

Since a large proportion of the isolated scFv fragments were specific for the first and second domains of human HER3, additional selections wherein the library pool of recombinant phages was depleted against a recombinant form of the first domain of human HER3 extracellular region were performed (human HER3 domain 1 fused to the amino acid sequence SAHHHHHHHH (SEQ ID NO: 100) was expressed as described for the HER3 extracellular region, UniProt accession number: P21860 (ERBB3_HUMAN) residues 20-209, SEQ ID NO: 74). This selection scheme allowed for the isolation of scFv fragments specific for the fourth domain of human HER3. Taken together the selection reported herein yielded scFv fragments having nanomolar affinities for human HER3 along with broad epitope coverage. ScFv fragments exhibiting high thermo-stability were isolated by mean of "cook- and-bind" ELISAs wherein secreted scFv fragments from raw bacterial supernatants were subjected to thermal challenge prior antigen ELISA (Miller BR et al., (2009) Methods Mol. Biol., 525:,279-89). Preferred scFv fragments were isolated from these selections. Most scFv fragments from different selections were found to bind HER3 positive cell lines by FACS.

### Human HER3 positive cell lines

Human cells expressing HER3 antigen on their surface have been described in PCT Publication No: WO10/108127 (Fuh G et al.). Calu-3 (ATCC-LGL standards, Teddington, UK; Cat. No: HTB-55), BxPC3 (ATCC-LGL standards; Cat. No: CRL-1687) and MDA-MB-175-VII (ATCC-LGL standards; Cat. No: HTB-25) are examples of human HER3 positive cell lines. Calu-3 cell line was primarily used herein to validate scFv fragments isolated by phage display.

### Cell culture conditions

Calu-3 cells were maintained in RPMI medium supplemented with 10% fetal calf serum (FCS) and 1% Glutamax/Penicillin/Streptomycin (Invitrogen AG).

### Cell proliferation assay

Calu-3 cells were seeded in 96-well plates (10,000 cells/well). The following day, cells were treated with antibodies or combinations of antibodies or bispecific antibodies diluted in medium containing 1% FCS. A final concentration of 3 nM of beta heregulin (R&D Systems, Abingdon, UK, Cat. No: 396-HB) was added after 1h of incubation with antibodies. alamarBlue® (AbD Serotec, Düsseldorf, Germany, Cat. No: BUF102) was added to the wells after 72h and the cells were incubated up to 24 h before fluorescence was read on a Biotek Synergy 2 plate reader (BioTek Instruments GmbH, Luzern, Switzerland) at an excitation wavelength of 540 nm and emission wavelength of 620 nm.

### Example 1: Mutations that reduce or abrogate binding to Protein A or G in homo-dimeric Fc fragments.

To identify Fc variants that would have reduced or no binding to Protein A or Protein G, engineered variants were designed and expressed as homo-dimers wherein both copies of the super antigen binding site were mutated. This allowed for the selection of substitutions that would lead to homo-dimeric immunoglobulins with little to no residual binding on the super antigen upon which the concept of differential purification is based.

### 1.1 Homo-dimeric Fc fragments with no or reduced binding to Protein A

To further investigate the usage of a mixed IGHG1-IGHG3 format, three IGHG1-IGHG3 mixed Fc variants were prepared and assayed for Protein A binding.

The first variant had a sequence originating from the naturally occurring human IGHG3 isotype wherein the hinge sequence was substituted for the entire hinge sequence from the naturally occurring human IGHG1 isotype (abbreviated Fc 133 - wherein the numerals in the name correspond to the immunoglobulin gamma isotype subclass of each domain in the order of: hinge/CH2/CH3; SEQ ID NO: 1).

The second Fc variant had a sequence originating from the naturally occurring human IGHG1 isotype wherein the entire CH3 domain sequence was substituted for the entire CH3 domain sequence from the naturally occurring human IGHG3 isotype (abbreviated Fc 113 - wherein the numerals in the name correspond to the immunoglobulin gamma isotype subclass of each domain in the order of: hinge/CH2/CH3; SEQ ID NO: 2).

The third variant had a sequence originating from the naturally occurring IGHG1 isotype wherein the substitutions H435R and Y436F described in US20100331527 were introduced (EU numbering; abbreviated Fc H435R/Y436F; SEQ ID NO: 3).

In addition, a human IGHG1 Fc fragment (abbreviated Fc IGHG1; SEQ ID NO: 4) was prepared and used as a positive control.

Homo-dimeric Fc variants and control Fc fragment were assayed for Protein A binding by gradient chromatography according to the protocol described the Methods section. FIG. 1 shows the chromatography profiles of the three variants and the Fc IGHG1 control fragment. None of the three variants retained Protein A binding while the Fc IGHG1 control fragment showed strong binding. It was concluded that homo-dimeric Fc variants encompassing the naturally occurring sequence of the human IGHG3 CH3 domain had reduced or no binding to Protein A.

Since the binding sites for Protein A and Protein G overlap at the CH2-CH3 domain interface, the Fc variants described above were tested for Protein G binding in capture-elution purification mode according to the protocol described the in Methods section. The results are shown in FIG.2. All three variants retained Protein G binding.

### 1.2 Homo-dimeric Fc fragments with no or reduced binding to Protein G

To identify critical Protein G binding residues in immunoglobulin heavy chains, the structure of a human Fc fragment in complex with the C2 domain of Protein G was used as a starting point for rational design (PDB code: 1FCC, www.pdb.org, Bernstein FC et al., (1977) Eur J Biochem, 80(2): 319-324 and Berman HM et al., (2000) Nucleic Acids Res, 28(1): 235-242; Sauer-Eriksson AE et al., (1995) Structure, 3(3): 265-278). Analysis of the interface between both molecules using the PISA Server (http://www.ebi.ac.uk/msd-srv/prot_int/pistart.html; Tina KG et al., (2007) Nucleic Acids Res., 35(Web Server issue): W473-476) identified a subset of 18 Protein G interacting residues in the Fc fragment, of which L251, M252, 1253, S254, Q311, E380, E382, S426, M428, N434, H435, Y436 and Q438 were the main contributors (EU numbering). Residues L251, 1253, H310, H433, H435 and Y436 were omitted from the original short list on the basis that these residues are known in the art to be essential for FcRn binding (Roopenian DC & Akilesh S, (2007) Nat. Rev. Immunol., 7(9): 715-25). In addition to physical-chemical properties, the nature of the substitutions was rationalized on the basis of sequence comparison between Protein G binding and non-binding immunoglobulin human isotypes (gamma isotypes *vs*. IGHA1, IGHA2, and IGHM; Bjorck L & Kronvall G (1984) J. Immunol., 133(2): 969-974).

Mutations were introduced in the context of the Fc fragment of human IGHG1 (SEQ ID NO: 4) by standard PCR based mutagenesis techniques. Substitutions made were not limited to but included the following changes: E380Y (SEQ ID NO: 5), E382R (SEQ ID NO: 6), E382Y (SEQ ID NO: 7), S426M (SEQ ID NO: 8), S426R (SEQ ID NO: 9), S426Y (SEQ ID NO: 10), S426W (SEQ ID NO: 11), Q438R (SEQ ID NO: 12), Q438Y (SEQ ID NO: 13) and the combinations E380A/E382A (SEQ ID NO: 14), E380M/E382L (SEQ ID NO: 15), E380Y/E382R (SEQ ID NO: 16), M252A/E380A/E382A (SEQ ID NO: 17), S254E/S426M/M428G (SEQ ID NO: 18), and S254M/E380M/E382L (SEQ ID NO: 19).

Homo-dimeric Fc variants and control Fc fragment were assayed for Protein G binding by gradient chromatography according to the protocol described in the Methods section.

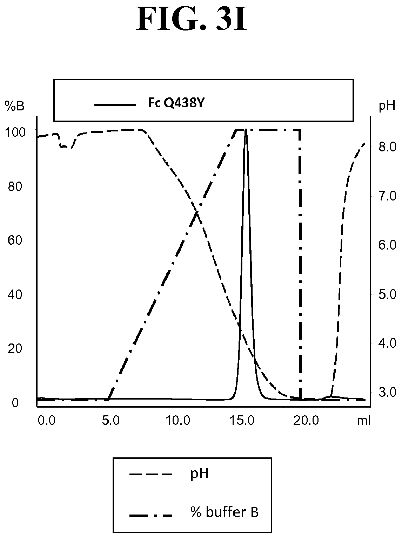

None of the tested single substitutions or their combinations led to a complete abrogation of binding to the Protein G HP column (FIG. 3). A small reduction in binding was observed with mutants combining substitutions at positions: S254, E380, and E382 (FIG. 3M) which were further investigated by groups of four or five with added substitutions at positions M252, M428, Y436, and Q438. Two new combinations were then prepared: M252A/E380A/E382A/Y436A/Q438A (SEQ ID NO: 20), and S254M/E380M/E382L/S426M/M428G (SEQ ID NO: 21). In addition, a third combination was prepared wherein previously investigated substitutions at positions S426 and M428 were further combined with substitutions at positions H433 and N434: S426M/M428G/H433D/N434A (SEQ ID NO: 22).

This new set of homo-dimeric Fc variants was also assayed for Protein G binding by gradient chromatography according to the protocol described in the Methods section. All three homo-dimeric Fc combination mutants showed complete abrogation of binding to the Protein G column, eluting during the loading step.

Since the binding sites for Protein A and Protein G overlap at the CH2-CH3 domain interface, the Fc variants described above were tested for Protein A binding in capture-elution purification mode according to the protocol described the in Methods section. The results are shown in FIG.4. All three variants bound Protein A thereby demonstrating that all variants retained Protein A binding.

To identify a minimal number of substitutions that would abrogate Protein G binding in homo-dimeric Fc fragments, the group of the four amino acid positions consisting of S426, M428, H433, and N434 were investigated in pairs, and in some cases substituted with different amino acids. The following combinations were prepared:
S426M/H433D (SEQ ID NO: 23), M428G/N434A (SEQ ID NO: 24), M428G/N434S (SEQ ID NO: 25), M428L/N434A (SEQ ID NO: 26), and M428L/N434S (SEQ ID NO: 27). Homo-dimeric Fc variants and the control Fc fragment were assayed for Protein G binding by gradient chromatography according to the protocol described in the Methods section. From FIG.5, it can be seen that only the homo-dimeric Fc combination mutants having the M428G and N434A or M428G and N434S substitutions had a complete abrogation of binding to the Protein G column, eluting during the loading step. It is worth mentioning that the combination of substitutions M428L/N434S which is known in the art for extending the serum half-life of human IGFtG1 immunoglobulins (Zalevsky J et al., Nat Biotechnol, 28(2): 157-159) and shown herein, did not lead to any reduction of Protein G binding (FIG. 5C).

Finally, the substitutions M428G and N434A were assessed in terms of their individual contribution towards the reduction or abrogation of Protein G binding. Two homo-dimeric Fc variants were prepared and assayed as above, one variant having the M428G substitution (SEQ ID NO: 28) and the other variant having the N434A substitution (SEQ ID NO: 29). Surprisingly, neither the M428G substitution nor the N434A substitution led to a reduction or an abrogation of Protein G binding (FIG. 6). Hence from these results, it was concluded that the combination of the M428G and the N434A substitutions is necessary and sufficient to induce a complete abrogation of Protein G binding in homo-dimeric Fc fragments.

Since the binding sites for Protein A and Protein G overlap at the CH2-CH3 domain interface, the Fc variants described above were tested for Protein A binding in capture-elution purification mode according to the protocol described in the Methods section. The results are shown in FIG.7. All variants retained Protein A binding.

### Example 2: Mutations that reduce or abrogate binding to Protein A or G in homo-dimeric immunoglobulins having homo-dimeric Fc fragments with reduced or no binding to Protein A or G

### 2.1 Homo-dimeric immunoglobulins with a reduced or no binding to Protein A

Methods to abrogate Protein A binding in homo-dimeric Fc fragments were shown in Example 1.1. To assess the use of Protein A abrogating methods in full-length homo-dimeric immunoglobulins, an anti-HER2 homo-dimeric immunoglobulin based on a mixed IGHG1-IGHG3 Fc format was prepared. The anti-HER2 homo-dimeric immunoglobulin was formatted similarly to a naturally occurring antibody and consisted of a FAB fragment with anti-HER2 specificity fused to the aforementioned Fc 133 fragment (abbreviated herein as anti-HER2 FAB-Fc 133; heavy chain with SEQ ID NO: 30 and light chain with SEQ ID NO: 31). Post transfection, the anti-HER2 FAB-Fc 133 homo-dimer was assayed for Protein A binding by gradient chromatography according to the protocol described in the Methods section. As shown in FIG.8A, the anti-HER2 FAB-Fc 133 homo-dimer still bound the commercial MabSelect SuRe™ Protein A column (GE Healthcare Europe GmbH). Since the Fc 133 variant was previously shown to have no binding to Protein A, further experiments were performed to investigate the contribution of the FAB region to the binding.

To assess the contribution of the FAB constant domains, the anti-HER2 homo-dimer described above was reformatted as an anti-HER2 scFv-Fc molecule wherein the scFv unit consisted of the parent immunoglobulin variable domains fused by a 15 amino-acid linker (abbreviated herein as anti-HER2 scFv-Fc 133; heavy chain with SEQ ID NO: 32). The resulting anti-HER2 scFv-Fc 133 homo-dimer was therefore identical to the parent anti-HER2 FAB-Fc 133 homo-dimeric immunoglobulin but lacked the CH1 and CK constant domains. As shown in FIG.8B, the scFv-Fc 133 homo-dimer exhibited Protein A binding as observed with the parent anti-HER2 homo-dimeric immunoglobulin. This finding led to the conclusion that the variable domains of the anti-HER2 FAB fragment were responsible for hampering the efficacy of the methods abrogating Protein A binding in the Fc portion of homo-dimeric immunoglobulins. More importantly, it was concluded that Protein A binding within variable domains of homo-dimeric immunoglobulins will prevent the preparation of hetero-dimeric immunoglobulins based on Protein A differential purification techniques.

All five domains of Protein A are known to bind the variable heavy chain domains from the VH3 variable domain subclass (Jansson B et al, (1998) FEMS Immunol. Med. Microbiol., 20(1): 69-78), a feature which is known to hamper the preparation of VH3 based FAB fragments following papain digestion of whole antibody molecules - since Protein A binds both VH3 based FAB and Fc fragments. The heavy chain variable domain found in the homo-dimeric anti-HER2 immunoglobulin or its scFv-Fc version belongs to the VH3-23 subclass, and explained why these homo-dimeric molecules bound Protein A in spite of having no Protein A binding site within their engineered Fc portions.

VH3 based immunoglobulins or fragments thereof are of major importance to the biotechnology industry. VH3 based molecules have been extensively developed since their ability to bind Protein A facilitates their functional pre-screening, and as such many synthetic or donor based phage display libraries or transgenic animal technologies used for antibody discovery are based on the VH3 domain subclass. In addition VH3 based molecules are often selected for their good expression and stability over other known heavy chain variable domain subclasses. A recombinant version of Protein A which does not bind VH3 based FAB fragments has been developed and commercialized by GE Healthcare under the trade name MabSelect SuRe™.

Since the MabSelect SuRe™ column was used herein for the Protein A binding assessment of the two homo-dimeric anti-HER2 immunoglobulins discussed above, it was concluded that the MabSelect SuRe™ column was unsuitable for the preparation of hetero-dimeric immunoglobulins having at least one VH3 variable domain when using Protein A differential purification techniques - since homo-dimeric species having no Protein A binding in their Fc regions will still bind Protein A through their VH3 domains.

To investigate substitutions that would abrogate or reduce VH3 based homo-dimeric immunoglobulins or fragments thereof, VH3 based FAB variants will need to be assayed for Protein A binding. Although the MabSelect SuRe™ resin kind is known to lack VH3 domain subclass binding, another commercial Protein A resin known as MabSelect™ does bind the VH3 domain subclass (also from GE healthcare) and was selected to analyse VH3 based FAB variants for Protein A binding.

The use of the MabSelect™ resin was validated by preparing a recombinant anti-HER2 FAB fragment derived from the parent anti-HER2 homo-dimeric immunoglobulin described earlier that is known to be of the VH3-23 variable domain subclass (abbreviated herein as anti-HER2 FAB; heavy chain with SEQ ID NO: 33 and light chain with SEQ ID NO: 31), and assaying the fragment onto the MabSelect™ and MabSelect SuRe™ columns (having a light chain based on the VK subclass I, the FAB fragment was first purified in capture-elution mode using protein L chromatography before Protein A gradient chromatography was performed on MabSelect™ or MabSelect SuRe™ columns, protocol for both columns followed the protocol described the Methods section). As shown in FIG. 8C, the VH3 based anti-HER2 FAB only bound to the MabSelect™ column, confirming that the MabSelect SuRe™ resin lacks binding to the VH3 based FAB fragments; at least as far as monomeric VH3 based FAB fragments are concerned, and further contrasted with the results observed earlier for the VH3 based homo-dimeric immunoglobulins with engineered Fc regions having no binding to Protein A. Conversely, the anti-HER2 FAB showed strong binding to the MabSelect™ column which offered the possibility to assay for VH3 based FAB variants that would have no or reduced Protein A binding.

To abrogate Protein A binding in VH3 based FAB fragments, critical Protein A binding residues in VH3 domains were identified from the crystal structure of a human FAB fragment in complex with the D domain of Protein A (PDB code: IDEE; www.pdb.org; Graille M et al., (2000) Proc. Natl. Acad. Sci. USA 97(10): 5399-5404). This analysis was used as a starting point for rational design wherein the nature of the substitutions undertaken was based on sequence comparison between Protein A binding and non Protein A binding VH subclasses from human origin. FIG. 9 shows an alignment of one representative framework for each human heavy chain variable domain subclass. Amino acid positions 15, 17, 19, 57, 59, 64, 65, 66, 68, 70, 81, 82a, and 82b (Kabat numbering) were identified as part of the protein-protein interaction interface between the D domain of Protein A and the VH3 based FAB fragment in the IDEE structure. Amongst human VH subclasses, VH3 is the only subclass to bind Protein A, and residues at equivalent amino acid sequence positions in other subclasses were selected to be the source of the substitutions with the view to abrogate or reduce Protein A binding while having potentially reduce immunogenicity - since these substitutions involved the replacement of one residue with another naturally occurring residue at the same equivalent amino acid position found in a non Protein A binding human VH subclass.

Mutations were introduced in the sequence of the aforementioned anti-HER2 FAB fragment by standard PCR based mutagenesis techniques, the following substitutions were made: G65S (heavy chain with SEQ ID NO: 34 and light chain with SEQ ID NO: 31), R66Q (heavy chain with SEQ ID NO: 35 and light chain with SEQ ID NO: 31), T68V (heavy chain with SEQ ID NO: 36 and light chain with SEQ ID NO: 31), Q81E (heavy chain with SEQ ID NO: 37 and light chain with SEQ ID NO: 31), N82aS (heavy chain with SEQ ID NO: 38 and light chain with SEQ ID NO: 31), and the combination R19G/T57A/Y59A (heavy chain with SEQ ID NO: 39 and light chain with SEQ ID NO: 31).

In addition, the T57A substitution (heavy chain with SEQ ID NO: 40 and light chain with SEQ ID NO: 31), and T57E substitution (heavy chain with SEQ ID NO: 41 and light chain with SEQ ID NO: 31) were made. T57A was previously shown to abrogate Protein A binding in WO2010/075548, and T57E was designed to engineer a charge switch (a change from a positively to a negatively charged amino acid). Having a light chain based on the VK subfamily I, FAB mutants were first purified in capture-elution mode using Protein L chromatography, and further assayed for Protein A binding using the MabSelect™ column operated under gradient mode as described in the Methods section. FIG. 10 shows that only T57A, T57E, G65S, Q81E, N82aS and the combination R19G/T57A/Y59A abrogated or reduced anti-HER2 FAB binding to the MAbSelect™ resin. Substitutions G65S, Q81E and N82aS are preferred when abrogating Protein A binding in VH3 based FAB fragments since these mutations substitute for the sequence equivalent residue found in non Protein A binding VH subclasses thereby potentially reducing immunogenicity.

Antibody affinity and specificity is essentially confined to the CDR regions, however, framework substitutions may impact on antibody properties as shown in the case of several humanized antibodies. To assess if the above substitutions may impact the specificity and/or the affinity of VH3 derived antibodies, two of the preferred FAB mutants were assayed for HER2 antigen binding by Surface Plasmon Resonance (SPR). SPR measurements with recombinant HER2 antigen were performed as described in the Methods section. Both preferred mutants showed identical binding to the HER2 antigen when compared to the original FAB molecule (FIG. 11) demonstrating that the substitutions had not impact in terms of specificity or affinity. It is therefore expected that these substitutions could be broadly used to engineer out Protein A binding in VH3 derived antibody molecules without significant loss of antigen binding.

Two of these preferred substitutions were introduced in the anti-HER2 homo-dimeric immunoglobulin and anti-HER2 scFv-Fc molecule described earlier, and variants were assayed for binding onto the MabSelect SuRe™ resin. The following variants were prepared: anti-HER2 scFv(G65S)-Fc 133 (heavy chain with SEQ ID NO: 42), anti-HER2 scFv(N82aS)-Fc 133 (heavy chain with SEQ ID NO: 43), anti-HER2 FAB(G65S)-Fc 133 (heavy chain with SEQ ID NO: 44 and light chain with SEQ ID NO: 31), and anti-HER2 FAB(N82aS)-Fc 133 (heavy chain with SEQ ID NO: 45 and light chain with SEQ ID NO: 31).

FIG. 12 shows the profiles from the MabSelect SuRe™ chromatography for all four mutants. All variants now displayed reduced to almost no binding to the MabSelect SuRe™ column indicating a successful removal of Protein A binding with the previously identified substitutions. More importantly, it was concluded that when combined with Protein A differential purification techniques, substitutions which abrogate or reduce VH3 based FAB affinity for Protein A will allow the preparation of hetero-dimeric immunoglobulins wherein at least one VH3 variable domain is present.

Variants described above were tested for Protein G binding in capture-elution purification mode according to the protocol described the Methods section. The results are shown in FIG. 13. All variants retained Protein G binding.

### 2.2 Homo-dimeric immunoglobulins with reduced or no binding to Protein G

Methods to abrogate Protein G binding in homo-dimeric Fc fragments were shown in Example 1.2. To assess the use of Protein G abrogating methods in full-length homo-dimeric immunoglobulins, an anti-HER3 homo-dimeric immunoglobulin based on the Fc M428G/N434A fragment was prepared. The anti-HER3 homo-dimeric immunoglobulin was formatted similarly to a naturally occurring antibody and consisted of a FAB fragment with anti-HER3 specificity fused to the aforementioned Fc M428G/N434A fragment (abbreviated herein as anti-HER3 FAB-Fc M428G/N434A, heavy chain with SEQ ID NO: 46 and light chain with SEQ ID NO: 47). Post transfection, the anti-HER3 FAB-Fc M428G/N434A homo-dimer was assayed for Protein G binding by gradient chromatography according to the protocol described in the Methods section. As shown in FIG. 14, the anti-HER3 FAB-Fc M428G/N434A homo-dimer still bound the commercial Protein G HP column (GE Healthcare Europe GmbH). Since the Fc M428G/N434A fragment was previously shown to have no binding to Protein G, a contribution of the FAB region to the binding was suspected. Such contribution will hamper the efficacy of the method abrogating Protein G binding in the Fc portion of homo-dimeric immunoglobulins, and more importantly will prevent the preparation of hetero-dimeric immunoglobulins based on this new differential purification technique.

FAB fragments from all human immunoglobulin gamma isotypes are known to bind Protein G within their CH1 domains (Nezlin R & Ghetie V, (2004) Advances in Immunology, Academic Press, Vol. 82: 155-215). Amongst human immunoglobulin isotypes, CH1 domains originating from IGHA1, IGHA2 and IGHM are known not to bind Protein G (Bjorck L & Kronvall G, *supra*). The differences in amino acid sequence between CH1 domains from gamma isotypes and CH1 domains from IGHA1, or IGHA2 or IGHM allow for the rational design of substitutions that would reduce or abrogate Protein G binding in CH1 domains from gamma isotypes while potentially having low immunogenicity. FIG. 15 shows the IMGT sequence alignment of the human CH1 domain from IGHG1 against the CH1 domain sequences from human IGHA1 and human IGHM (IMGT®, *supra*). Since the IMGT® numbering is based on the comparative analysis of the 3D structures of the immunoglobulin super-family domains, it defines the 3D equivalent positions between CH1 domains. Hence substitutions to reduce or abrogate Protein G binding within the CH1 domain of IGHG1 were selected from the sequence alignment shown in FIG. 15. Another input to select which 3D positions would be substituted was the analysis of the crystal structure of a mouse FAB fragment in complex and the third domain of Protein G (PDB code 1IGC, www.pdb.org, *supra* ; Derrick JP & Wigley DB, (1994) J. Mol. Biol., 243: 906-918). Two beta strands, (strands A (EU numbering 122 to 136) and strand G (EU numbering 212 to 215), FIG. 15) and a loop structure (FG loop (EU numbering 201 to 211), FIG.15) within the CH1 domain crystal structure appeared to mediate most of the protein-protein interactions and were the focus of the engineering work.

To assess the use of human IGHA1 or IGHM derived substitutions, the following mutants were prepared in the background of the anti-HER3 FAB-Fc M428G/N434A homo-dimeric immunoglobulin described above: a variant wherein the entire CH1 domain from IGHG1 was replaced with the entire CH1 domain from IGHA1 (abbreviated herein as anti-HER3 FAB(IGHA1)-Fc M428G/N434A; heavy chain with SEQ ID NO: 48 and light chain with SEQ ID NO: 47), a variant wherein the entire CH1 domain from IGHG1 was replaced with the entire CH1 domain from IGHM (abbreviated herein as anti-HER3 FAB(IGHM)-Fc M428G/N434A; heavy chain with SEQ ID NO: 49 and light chain with SEQ ID NO: 47), a variant wherein the IGHG1 CH1 domain strand A, strand G, and part of the FG loop sequences were replaced with the IGHA1 CH1 domain strand A, strand G, and part of the FG loop sequences (abbreviated herein as anti-HER3 FAB(IGHA1-A-FG/G)-Fc M428G/N434A, heavy chain with SEQ ID NO: 50 and light chain with SEQ ID NO: 47), a variant wherein the IGHG1 CH1 domain strand A, strand G, and part of the FG loop sequences were replaced with the IGHM CH1 domain strand A, strand G, and part of the FG loop sequences (abbreviated herein as anti-HER3 FAB(IGHM-A-FG/G)-Fc M428G/N434A; heavy chain with SEQ ID NO: 51 and light chain with SEQ ID NO: 47), a variant wherein the IGHG1 CH1 domain strand A sequence was replaced with the IGHA1 CH1 domain strand A sequence (abbreviated herein as anti-HER3 FAB(IGHA1-A)-Fc M428G/N434A; heavy chain with SEQ ID NO: 52 and light chain with SEQ ID NO: 47), a variant wherein the IGHG1 CH1 domain strand G and part of the FG loop sequences were replaced with the IGHA1 CH1 domain strand G and part of the FG loop sequences (abbreviated herein as anti-HER3 FAB(IGHA1-FG/G)-Fc M428G/N434A; heavy chain with SEQ ID NO: 53 and light chain with SEQ ID NO: 47), a variant wherein the IGHG1 CH1 domain strand A sequence was replaced with the IGHM CH1 domain strand A sequence (abbreviated herein as anti-HER3 FAB(IGHM-A)-Fc M428G/N434A; heavy chain with SEQ ID NO: 54 and light chain with SEQ ID NO: 47), and a variant wherein the IGHG1 CH1 domain strand G and part of the FG loop sequences were replaced with the IGHM CH1 domain strand G and part of the FG loop sequences (abbreviated herein as anti-HER3 FAB(IGHM-FG/G)-Fc M428G/N434A; heavy chain with SEQ ID NO: 55 and light chain with SEQ ID NO: 47). Post transfection, the anti-HER3 FAB-Fc variants were assayed for Protein G binding by gradient chromatography according to the protocol described in the Methods section. FIG. 16 and FIG. 17 show the Protein G binding profiles for the IGHA1 and IGHM based variants, respectively. From these results, it was concluded that replacing the entire CH1 domain sequence from IGHG1 with the entire CH1 domain sequence from either IGHA1 or IGHM allows for complete abrogation of Protein G binding in gamma FAB based homo-dimeric immunoglobulins wherein Fc regions have also no or reduce binding to Protein G. In addition, it was found that abrogation through single strand exchange was only successful when using strands G with parts of the FG loops from IGHA1 or IGHM while replacing with strands A had little to no impact on Protein G binding.

To identify a minimal number of substitutions that would abrogate Protein G binding in gamma isotype FAB fragments, additional substitutions derived from the analysis of the CH1 domain strand G and part of the FG loop sequences were investigated. The following pairs of substitutions were tested: T209P/K210S (FG loop), K213V/K214T (strand G), T209G/K210N (FG loop) and D212E/K214N (strand G) (EU numbering; EU position 209, 210, 212, 213, and 214 correspond to IMGT position 115, 116, 118, 119, and 120, respectively). The first two combinations were derived from the analysis of the IGHG1 CH1 domain strand G and part of the FG loop sequences against the IGHA1 CH1 domain strand G and part of the FG loop sequences, while the other two pairs of substitutions were derived from the analysis of the IGHG1 CH1 domain strand G and part of the FG loop sequences against the IGHM CH1 domain strand G and part of the FG loop sequences. Variants were formatted as above, and can be described as follows: an anti-HER3 FAB(T209G/K210N)-Fc M428G/N434A with heavy chain with SEQ ID NO: 56 and light chain with SEQ ID NO: 47, an anti-HER3 FAB(T209P/K210S)-Fc M428G/N434A with heavy chain with SEQ ID NO: 57 and light chain with SEQ ID NO: 47, an anti-HER3 FAB(D212E/K214N)-Fc M428G/N434A with heavy chain with SEQ ID NO: 58 and light chain with SEQ ID NO: 47, and an anti-HER3 FAB(K213V/K214T)-Fc M428G/N434A with heavy chain with SEQ ID NO: 59 and light chain with SEQ ID NO: 47.

Homo-dimeric immunoglobulin variants were then assayed for Protein G binding by gradient chromatography according to the protocol described the Methods section. FIG. 18 shows the gradient chromatography profiles for the IGHA1 derived substitutions, both T209P/K210S and K213V/K214T substitutions were able to completely abrogate Protein G binding. In the case of the IGHM based substitutions, only the T209G/K210N substitutions led to a complete abrogation of Protein G binding, the D212E/K214N substitutions had no impact on Protein G binding (FIG. 19). From these results, it was concluded that substitutions T209P/K210S, K213V/K214T, and T209G/K210N (EU numbering) can abrogate Protein G binding in gamma isotype FAB based homo-dimeric immunoglobulins wherein Fc regions have also no or reduced binding to Protein G. More importantly substitutions that abrogate or reduce gamma isotype FAB binding to Protein G when combined with the Protein G differential purification technique described in Example 1.2 will allow for the preparation of hetero-dimeric immunoglobulins with at least one CH1 domain present.

Variants described above were tested for Protein A binding in capture-elution purification mode. The results are shown in FIG.20. All variants retained Protein A binding.

Since CH1 domain sequences from gamma isotypes are unchanged at amino acid positions 209 and 210, it is expected that the substitutions at position 209 and 210 shown herein can abrogate CH1 domain binding to Protein G in all gamma isotype CH1 domains. Between the two positions, position 209 is expected to play a major role in the CHI-Protein G interaction. Analysis of the hydrogen bond network in 1IGC using the PDBsum online tool (http://www.ebi.ac.uk/pdbsum/, Laskowski RA et al., (1997) Trends Biochem. Sci., 22(12): 488-490) revealed an important hydrogen bond interaction between the side chain of T209 and an amino acid side chain from Protein G (residue T21, numbering according to the sequence of 1IGC). K210 was also shown to make a hydrogen bond interaction with an amino acid from Protein G (E20, numbering according to the sequence of 1IGC) but since the interaction only involves main chain atoms, it is expected to be less prone to disruption by amino acid substitutions.

Position K213 is conserved across gamma isotypes but K214 in IGHG1 corresponds to R214 in IGHG3 and IGHG4, a conservative amino-acid change since a positive charge is maintained at this position. In IGHG2, position 214 is a threonine and as such represents a non-conservative change. Since the K213V/K214T substitutions abrogated Protein G binding to the IGHG1 CH1 domain (as shown herein), it is expected that substitutions at position 213 will be sufficient to abrogate Protein G binding in all gamma isotypes. Similarly to position 209, the side chain of K213 also mediates an important hydrogen bond interaction with an amino acid side chain from Protein G (residue T16, numbering according to the sequence of 1IGC), while similarly to K210, K214 only makes hydrogen bond interactions involving main chain atoms with amino acids from Protein G (K15 and T16, numbering according to the sequence of 1IGC), interactions which are therefore expected to be less prone to disruption by amino acid substitutions.

To identify single substitutions that would abrogate Protein G binding within FAB fragments from gamma isotypes, the following single substitutions were investigated: T209P, K213V (both IGHA1 derived substitutions), and T209G (IGHM derived substitution).

Variants were formatted as above, and can be described as follows: an anti-HER3 FAB(T209P)-Fc M428G/N434A with heavy chain with SEQ ID NO: 75 and light chain with SEQ ID NO: 47, an anti-HER3 FAB(K213V)-Fc M428G/N434A with heavy chain with SEQ ID NO: 76 and light chain with SEQ ID NO: 47, and an anti-HER3 FAB(T209G)-Fc M428G/N434A with heavy chain with SEQ ID NO: 77 and light chain with SEQ ID NO: 47. FIG. 18C and 18D show the gradient chromatography profiles for the IGHA1 derived substitutions, both T209P and K213V substitutions were able to completely abrogate Protein G binding. In the case of the IGHM based substitution, T209G led to a complete abrogation of Protein G binding (FIG. 18E). From these results, it was concluded that substitutions T209P, K213V, and T209G (EU numbering) can abrogate Protein G binding in FAB fragments from gamma isotypes within homo-dimeric immunoglobulins wherein Fc regions have also no or reduced binding to Protein G. More importantly, when combined with the Protein G differential purification technique described in Example 1.2, substitutions that abrogate or reduce FAB binding to Protein G will allow the preparation of hetero-dimeric immunoglobulins having at least one CH1 domain.

To assess if the above substitutions may impact antigen specificity and/or affinity in derived antibodies, all three CH1 single mutant antibodies described above were assayed for HER3 antigen binding by SPR. Measurements on recombinant HER3 antigen were performed as described in the Methods section. All three mutants showed identical binding to the antigen when compared to the control antibody (FIG. 18F) demonstrating that the substitutions had not impact in terms of specificity or affinity. It is therefore expected that these substitutions could be broadly used to engineer out Protein G binding within FAB fragments from gamma isotypes without significant loss of antigen binding.

### Example 3: Purification of hetero-dimeric immunoglobulins having differential purification for Protein A and/or G.

Methods to abrogate or reduce Protein A or Protein G binding in homo-dimeric immunoglobulins were shown in Examples 1 and 2. These methods were developed to allow the purification of hetero-dimeric immunoglobulins either on their own or in combination. When used on their own, both methods require gradient mode chromatography to allow for the separation of hetero-dimers of heavy chains wherein one heavy chain has reduced or no binding to Protein A or G when compare to the other heavy chain. When used in combination, these methods can be conveniently used for the preparation of hetero-dimers of heavy chains by performing two capture-elution chromatography steps in series, one over Protein A and the other over Protein G - in no particular order. The hetero-dimeric immunoglobulins encompassing both technologies consist of one heavy chain able to bind Protein A but having reduced or no binding to Protein G, paired with another heavy chain able to bind Protein G but having reduced or no binding to Protein A. The hetero-dimeric immunoglobulin of interest will therefore have differential purification properties over both of its homo-dimeric species; the two possible homo-dimeric species having either no binding to Protein A or no binding to Protein G.

Importantly, only the combination of these two methods allows for the homogenous preparation of hetero-dimeric immunoglobulins in capture-elution mode since at each affinity step one of the two homo-dimeric immunoglobulin contaminants is efficiently removed without the use of gradient mode chromatography - since it does not bind the affinity resin. This is of particular interest since capture-elution mode chromatography is preferred for industrial scale preparation. Hence the combination of these two technologies and the sequential use of Protein A chromatography followed by Protein G chromatography (or vice versa) will allow the preparation of hetero-dimeric immunoglobulins of the highest purity (above 95%, more preferably above 98%) in capture-elution mode without the need to run any form of gradient chromatography.

### 3.1 Hetero-dimeric immunoglobulins having differential purification for Protein A

To assess the use of Protein A abrogating methods for the preparation of hetero-dimeric immunoglobulins, an anti-HER2/HER3 hetero-dimeric immunoglobulin based on a mixed IGHG1-IGHG3 Fc format was prepared.

When making hetero-dimeric immunoglobulins based on hetero-dimers of heavy chains because naturally occurring heavy chains have identical molecular weights, it is impossible to identify hetero-dimers from homo-dimers by SDS-PAGE analysis. Consequently to generate a difference in SDS-PAGE mobility and facilitate the identification of hetero-dimer formation, a scFv-FAB format was used wherein one heavy chain carries a FAB fragment and the other heavy chain carries a scFv fragment.

The anti-HER3 heavy chain was formatted as described in Example 2 and consisted of a FAB fragment with anti-HER3 specificity fused to the aforementioned Fc 133 fragment (abbreviated herein as anti-HER3 FAB-Fc 133; heavy chain with SEQ ID NO: 60 and light chain with SEQ ID NO: 47). Importantly, the variable heavy chain domain found in the anti-HER3 FAB-Fc 133 heavy chain belongs to the VH subclass two and does not bind Protein A. The anti-HER2 heavy chain was formatted as described in Example 2 and consisted of an anti-HER2 scFv-Fc heavy chain with a Fc portion from the naturally occurring IGHG1 isotype (abbreviated herein as anti-HER2 scFv-Fc IGHG1; heavy chain with SEQ ID NO: 61). Importantly, the variable heavy chain domain found in the anti-HER2 scFv-Fc heavy chain belongs to the VH subclass three (VH3) and does bind Protein A.

The anti-HER2/HER3 hetero-dimeric immunoglobulin resulting from the covalent association of the anti-HER3 FAB-Fc 133 heavy chain with anti-HER2 scFv-Fc IGHG1 heavy chain was therefore expected to have one heavy chain with no binding site for Protein A (the anti-HER3 FAB-Fc 133 heavy chain is abrogated in its Fc region for Protein A binding and there is no Protein A binding site present in its variable heavy chain domain), and one heavy chain with two binding sites for Protein A (the anti-HER2 scFv-Fc IGHG1 heavy chain has the natural Protein A binding site found in the IGHG1 Fc region and has a second Protein A binding site present in its VH3 domain). This particular heavy chain combination results in the production of the anti-HER2/HER3 hetero-dimeric immunoglobulin of interest with a total of two Protein A binding sites as well as two homo-dimeric immunoglobulin species, one having no binding site for Protein A while the second species has a total of four. The difference in the number of Protein A binding sites between hetero and homo-dimeric species allows for efficient separation of all three molecules by gradient chromatography as shown below.

Post production, the cell culture supernatant containing all three species was assayed for Protein A binding by gradient chromatography according to the protocol described in the methods. As shown in FIG. 21, all three species were resolved upon Protein A gradient chromatography, the species having no binding site did not bind the MabSelect SuRe™ Protein A column, while the hetero-dimeric immunoglobulin of interest eluted before the homo-dimeric species having the greatest number of Protein A binding sites.

This last example shows that when implementing Protein A abrogating methods to purify hetero-dimers of heavy chains wherein only one VH3 domain is present, hetero-dimer purification can only successful if the VH3 domain is engineered to be part of the heavy chain which binds to Protein A and which has not been modified in its Fc region.

When dealing with hetero-dimers of heavy chains wherein each heavy chain carries one VH3 domain, the substitutions shown in Example 2.1 can be used to mutate Protein A binding in at least one VH3 domain or both, thereby preserving the Protein A binding site imbalance which is the basis of this differential purification technique.

### 3.2 Hetero-dimeric immunoglobulins having differential purification for Protein G

To assess the use of the Protein G abrogating method for the preparation of hetero-dimeric immunoglobulins, an anti-HER2/HER3 hetero-dimeric immunoglobulin based a minimal number of substitutions that would abrogate Protein G binding in homo-dimeric Fc fragments was prepared. Similarly to Example 3.1, a scFv-FAB format was used to generate a difference in SDS-PAGE mobility and facilitate hetero-dimer identification.

The anti-HER2 heavy chain was formatted as described in Example 2 and corresponded to a scFv-Fc type of heavy chain consisting of the anti-HER2 scFv used in Example 2.1 and the aforementioned Fc M428G/N434A fragment (abbreviated herein as anti-HER2 scFv-Fc M428G/N434A; heavy chain with SEQ ID NO: 62).

The anti-HER3 heavy chain was formatted as described in Example 2 and consisted of a FAB fragment with anti-HER3 specificity fused to a naturally occurring IGHG1 Fc fragment (abbreviated herein as anti-HER3 FAB-Fc IGHG1; heavy chain with SEQ ID NO: 63 and light chain with SEQ ID NO: 47).

The anti-HER2/HER3 hetero-dimeric immunoglobulin resulting from the covalent association of the anti-HER2 scFv-Fc M428G/N434A heavy chain with anti-HER3 FAB-Fc IGHG1 heavy chain was therefore expected to have one heavy chain with no binding site for Protein G (the anti-HER2 scFv-Fc M428G/N434A is abrogated in its Fc portion for Protein G binding and there is no additional Protein G binding site present in the scFv format, i.e. there is no CH1 domain), and one heavy chain with two binding sites for Protein G (the anti-HER3 FAB-Fc IGHG1 heavy chain has the natural Protein G binding site found in the IGHG 1Fc region and has a second Protein G binding site present in its CH1 domain). This particular heavy chain combination results in the production of the anti-HER2/HER3 hetero-dimeric immunoglobulin of interest with a total of two Protein G binding sites as well as two homo-dimeric immunoglobulin species, one having no binding site for Protein G while the second species has a total of four. The difference in the number of Protein G binding sites between hetero and homo-dimeric species allows for efficient separation of all three molecules by gradient chromatography. Post production, the cell culture supernatant containing all three species was assayed for Protein G binding by gradient chromatography according to the protocol described in the Methods section. As shown in FIG. 22, all three species were resolved, the species having no binding site did not bind the Protein G HP column, while the hetero-dimeric immunoglobulin of interest eluted before the homo-dimeric species having the greatest number of Protein G binding sites.

In the above experiment, usage of the Protein G abrogating method for the purification of hetero-dimeric immunoglobulins was restricted to a format wherein the heavy chain carrying the substitutions that abrogate Protein G binding had no CH1 domain, since using a FAB format will inherently restore Protein G binding of the unwanted homo-dimeric species. By abrogating the Protein G binding site in the CH1 domain of the FAB fragment, hetero-dimeric immunoglobulins wherein a FAB fragment is present within the heavy chain carrying substitutions that abrogate Protein G binding in the Fc region can be prepared; an example is provided below.

Similarly to the last experiment, an anti-HER2/HER3 hetero-dimeric immunoglobulin was prepared using a scFv-FAB format to generate a difference in SDS-PAGE mobility and facilitate hetero-dimer identification.

The anti-HER2 heavy chain was the anti-HER2 scFv-Fc IGHG1 heavy chain described in Example 3.1 (heavy chain with SEQ ID NO: 61). The anti-HER3 heavy chain was the anti-HER3 FAB(IGHA1-FG/G)-Fc M428G/N434A heavy chain described in Example 2.2 (heavy chain with SEQ ID NO: 53 and light chain with SEQ ID NO: 47).

The anti-HER2/HER3 hetero-dimeric immunoglobulin resulting from the covalent association of the anti-HER2 scFv-Fc IGHG1 heavy chain with the anti-HER3 FAB(IGHA1-FG/G)-Fc M428G/N434A heavy chain was therefore expected to have one heavy chain with no binding site for Protein G (the anti-HER3 FAB(IGHA1-FG/G)-Fc M428G/N434A heavy chain is abrogated for Protein G binding both its Fc region and CH1 domain), and one heavy chain with only one binding site for Protein G (the anti-HER2 scFv-Fc IGHG1 heavy chain has the natural Protein G binding site found in the IGHG1Fc region and there is no additional Protein G binding site present in the scFv format, i.e. there is no CH1 domain). This particular heavy chain combination results in the production of the anti-HER2/HER3 hetero-dimeric immunoglobulin of interest with only one Protein G binding site as well as two homo-dimeric immunoglobulin species, one having no binding site for Protein G while the second species has a total of two. The difference in the number of Protein G binding sites between hetero and homo-dimeric species allows for efficient separation of all three molecules by gradient chromatography. Post production, the cell culture supernatant containing all three species was assayed for Protein G binding by gradient chromatography according to the protocol described in the Methods section. As shown in FIG. 23, all three species were resolved, the species having no binding site did not bind the Protein G HP column, while the hetero-dimeric immunoglobulin of interest eluted before the homo-dimeric species having the greatest number of Protein G binding sites.

### 3.3 Hetero-dimeric immunoglobulins having differential purification for Protein A and Protein G

The methods for the differential purification of hetero-dimeric immunoglobulins on Protein A or Protein G can be combined in a sequential manner to easily purify hetero-dimeric immunoglobulins in capture-elution mode, i.e. without the need to run any form of gradient chromatography; two examples are shown below.

Similarly to Examples 3.1 and 3.2, an anti-HER2/HER3 hetero-dimeric immunoglobulin was prepared using a scFv-FAB format to generate a difference in SDS-PAGE mobility and facilitate hetero-dimer identification. The anti-HER2 heavy chain was the anti-HER2 scFv-Fc M428G/N434A heavy chain described in Example 3.2 (heavy chain with SEQ ID NO: 62). The anti-HER3 heavy chain was the anti-HER3 FAB-Fc 133 heavy chain described in Example 3.1 (heavy chain with SEQ ID NO: 60 and light chain with SEQ ID NO: 47).

The anti-HER2/HER3 hetero-dimeric immunoglobulin resulting from the covalent association of the anti-HER2 scFv-Fc M428G/N434A heavy chain with the anti-HER3 FAB-Fc 133 heavy chain was therefore expected to have one heavy chain with two binding sites for Protein G but no binding site for Protein A (the anti-HER3 FAB-Fc 133 heavy chain is abrogated for Protein A binding in its Fc region and its variable domain does not bind Protein A since it belongs to the VH2 subclass, in addition there are two Protein G binding sites, one in its Fc portion and another one in its CH1 domain), and one heavy chain with two binding sites for Protein A but no binding site for Protein G (the anti-HER2 scFv-Fc M428G/N434A heavy chain is abrogated for Protein G binding in its Fc region and there is no additional Protein G binding site present in the scFv format, i.e. there is no CH1 domain; there are also two Protein A binding sites, one in its Fc portion and another one in its VH domain since the latter belongs to the VH3 subclass); whereas one of the two homo-dimeric immunoglobulin species has no binding site for Protein G and four binding sites for Protein A (homo-dimer of the anti-HER2 scFv-Fc M428G/N434A heavy chain) while the other homo-dimeric immunoglobulin species has no binding site for Protein A and four binding sites for Protein G (homo-dimer of the anti-HER3 FAB-Fc 133 heavy chain).

The difference in the number of Protein G and A binding sites between hetero and homo-dimeric species allows for efficient separation of all three molecules using a capture-elution chromatography step on Protein A followed by a second capture-elution chromatography step on Protein G.

Post production, the cell culture supernatant containing all three species was purified by two capture-elution chromatography steps in series, first Protein A and second Protein G, both according to the protocol described in the Methods section. As shown in FIG. 24, all three species were resolved; at each purification step, in capture-elution mode, one of the homo-dimeric immunoglobulin species is efficiently removed since it does not bind to the affinity resin. It is possible to assess the proportion of hetero-dimer in the purified preparation by scanning densitometry analysis of the non-reduced SDS-polyacrylamide (4-12%) gel bands. Using a FluorChem SP imaging system (Witec AG, Littau, Switzerland) and the protocol provided by the manufacturer, it was found that the hetero-dimeric immunoglobulin of interest was purified to homogeneity with >99% purity (FIG. 24C).

In a final example, examples of Protein A and G differential purification methods were combined with the complementary method which abrogates of Protein A binding in VH3 domains and the complementary method which abrogates of Protein G binding in CH1 domains.

Similarly to the last example, an anti-HER2/HER3 hetero-dimeric immunoglobulin was prepared using a scFv-FAB format to generate a difference in SDS-PAGE mobility and facilitate hetero-dimer identification. The anti-HER2 heavy chain was the anti-HER2 scFv(G65S)-Fc 133 heavy chain described in Example 2.1 (heavy chain with SEQ ID NO: 42). The anti-HER3 heavy chain was the anti-HER3 FAB(IGHA1-FG/G)-Fc M428G/N434A heavy chain described in Example 2.2 (heavy chain with SEQ ID NO: 53 and light chain with SEQ ID NO: 47).

The anti-HER2/HER3 hetero-dimeric immunoglobulin resulting from the covalent association of the anti-HER2 scFv(G65S)-Fc 133 heavy chain with the anti-HER3 FAB(IGHA1-FG/G)-Fc M428G/N434A heavy chain was therefore expected to have one heavy chain with one binding site for Protein G but no binding site for Protein A (the anti-HER2 scFv(G65S)-Fc 133 heavy chain is abrogated for Protein A binding in its Fc region and VH3 domain, in addition there is one Protein G binding site in its Fc region but there is no additional Protein G binding site present in the scFv format, i.e. there is no CH1 domain), and one heavy chain with one binding site for Protein A but no binding site for Protein G (the anti-HER3 FAB(IGHA1-FG/G)-Fc M428G/N434A heavy chain is abrogated for Protein G binding in its Fc region and CH1 domain; there is also one Protein A binding site in its Fc portion and its variable domain does not bind Protein A since it belongs to the VH2 subclass); whereas one of the two homo-dimeric immunoglobulin species has no binding site for Protein G and two binding sites for Protein A (homo-dimer of the anti-HER3 FAB(IGHA1-FG/G)-Fc M428G/N434A heavy chain) while the other homo-dimeric immunoglobulin species has no binding site for Protein A and two binding sites for Protein G (homo-dimer of the anti-HER2 scFv(G65S)-Fc 133 heavy chain).

The difference in the number of Protein G and A binding sites between hetero and homo-dimeric species allows for efficient separation of all three molecules using a capture-elution chromatography step on Protein A followed by a second capture-elution chromatography step on Protein G.

Post production, the cell culture supernatant containing all three species was purified by two capture-elution chromatography steps in series, first Protein A and second Protein G, both according to the protocol described in the Methods section. As shown in FIG. 25, all three species were resolved; at each purification step, in capture-elution mode, one of the homo-dimeric immunoglobulin species is efficiently removed since it does not bind to the affinity resin. It is possible to assess the proportion of hetero-dimer in the purified preparation by scanning densitometry analysis of the non-reduced SDS-polyacrylamide (4-12%) gel bands. Using a FluorChem SP imaging system (Witec AG, Littau, Switzerland) and the protocol provided by the manufacturer, it was found that the hetero-dimeric immunoglobulin of interest was purified to homogeneity with >99% purity (FIG. 25C).

This last example shows that the complementary method which abrogates Protein A binding in VH3 domains and the complementary method which abrogates Protein G binding in CH1 domains can be used to tune the number of Protein A and G sites within hetero-dimeric immunoglobulins. Using these complementary techniques, it therefore possible to decrease the number of binding sites that allow for differential purification to a minimum, thereby allowing the use of milder eluting conditions; a feature which is expected to be beneficial in terms of overall hetero-dimer recovery.

### Example 4: surface Plasmon Resonance analysis of Protein A and G abrogating mutations

### 4.1 Binding to human neonatal Fc receptor

Binding to the neonatal Fc receptor protects immunoglobulins from degradation and increase their half-life, it therefore essential that substitutions made in the Fc region that would abrogate or reduce their binding to Protein A or G do not disrupt binding to the neonatal receptor.

To assess the impact of the substitutions used herein on human FcRn binding, SPR experiments were performed on homo-dimeric immunoglobulins. Hetero-dimers of heavy chains having one engineered heavy chain carrying an engineered Fc region and the other heavy chain carrying one unmodified Fc region cannot be used in SPR experiments as the binding signal from the unmodified heavy chain may compensate any negative impact which may have been induced in the engineered heavy chain.

Homo-dimeric immunoglobulins were all formatted with the same variable heavy chain and variable light chain domains originating from a humanized anti human CD19 antibody disclosed in the PCT Publication No: WO10/095031.

When performing SPR measurements, it is best to immobilize bivalent molecules, e.g. homo-dimeric immunoglobulins onto the sensor chip. If bivalent molecules are used as analytes, SPR measurements will bear an avidity component in addition to affinity. Software analysis can model bivalency and extract affinity constants however it is always preferable to circumvent any avidity bias by working with a monovalent analyte whenever possible. To fit this purpose, each homo-dimeric immunoglobulin was directly coupled onto a CM5 sensor chip. A soluble form of the extracellular region of the human FcRn consisting of its alpha chain non-covalently associated with beta2-microglobulin protein was prepared and used as analyte.

Human FcRn production and details of the experimental procedure for SPR measurements can be found in the Methods section.

Importantly, all variants showed binding at pH 6.0 and retained pH dependent release; their affinities and relative affinities for human FcRn are shown in FIG. 26 and 27, respectively, examples of SPR sensorgrams are shown in FIG. 28.

FIG. 26 shows the KD values for the substitutions used in the methods based on Protein A or Protein G abrogation. The unmodified IGHG1 control immunoglobulin had a KD of about 2000 nM, a value in agreement with KD values previously reported for the binding of native human IGHG1 antibodies to human FcRn (1700-2500 nM, Zalevsky J et al., (2010) Nat. Biotechnol., 28(2): 157-159). All IGHG1-IGHG3 based substitutions used in the Protein A abrogating methods had KD values on the upper range or above the values observed for the binding of native human IGHG1 antibodies to human FcRn (anti-hCD19 FAB-Fc 133, anti-hCD19 FAB-Fc 113 and anti-hCD19 FAB-Fc H435R/Y436F). This observation was evident when the binding of the different variants was expressed in terms of relative binding to that of the unmodified IGHG1 control immunoglobulin (FIG. 27). Substitutions used in the Protein A methods including the minimal pair of substitutions H435R/Y436F only retained 73 to 77% of the binding observed for the native human IGHG1control, while the minimal pair of substitutions used in the Protein G method achieved 93% retention (M428G/N434A). From these measurements, it can be concluded that the method for differential purification on Protein G is the most efficient method to purify hetero-dimeric immunoglobulins while maintaining human FcRn binding. Surprisingly, it was found that the substitution N434A compensates for the negative impact of the M428G substitution (relative ratio of 3.13 and 0.45, respectively).

### 4.2 Binding to human Fc gamma receptor 3a

Antibody affinity for hFcyR3a is confined to the Fc region of antibodies. Fc engineering studies have shown that Fc substitutions can have a great impact on antibody's ability to bind hFcyR3a and elicit effector functions such as antibody-dependent-cell-cytotoxicity (ADCC) (Strohl WR et al. (2009) Curr Opin Biotechnol., 20(6): 685-91).

To assess if substitutions M428G and N434A impacted on hFcyR3a affinity, the homo-dimeric anti-HER3 FAB-Fc M428G/N434A antibody and an isotype control antibody (homo-dimeric anti-hCD19 FAB-Fc IGHG1 antibody) were assayed for hFcyR3a binding by SPR. Measurements on recombinant hFcyR3a were performed and the results are shown in FIG. 29. Both antibodies had close KD values demonstrating that the substitutions had no impact in terms of specificity or affinity. It is therefore expected that substitutions M428G and N434A could be broadly used to engineer out Protein G binding within gamma isotype Fc regions without significant loss of hFcγR3a binding.

### Example 5: Immunogenicity prediction of Protein A and G abrogating substitutions

Many approved chimeric, humanized, and fully human antibodies induce a marked anti-drug antibody response in humans. Neutralizing anti-drug antibodies can interfere with drug-target interaction resulting in a decrease of efficacy. In some cases anti-drug antibodies might lead to toxicity due to the formation of immune complexes. Computational models and *in vitro* T cell stimulation tests have been developed to predict CD4+ T cell epitopes.

The predicted immunogenicity of the Protein A and Protein G abrogating mutations was investigated using Lonza's Epibase platform™ (Lonza, Applied Protein Services, Cambridge, UK). The Epibase™ v.3 technology, a structural bioinformatics approach to predict immunogenicity was used to search for potential T cell epitopes in targeted amino acid sequences. The technology integrates experimentally derived binding affinities of peptides to HLA receptors as well as the characteristics of the latest 3D structures of HLA receptors. Practically, this *in-silico* method cuts an amino acid sequence in peptides of ten amino acids in length (10-mer) and calculates a quantitative estimate of their binding strength to HLA class II receptors from 43 DRB1 allotypes. Self-peptides corresponding to human antibody germline amino acid sequences are excluded from the analysis.

Immunogenicity prediction for substitutions M428G and N434A that abrogate Protein G binding in IgG Fc regions and substitution N82aS that abrogate Protein A binding in the VH3 domain subclass were investigated.

The amino acid sequence of the anti-HER2 scFv fragment mentioned in Example 2.1 fused to a Fc IGHG1 region (SEQ ID NO: 61) was used as a control sequence, and was further modified to design two additional input sequences for Epibase: a second amino acid sequence having substitutions M428G and N434A and a third amino acid sequence having substitution N82aS.

Among the 16 *in-silico* peptides generated to encompass substitutions M428G and N434A, only one peptide appeared as a strong epiptope (LHAHYTQKSL (SEQ ID NO: 99)) for DRB 1*15 and DRB 1*16, other DRB 1 allotypes showed medium or no binding to this specific peptide. Two peptides out of 16 were predicted to have a medium affinity to some DRB1 allotypes.

Peptides generated to encompass substitution N82aS did not show any strong DRB1 binding. Moreover, one peptide from the control sequence which was predicted to bind strongly only did with medium affinity when substitution N82aS was introduced. Results are summarized in FIG. 30 and are compared to other therapeutic antibodies provided by Lonza as reference antibodies.

Immunogenicity predictions for substitutions T209G, T209P, and K213V that abrogate Protein G binding in IgG FAB regions were investigated. Substitutions were not directly tested with Epibase, instead a randomized analysis to assess their immunogenic potential was performed. In this analysis, Epibase is giving a relative score to every possible substitution; the higher the score, the stronger the binding is predicted to be for this substitution. Global DRB 1 score takes in account the critical epitope count, the number of affected allotypes as well as the frequency of affected allotypes. Here substitutions were analyzed within a CH1 IGHG1 context (FIG. 31). The preferred substitution K213V showed a really low immunogenic potential compare to other two amino acid substitutions. Substitutions at position 209 generated higher scores but still presented a very low immunogenic risk.

Overall, all substitutions used in the present example displayed a low immunogenic potential compared to human or humanized antibodies currently used in human therapies.

### Example 6: Thermo-stability analysis of Protein A and G abrogating substitutions

Melting profiles for the human IgG subclasses are known (Garber E & Demarest SJ (2007) Biochem. Biophys. Res. Commun., 355(3): 751-7) and all profiles have been shown to contain three unfolding transitions corresponding to independent unfolding of the CH2, CH3, and FAB regions. Of the four human IgG subclasses, IGHG1 has the most stable CH3 domain (∼85 °C); while CH3 domains from other IgG subclasses are less stable, although none are known to melt below ∼70 °C under physiological conditions. Similarly, all subclasses are known to have CH2 domains with a melting temperature of ∼70 °C.

### 6.1 Thermo-stability analysis of Protein G abrogating substitutions

FIG. 32 shows the melting profiles of a human homo-dimeric Fc region (a dimer of a chain encompassing a γ1 hinge region, a γ1 CH2 domain, and a γ1 CH3 domain) having substitutions M428G and N434A and a non-substituted control Fc region. The first transition having a Tm of 61.6°C represents melting of the CH2 domains while the second transition having a Tm of 79.1°C represents melting of the CH3 domains. These two transitions compared well with the two transitions observed for the control Fc region. From these results, it was concluded that substitutions M428G and N434A had a small impact in terms of thermo-stability since CH2 and CH3 domains have lost 5.9°C and 5.2°C of thermo-stability, respectively.

The impact of substitutions M428G and N434A were also investigated within the context of a homo-dimeric immunoglobulin. The melting profile of the anti-HER3 homo-dimeric immunoglobulin having substitutions M428G and N434A from Example 2.2 is shown in FIG. 33A. The profile displays an additional third transition having a Tm of 82 °C when compared to the profile obtained for the homo-dimeric Fc region having the same substitutions (FIG. 32). This additional transition represents melting of the FAB region, while the other two transitions represent melting of CH2 and CH3 domains as described above: the first transition having a Tm of 65°C represents melting of the CH2 domains and the second transition having a Tm of 79°C represents melting of the CH3 domains. From this result, it was concluded that substitutions M428G and N434A also had a small impact in terms of thermo-stability within a homo-dimeric immunoglobulin since CH2 and CH3 domains have lost 6.1°C and 4°C of thermo-stability, respectively, when compared to the melting profile of a non-substituted equivalent immunoglobulin such as the anti-hCD19 antibody shown in FIG. 34A.

Finally, the impact of substitutions T209G, T209P and K213V that abrogate Protein G binding within FAB fragments from gamma isotypes were also investigated within the context of a homo-dimeric immunoglobulin. Melting profiles of the anti-HER3 homo-dimeric immunoglobulins having substitution T209G or T209P or K213V combined with Fc substitutions M428G and N434A from Example 2.2 are shown in FIG. 33A and 33B. The profiles show that FAB thermo-stability was only marginally affected by substitutions T209G and K213V (-3.6°C and -3.4°C, respectively), while substitution T209P had the greatest impact with a loss of 10.8 °C. Substitutions T209G and K213V are therefore preferred when substituting immunoglobulins to abrogate Protein G binding within a FAB region.

### 6.2 Thermo-stability analysis of homo-dimeric immunoglobulin having reduced or no binding to protein A

Thermo-stability of different combinations of gamma isotype CH2 and CH3 domains that reduce or abrogate protein A binding in Fc regions was investigated within the context of a homo-dimeric immunoglobulin format. The melting profiles of the anti-hCD19 homo-dimeric immunoglobulins discussed in Example 4 are shown in FIG. 34. The profiles displays two transitions, the first transition represents melting of CH2 domains (∼70⁰C) while the second transition represents melting of the FAB region overlapping with the expected transition for melting of CH3 domains (∼82°C). From these results, it was concluded that domain combinations Fc 113 and Fc 133 (wherein the numerals correspond to the immunoglobulin gamma isotype subclass of each domain in the order of: hinge/CH2/CH3), and their IGHG1 control (FIG. 34A) had almost identical melting profiles (differences of -0.8 to -2.1°C) and therefore that these domain combinations had only a marginal impact in terms of thermo-stability within a homo-dimeric immunoglobulin format.

### Example 7: Pharmacokinetic analysis of Protein G abrogating substitutions

Pharmacokinetics of a hetero-dimeric anti-HER2/HER2 antibody and its related homo-dimeric anti-HER2 control antibody were investigated.

The anti-HER2/HER2 hetero-dimeric immunoglobulin was built and purified as described for the anti-HER2/HER3 hetero-dimeric immunoglobulin from Example 3.2 and resulted from the covalent association of the anti-HER2 scFv-Fc M428G/N434A heavy chain (SEQ ID NO: 62) with the anti-HER2 FAB-Fc IGHG1 heavy chain (heavy chain with SEQ ID NO: 78 and light chain with SEQ ID NO: 31). The hetero-dimeric immunoglobulin was therefore expected to have one heavy chain with no binding site for Protein G (the anti-HER2 scFv-Fc M428G/N434A is abrogated in its Fc portion for Protein G binding and there is no additional Protein G binding site present in the scFv format, i.e. there is no CH1 domain), and one heavy chain with two binding sites for Protein G (the anti-HER2 FAB-Fc IGHG1 heavy chain has the natural Protein G binding site found in the IGHG1 Fc region and has a second Protein G binding site present in its CH1 domain). Importantly, this particular heavy chain combination resulted in the production of a hetero-dimeric immunoglobulin with only one specificity i.e. towards HER2.

The homo-dimeric anti-HER2 control antibody resulted from the covalent assembly of two copies of the anti-HER2 FAB-Fc IGHG1 heavy chain (heavy chain with SEQ ID NO: 78 and light chain with SEQ ID NO: 31) and was identical to the marketed anti-HER2 antibody known as Trastuzumab (rhuMAbHER2, huMAB4D5-8, trade name Herceptin®; U.S. Patent 5,821,337).

This hetero-dimeric immunoglobulin abrogated for Protein G binding in one heavy chain having only specificity for HER2 was designed to allow a direct comparison with a homo-dimeric immunoglobulin having same specificity in pharmacokinetic analyses. By having the same specificity, the hetero-dimeric immunoglobulin and its related homo-dimeric immunoglobulin control were expected to have similar level of target related degradation. Pharmacokinetic measurements (FIG. 35) showed close serum half-lives for the hetero- and homo-dimeric antibodies. The hetero-dimeric immunoglobulin had serum half-life of approximately 194 h ± 15 (∼8 days) in comparison to 249 h ± 58 (∼10 days) for the control homo-dimeric immunoglobulin (FIG.36).

### Example 8: Functional analysis of Protein A substitutions

HER3 is implicated in tumour genesis of various human cancers including breast and ovarian cancers (Hsieh AC & Moasser MM (2007) Br J Cancer, 97: 453-457; Baselga J & Swain SM (2009) Nat Rev Cancer, 9(7): 463-75). Several anti-HER3 antibodies have been described with some being investigated in human clinical trials (MM-121 antibody (Merrimack Pharmaceuticals Inc., PCT publication No: WO08/100624) and U3-1287 or AMG-888 (U3 PharmaAG/Daiichi Sankyo/Amgen, PCT publication No: WO07/077028).

Bispecific antibodies that would target HER3 and another cancer antigens may have a greater therapeutic impact than conventional i.e. "monospecific" anti-HER3 antibodies. One particularly attractive combination of targets in oncology is the co-targeting of two HER family members. Amongst the HER family of growth factor receptor, co-targeting of EGFR and HER3 or HER2 and HER3 has been described using bispecific antibodies (Schaefer G et al. (2011) Cancer Cell, 20(4): 472-86; McDonagh CF et al. (2012) Mol Cancer Ther., 11(3):582-93).

Since HER3 and HER2 antigens are two preferred targets in oncology, production of a hetero-dimer of heavy chains co-targeting HER2 and HER3 using the Protein A differential purification technologies from the present invention was investigated. To improve heavy chain hetero-dimerization, the hetero-dimeric immunoglobulin co-targeting HER2 and HER3 also made use of the BEAT® technology.

BEAT antibodies are heavy chain hetero-dimers based on a unique concept of bio-mimicry that exhibit superior hetero-dimerization over the "knob-into-hole" method (PCT publication No: WO12/131555 Blein S et al.). The BEAT platform is based on an interface exchange between naturally occurring homo or hetero-dimeric immunoglobulin domain pairs to create new hetero-dimers that can be used as building blocks to design bispecific antibodies. The technology allows for the design of bispecific antibodies from any type of antigen binding scaffold. A scFv-FAB format is used herein to design bispecific antibodies without the need to develop a common light to the antigen binding sites.

Variable heavy and light chain domains from the anti-HER3 antibody described in Examples 2 and 3 were first reported in the PCT publication No: WO07/077028 (Rothe M et al.). Since this variable heavy chain domain does not bind Protein A as it belongs to the VH2 subclass, another anti-HER3 antibody based on the VH3 subclass was developed to demonstrate the utility of Protein A abrogation in VH3 domains when developing bispecific hetero-dimeric immunoglobulins.

To this aim, a scFv-phage display library was screened as described in the Methods section. One preferred scFv fragment exhibited high thermo-stability and was further selected for affinity maturation (SEQ ID NO: 79). Techniques to affinity mature antibodies using phage display are known (Benhar I (2007) Expert Opin Biol Ther., 7(5): 763-79). Diversity was introduced within the scFv gene sequence via NNK diversification in CDR-H1 (Kabat residues: 31 and 32) and CDR-H2 (Kabat residues: 52, 53, 56, and 58) simultaneously, while all others CDRs were kept constant. The resulting affinity maturation library had a diversity of 2.5x10e7 and three rounds of selection using biotinylated antigen and streptavidin capture were performed wherein decreasing amounts of antigen were used as well as competition with non biotinylated antigen. One preferred affinity matured scFv fragment (SEQ ID NO: 80, VH domain of SEQ ID NO: 81; VL domain of SEQ ID NO: 82) had sub-nanomolar affinity for HER3 (as measured by SPR, data not shown) and was further selected for formatting into a bispecific hetero-dimeric immunoglobulin.

Since this affinity matured scFv fragment was based on the VH3 subclass, it was first abrogated for Protein A binding using substitution N82aS (Kabat numbering, SEQ ID NO: 83) and then formatted into a FAB fragment (abbreviated herein as anti-HER3 FAB(N82aS)). The resulting anti-HER3 FAB(N82aS) fragment having a heavy chain of SEQ ID NO: 84 and a light chain of SEQ ID NO: 85, was then used in the design of a bispecific BEAT antibody with the aforementioned anti-HER2 scFv fragment from the anti-HER2 homo-dimeric immunoglobulin described in Example 2.1.

Since BEAT antibodies are heavy chain hetero-dimers, it is needed to distinguish between the two different heavy chains. These are referred herein as BTA and BTB chains. BTA and BTB chains as used herein encompass an antigen binding site, a human IgG1 hinge region, a CH2 domain originating from human IgG1 or IgG3 isotype, and a modified CH3 domain originating from human IgG1 or IgG3 isotype. BTA and BTB chains can be abrogated asymmetrically for Protein A and/or G binding when appropriate.

The anti-HER3 part of the BEAT antibody encompassed the anti-HER3 FAB(N82aS) fragment described above, a CH1γ1 region, a γ1 hinge region, a γ3 CH2 region, and a γ3 based BTA CH3 domain (complete heavy chain sequence with SEQ ID NO: 86 assembled with its cognate light chain having SEQ ID NO: 85, and referred herein as anti-HER3 FAB(N82aS)-BTA IGHG3 heavy chain). The y3 based BTA CH3 domain has been described in WO12/131555 *supra* with SEQ ID NO: 75 (CH3-BT alpha IGHG3 domain).

The anti-human HER2 part of the heterodimeric immunoglobulin encompassed the aforementioned anti-HER2 scFv fragment, a CH1γ1 region, a γ1 hinge region, a γ1 CH2 region, and a γ1 based BTB CH3 domain (complete heavy chain sequence with SEQ ID NO: 87, and referred herein as anti-HER2 scFv-BTB IGHG1 heavy chain). The γ1 based BTB CH3 domain has been described in WO12/131555 *supra,* with SEQ ID NO: 14 (CH3-BT beta domain with substitution F405A).

The hetero-dimeric immunoglobulin resulting from the assembly of these two heavy chains (one being assembled to its cognate light chain) is referred herein BEAT HER2/HER3.

To summarize, the BEAT HER2/HER3 described herein resulted from the covalent association of the anti-HER3 FAB(N82aS)-BTA IGHG3 heavy chain with the anti-HER2 scFv-BTB IGHG1 heavy chain and was therefore expected to have one heavy chain with no binding site for Protein A (the anti-HER3 FAB(N82aS)-BTA IGHG3 heavy chain is abrogated in its Fc region for Protein A binding and the Protein A binding site present in its variable heavy chain domain had been abrogated with substitution N82aS), and one heavy chain with two binding sites for Protein A (anti-HER2 scFv-BTB IGHG1 heavy chain had the natural Protein A binding site found in the IGHG1 Fc region and had a second Protein A binding site present in its VH3 domain). This particular heavy chain combination resulted in the production of the BEAT HER2/HER3 of interest with a total of two Protein A binding sites as well as two homo-dimeric immunoglobulin species, one having no binding site for Protein A while the second species has a total of four.

The difference in the number of Protein A binding sites between hetero and homo-dimeric species allowed for efficient separation of all three molecules by Protein A gradient chromatography as shown in FIG. 37 (same methods as described in Example 3).

To determine whether the BEAT HER2/HER3 could inhibit heregulin induced proliferation of the lung cancer cell line Calu-3, an inhibition of proliferation assay was performed as described in the Methods section. FIG. 38A demonstrates that the BEAT HER2/HER3 inhibited heregulin induced cell proliferation in a dose dependent manner and thus to a greater extent than the anti-HER2 and anti-HER3 control antibodies (Trastuzumab and the aforementioned anti-HER3 described in WO07/077028 *supra,* respectively; data not shown) or their combination. In addition, the BEAT HER2/HER3 inhibited heregulin induced cell proliferation to a greater extent than of the DL11f antibody, an anti-EGFR and anti-HER3 bispecific antibody described in WO10/108127 *supra* (FIG. 38B and C).

## Claims

1. An immunoglobulin or fragment thereof, comprising:
a polypeptide comprising an epitope binding region having at least a VH3 region,
wherein the VH3 region comprises a modification that reduces or eliminates binding of the immunoglobulin or fragment thereof to Protein A, wherein the modification of the VH3 region comprises:
(i) an amino acid substitution at position 65 and/or an amino acid substitution selected from the group consisting of:-57E, 65S, 66Q, 68V, 81E, 82aS and combination 19G/57A/59A (Kabat numbering); or
(ii) an amino acid substitution selected from the group consisting of: 65S, 81E and 82aS (Kabat numbering); or
(iii) the amino acid substitution 65S (Kabat numbering); or
(iv) the amino acid substitution 82aS (Kabat numbering).

2. The immunoglobulin or fragment thereof of claim 1, wherein the polypeptide comprises one or more additional epitope binding regions having at least a VH3 region.

3. The immunoglobulin or fragment thereof of any one of claims 1 to 2, wherein the polypeptide further comprises an immunoglobulin constant region comprising at least a CH2 and/or a CH3 region of a human IGHG selected from IGHG1, IGHG2 and IGHG4, wherein
(i) the immunoglobulin constant region comprises a CH3 region wherein the CH3 region is replaced by a CH3 region from a human IGHG3; or
(ii) the immunoglobulin constant region comprises a CH3 region comprising the amino acid substitution 435R (EU numbering system); or
(iii) the immunoglobulin constant region comprises a CH3 region comprising the amino acid substitution 435R and 436F (EU numbering system).

4. The immunoglobulin or fragment thereof of claim 1, wherein the immunoglobulin or fragment thereof is a hetero-dimeric immunoglobulin or fragment thereof comprising:
(a) a first polypeptide comprising an epitope binding region that binds a first epitope; and
(b) a second polypeptide comprising an epitope binding region having at least a VH3 region that binds a second epitope;
wherein the VH3 region of the second polypeptide comprises said modification that reduces or eliminates binding of the hetero-dimeric immunoglobulin to Protein A.

5. The immunoglobulin or fragment thereof of any one of claim 1 to 3 or the hetero-dimeric immunoglobulin or fragment thereof of claim 4, wherein
(i) the modification increases the half life of the immunoglobulin or fragment thereof or the hetero-dimeric immunoglobulin or fragment thereof *in vivo* compared to an unmodified immunoglobulin or fragment thereof or unmodified hetero-dimeric immunoglobulin or fragment thereof; or
(ii) the modification increases the affinity of the immunoglobulin or fragment thereof or the hetero-dimeric immunoglobulin or fragment thereof for human FcRn compared to an unmodified immunoglobulin or fragment thereof or an unmodified hetero-dimeric immunoglobulin or fragment thereof; or
(iii) the modification results in at least 10% retention of binding of the immunoglobulin or fragment thereof or the hetero-dimeric immunoglobulin or fragment thereof to human FcRn compared to an unmodified immunoglobulin or fragment thereof or an unmodified hetero-dimeric immunoglobulin or fragment thereof, as measured by surface plasmon resonance.

6. The hetero-dimeric immunoglobulin or fragment thereof of any one of claims 4 or 5, further comprising:
(a) a first polypeptide comprising an epitope-binding region that binds a first epitope and an immunoglobulin constant region comprising at least a CH1 and/or a CH2 and/or a CH3 region; and
(b) a second polypeptide comprising an epitope-binding region that binds a second epitope comprising at least a VH3 and/or an immunoglobulin constant region comprising at least a CH2 and/or a CH3 region;
wherein the first polypeptide comprises a modification that reduces or eliminates binding of the hetero-dimeric immunoglobulin or fragment thereof to protein G; and
wherein the second polypeptide comprises a modification that reduces or eliminates binding of the hetero-dimeric immunoglobulin or fragment thereof to protein A.

7. The hetero-dimeric immunoglobulin or fragment thereof of claim 6, wherein the immunoglobulin constant region of the first polypeptide is from human IGHG and the second polypeptide is selected from IGHG1, IGHG2 or IGHG4, wherein the modification of the first polypeptide comprises a modification in the immunoglobulin constant region and said modification of the immunoglobulin constant region comprises:
(i) amino acid substitutions selected from the group consisting of (EU numbering system):
252A/380A/382A/436A/438A;
254M/380M/382L/426M/428G;
426M/428G/433D/434A; or
(ii) an amino acid substitution selected from the group consisting of: 428G, 428S, 428T and 428V and a further substitution at any position within its CH2 region and/or CH3 region or wherein the modification of the immunoglobulin constant region comprises an amino acid substitution selected from 434A or 434S and a further substitution at any position within its CH2 region and/or CH3 region (EU numbering system).

8. The hetero-dimeric immunoglobulin or fragment thereof of claim 7, wherein the modification of the immunoglobulin constant region reduces binding of the immunoglobulin or fragment thereof to Protein G by at least 10% compared to the binding of an unmodified immunoglobulin or fragment thereof.

9. The hetero-dimeric immunoglobulin or fragment thereof of claim 7, wherein the modification in the immunoglobulin constant region further comprises an amino acid substitution at position 250 (EU numbering system) and wherein the amino acid substitution is not 250Q (EU numbering system) or wherein the amino acid substitution is not 428L (EU numbering system).

10. The hetero-dimeric immunoglobulin or fragment thereof of any one of claims 4 to 9, wherein the CH1 region is from human IGHG and is replaced by a CH1 region from IGHA1 or IGHM or wherein the CH1 is from IGHG and strand G and part of the FG loop are replaced by a CH1 strand G and part of the FG loop from IGHA1 or IGHM or wherein the modification of the CH1 region comprises an amino acid substitution at a position selected from the group consisting of 209, 210, 213 and 214 (EU numbering system) or wherein the modification of the CH1 region comprises:
(i) an amino acid substitution at positions 209 and 213 (EU numbering system); or
(ii) amino acid substitutions selected from the group of substitutions consisting of: (EU numbering system):
209P/210S;
213V/214T;
209G/210N;
(iii) the modification comprises the amino acid substitution 209G; or
(iv) the modification comprises the amino acid substitution 213V.

11. A method for the purification of an immunoglobulin or fragment thereof comprising a VH3 region of any one of claims 1 to 10, comprising the steps of:
(i) isolating from a mixture of immunoglobulins a hetero-dimeric immunoglobulin or fragment thereof comprising one modified heavy chain, wherein the modified heavy chain comprises a modification in a VH3 region or in a VH3 region and an immunoglobulin constant region and wherein the modification reduces or eliminates binding of the hetero-dimeric immunoglobulin or fragment thereof to Protein A;
(ii) applying the mixture of immunoglobulins to Protein A; and
(iii) eluting the hetero-dimeric immunoglobulin or fragment thereof from Protein A.

12. An affinity chromatography method for the purification of hetero-dimers of immunoglobulin heavy chains or fragments thereof of any one of claims 4 to 10, wherein at least one VH3 region is present, comprising the steps:
(i) modifying one of the heavy chains to reduce or eliminate binding to Protein A;
(iia) if only one VH3 region is present within the hetero-dimer, said VH3 region is part of the unmodified heavy chain that retains binding to Protein A, or said VH3 region is modified to reduce or eliminate binding to Protein A; or
(iib) if two or more VH3 regions are present within the hetero-dimer, all except one VH3 region is modified to reduce or eliminate binding to Protein A, and the unmodified VH3 region is part of the unmodified heavy chain that retains binding to Protein A; or all VH3 regions are modified to reduce or eliminate binding to Protein A;
(iii) expressing separately or co-expressing the two heavy chains;
(iv) applying the co-expressed heavy chains or previously assembled separately expressed heavy chains to Protein A; and
(v) eluting the hetero-dimers of heavy chains or fragments thereof from Protein A.

13. A method for the differential purification of hetero-dimers of heavy chains of any one of claims 4 to 10, comprising:
(i) isolating from a mixture of heavy chains a hetero-dimer of heavy chains comprising a first heavy chain comprising a modification that reduces or eliminates binding to a first affinity reagent and having a second heavy chain comprising a modification that reduces or eliminates binding to a second affinity reagent;
(ii) applying the mixture of heavy chains to a first column comprising the first affinity reagent;
(iii) eluting the hetero-dimers of heavy chains from the first column;
(iv) applying the eluate from the first column to a second column comprising the second affinity reagent; and
(v) eluting the hetero-dimers of heavy chains from the second column;
wherein the first affinity reagent is Protein A and the second affinity reagent is Protein G or wherein the first affinity reagent is Protein G and the second affinity reagent is Protein A.

14. A method for isolating an immunoglobulin of interest or fragment thereof of any one of claims 1 to 10, from a mixture of immunoglobulins comprising:
(i) isolating the immunoglobulin of interest or fragment thereof from a mixture of immunoglobulins, wherein the immunoglobulin of interest or fragment thereof is eliminated in all its binding sites for Protein A and/or Protein G;
(ii) applying the mixture of immunoglobulins in a first step to Protein A or Protein G;
(iii) collecting the unbound immunoglobulin of interest or fragment thereof from step (ii); and optionally
(iv) applying the unbound immunoglobulin of interest or fragment thereof from step (iii) in a second step to Protein A or Protein G; and
(v) collecting the unbound immunoglobulin of interest or fragment thereof from step (iv) ;
wherein in step (ii) the mixture of immunoglobulins is applied to Protein A and in step (iv) the mixture of immunoglobulins is applied to Protein G; or
wherein in step (ii) the mixture of immunoglobulins is applied to Protein G and in step (iv) the mixture of immunoglobulins is applied to Protein A.
